# FASCICULE DE BREVET EUROPEEN

(11) **EP 1 098 878 B1**
(45) Date de publication et mention de la délivrance du brevet: **17.12.2003**
(21) Numéro de dépôt: 99929495.2
(22) Date de dépôt: 16.07.1999
(51) Int. Cl.: C07D 211/34, A61K 31/445, C07D 405/14, C07D 401/14, C07D 409/14, C07D 417/14, C07D 211/60

(54) **BISPIPERIDINES COMME AGENTS ANTITHROMBOTIQUES**
BISPIPERIDINE MIT ANTITHROMBOTISCHER WIRKUNG
BISPIPERIDINES AS ANTITHROMBOTIC AGENTS

(30) Priorité: 17.07.1998 FR 9809166
(43) Date de publication de la demande: 16.05.2001
(73) Titulaire: CEPHALON FRANCE, 94700 Maisons-Alfort (FR)
(72) Inventeur: YUE, Christophe, Laboratoire L. Lafon, 94701 Maisons Alfort (FR); HENRY, Marguerite, Laboratoire L. Lafon, F4701 Maisons Alfort (FR); GIBOULOT, Thierry, F-94300 Vincennes (FR); LESUR, Brigitte, F-77420 Champs sur Marne (FR)
(74) Mandataire: Bernasconi, Jean Raymond
(86) Numéro de dépôt international: FR9901745
(87) Numéro de publication internationale: WO00003986

(56) Documents cités:
- EP-A- 0 478 362
- DUGGAN, MARK E. ET AL: "Non-Peptide Fibrinogen Receptor Antagonists. 7. Design and Synthesis of a Potent, Orally Active Fibrinogen Receptor Antagonist" J. MED. CHEM. (1995), 38(17), 3332-41 CODEN: JMCMAR;ISSN: 0022-2623, XP002098353

## Description

La présente invention concerne de nouveaux composés qui sont des inhibiteurs de la fixation du fibrinogène sur les récepteurs plaquettaires Gp IIb/IIIa, et qui sont utilisables en thérapeutique comme antithrombotiques.

Au cours des processus pathologiques qui conduisent à la constitution du thrombus (caillot) puis de son extension, l'agrégation plaquettaire représente une étape-dé parce qu'elle est à l'origine de la gravité du phénomène. En effet, dès l'initiation du thrombus, en particulier dans la circulation sanguine artérielle, la mise en jeu de plusieurs réactions biochimiques interdépendantes induit l'agrégation d'un nombre de plus en plus grand de plaquettes par l'intermédiaire de la transformation du fibrinogène soluble en filaments de fibrine insolubles qui augmentent la taille de l'amas de plaquettes, d'abord au siège même de la lésion vasculaire artérielle, puis de plus en plus dans la lumière du vaisseau.

Dans ce mécanisme d'agrégation plaquettaire, l'activation des récepteurs Gp IIb/IIIa est à l'origine de l'amplification de l'agrégation plaquettaire. Le fibrinogène, qui peut se lier par ses deux dimères à ces récepteurs, amplifie la liaison des plaquettes entre elles et induit ainsi la formation d'une masse plaquettaire formant un thrombus au site de rupture de la plaque d'athérome.

Ce mécanisme d'agrégation plaquettaire est particulièrement actif dans toutes les thromboses artérielles qu'elles apparaissent au cours de pratiques de cardiologie interventionnelle (angioplastie percutanée transiuminale; pose de "stents"), de chirurgie cardiaque (pontage aorto-coronarien ; chirurgie valvulaire), au cours de maladies cardiaques alguës (infarctus du myocarde, angor instable, syndromes coronariens aigus, ..) ou au cours de certaines ischémies cérébrales, ou enfin au cours des ischémies myocardiques qui peuvent venir compliquer les suites d'un traitement anti-thrombotique.

Réduire ou empêcher l'activation des plaquettes au contact d'une plaque athérosdérotique rompue représente donc une approche thérapeutique originale et efficace du traitement des thromboses, en particulier des thromboses artérielles et donc un moyen de prévention efficace des syndromes coronariens aigus, dont l'angor instable et l'infarctus du myocarde.

Des composés inhibiteurs de la fixation du fibrinogène sur ses récepteurs sont décrits dans EP-A-0 478 362 et J. Med. Chem., 1995, 38, 3332.

La présente invention vise à fournir de nouveaux inhibiteurs compétitifs de la fixation du fibrinogène sur les récepteurs GpIIb/IIIa.

La présente invention vise en outre à fournir des composés qui puissent être administrés par voie orale, qui permettent l'obtention d'une durée d'action prolongée et qui évitent les risques de saignement.

La présente invention a pour objet des composés de formule générale (I) : 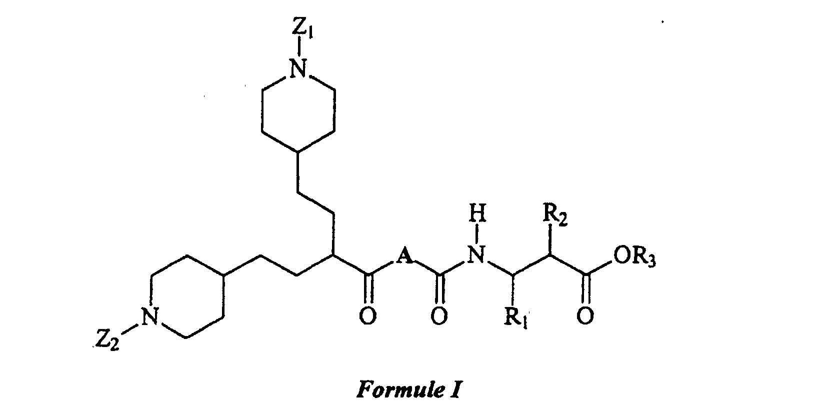 dans laquelle:
i) ou bien R₁ est choisi parmi :
   - les groupes alkyle en C₁-C₄, cycloalkyle mono ou bicyclique en C₃-C₁₂, alkényle en C₂-C₄ ou alkynyle en C₂-C₄, ces groupes étant éventuellement substitués par des groupes choisis parmi les halogènes et le groupe hydroxy ;
   - les groupes aryle mono, bi ou tricycliques en C₆-C₁₄,
   - les groupes hétéroaryle choisis parmi les groupes pyridyle, thiényle, furannyle, quinolyle, benzodioxannyle, benzodioxolyle, benzothiényle, benzofurannyle et pyrazinyle ;
   - les groupes phénylalkyle(C₁-C₄) et naphtylalkyle(C₁-C₄) éventuellement substitués sur le noyau aryle.
      les groupes aryle et hétéroaryle pouvant être substitués par un ou plusieurs groupes choisis indépendamment parmi les halogènes, les groupes alkyle en C₁-C₄, trifluorométhyle, alkyl(C₁-C₄)thio, alkyl(C₁-C₄)sulfonyle, alkyl(C₁-C₄)oxy, nitro, les groupes -COOR, -CH₂COOR ou -O-CH₂-COOR, R étant un groupe alkyle en C₁-C₄,
   - les groupes de formule : 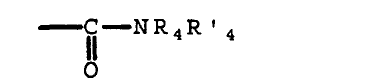
   où R₄ et R'₄ sont choisis parmi les groupes alkyle en C₁-C₈, cycloalkyle mono ou polycyclique en C₃-C₁₂, ces groupes étant éventuellement substitués par des groupes choisis parmi les halogènes et le groupe hydroxy, R'₄ pouvant en outre être l'hydrogène ou bien R₄ et R'₄ forment ensemble un groupe tétraméthylène ou pentaméthylène, ces deux derniers groupes pouvant être eux-mêmes substitués notamment par un reste aryle en C₆-C₁₄ ou aryle (C₆-C₁₄)alkyle(C₁-C₄);
   - les groupes de formule : 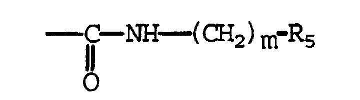
   où m = 1 à 4 et R₅ est choisi parmi les groupes phényle, méthoxyphényle, indolyle, benzodioxolyle, benzodioxannyle, benzothiényle et benzofurannyle,
   et R₂ est l'hydrogène,
ii) ou bien R₁ est l'hydrogène et R₂ est choisi parmi les groupes de formule :

   -NH-CO-R₆,

   R₆ étant choisi parmi les groupes alkoxy en C₁-C₄, cycloalkoxy en C₃-C₇, benzyloxy, méthoxyphényle, diméthoxyphényle, benzodioxolyle et benzodioxannyle,
   et les groupes de formule :

   -NH-SO₂-R₇,

   R₇ étant choisi parmi :
   - les groupes alkyle en C₁-C₅ éventuellement substitués par un ou plusieurs groupes choisis parmi les halogènes, les groupes hydroxy et le groupe trifluorométhyle ;
   - les groupes alkényle en C₂-C₅;
   - les groupes cycloalkyle mono ou bicycliques en C₃-C₁₂;
   - les groupes aryle mono, bi ou tricycliques en C₆-C₁₄ ;
   - les groupes hétéroaryle choisis parmi les groupes pyridyle, furyle, thiényle, quinolyle, benzodioxannyle, benzodioxolyle, isoxazolyle, benzodioxinnyle, benzothiényle, thiazolyle, pyrazolyle, benzofuryle et benzothiazolyle ;
   - les groupes phénylalkyle (C₁-C₄) et naphtylalkyle (C₁-C₄) ;
   - et les groupes de formule : 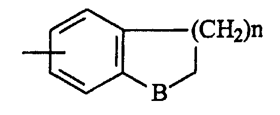 avec n = 1, 2 ou 3 et B étant choisi parmi -CH₂-, O ou S et -NH- les groupes aryle ou hétéroaryle étant éventuellement substitués par un ou plusieurs groupes choisis indépendamment parmi les halogènes, les groupes alkyle en C₁-C₄, cycloalkyle en C₃-C₇, trifluorométhyle, alkyl (C₁-C₄)thio, alkyl(C₁-C₄)oxy, alkyl(C₁-C₄)sulfonyle, nitro, di(alkylC₁-C₄)amino, les groupes -COOR,-CH₂-COOR, ou -O-CH₂COOR, R étant un groupe alkyle en C₁-C₄, les groupes phényle, naphtyle et les groupes hétéroaryle choisis parmi les groupes thiènyle, furyle et pyridyle,
iii) R₃ est choisi parmi un atome d'hydrogène, un groupe alkyle en C₁-C₄ et un groupe phényl(alkyle en C₁-C₄);
iv) A est choisi parmi les groupes -NH-CHR₁₀-, -NH-CHR₁₀-CH₂- et 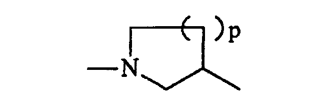 avec p = 1 ou 2,
   - R₁₀ étant choisi parmi l'hydrogène, un groupe alkyle en C₁-C₄ et un groupe aryle en C₆-C₁₄,
v) et Z₁ et Z₂ représentent l'hydrogène ou un groupe protecteur d'amine, et leurs sels d'addition avec des acides pharmaceutiquement acceptables.

Un groupe particulier de composés de formule (I) est représenté par les composés de formule (Ia) : 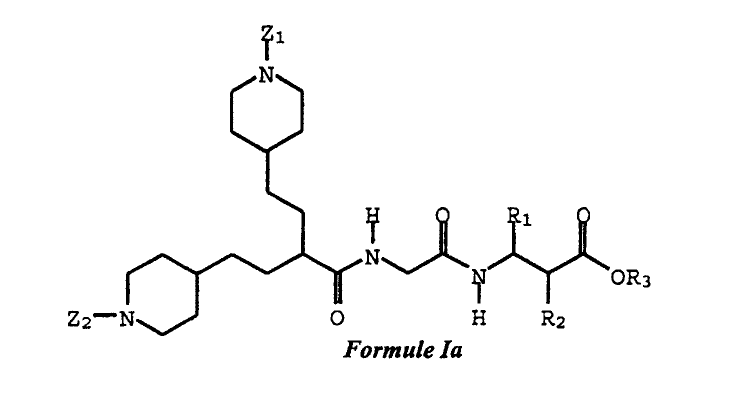 dans laquelle :
i) ou bien R₁ est choisi parmi :
   - les groupes alkyle en C₁-C₄, cycloalkyle mono ou bicyclique en C₃-C₁₂, alkényle en C₂-C₄ ou alkynyle en C₂-C₄, ces groupes étant éventuellement substitués par des groupes choisis parmi les halogènes et le groupe hydroxy ;
   - les groupes aryle mono, bi ou tricycliques en C₆-C₁₄,
   - les groupes hétéroaryle choisis parmi les groupes pyridyle, thiényle, furannyle, quinolyle, benzodioxannyle, benzodioxolyle, benzothiényle, benzofurannyle et pyrazinyle ;
   - les groupes phénylalkyle(C₁-C₄), naphtylalkyle(C₁-C₄) éventuellement substitués sur le noyau aryle.
      les groupes aryle et hétéroaryle pouvant être substitués par un ou plusieurs groupes choisis indépendamment parmi les halogènes, les groupes alkyle en C₁-C₄, trifluorométhyle, alkyl(C₁-C₄)thio, alkyl(C₁-C₄)sulfonyle, alkyl(C₁-C₄)oxy, nitro, les groupes -COOR, -CH₂COOR ou -O-CH₂-COOR, R étant un groupe alkyle en C₁-C₄,
   - les groupes de formule : 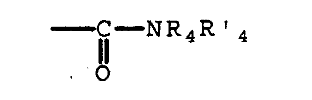
   où R₄ et R'₄ sont choisis parmi les groupes alkyle en C₁-C₈, cycloalkyle mono ou polycyclique en C₃-C₁₂, ces groupes étant éventuellement substitués par des groupes choisis parmi les halogènes et le groupe hydroxy, R'₄ pouvant en outre être l'hydrogène ou bien R₄ et R'₄ forment ensemble un groupe tétraméthylène ou pentaméthylène, ces deux derniers groupes pouvant être eux-mêmes substitués notamment par un reste aryle en C₆-C₁₄ ou aryle (C₆-C₁₄)alkyle(C₁-C₄);
   - les groupes de formule : 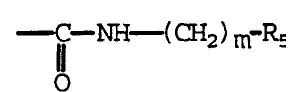
   où m = 1 à 4 et R₅ est choisi parmi les groupes phényle, méthoxyphényle, indolyle, benzodioxolyle, benzodioxannyle, benzothiényle et benzofurannyle,
   et R₂ est l'hydrogène,
ii) ou bien R₁ est l'hydrogène et R₂ est choisi parmi les groupes de formule :

   -NH-CO-R₆,

   R₆ étant choisi parmi les groupes alkoxy en C₁-C₄, cycloalkoxy en C₃-C₇, benzyloxy, méthoxyphényle, diméthoxyphényle, benzodioxolyle et benzodioxannyle,
   et les groupes de formule :

   -NH-SO₂-R₇,

   R₇ étant choisi parmi :
   - les groupes alkyle en C₁-C₅ éventuellement substitués par un ou plusieurs groupes choisis parmi les halogènes, les groupes hydroxy et le groupe trifluorométhyle ;
   - les groupes cycloalkyle mono ou bicycliques en C₃-C₁₂ ;
   - les groupes aryle mono, bi ou tricycliques en C₆-C₁₄ ;
   - les groupes hétéroaryle choisis parmi les groupes pyridyle, thiényle, quinolyle, benzodioxannyle, benzodioxolyle, et isoxazolyle ;
   - les groupes phénylalkyle (C₁-C₄) et naphtylalkyle (C₁-C₄) ;
   - et les groupes de formule : 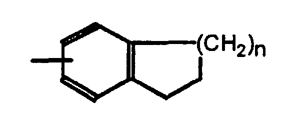 avec n = 1, 2 ou 3 ;
      les groupes aryle ou hétéroaryle étant éventuellement substitués par un ou plusieurs groupes choisis indépendamment parmi les halogènes, les groupes alkyle en C₁-C₄, trifluorométhyle, alkyl (C₁-C₄)thio, alkyl(C₁-C₄)oxy, alkyl(C₁-C₄)sulfonyle, nitro, di(alkylC₁-C₄)amino, les groupes -COOR, -CH₂-COOR, ou -O-CH₂COOR, R étant un groupe alkyle en C₁-C₄,
iii) R₃ est choisi parmi un atome d'hydrogène, un groupe alkyle en C₁-C₄ et un groupe phényl(alkyle en C₁-C₄),
iv) et Z₁ et Z₂ représentent l'hydrogène ou un groupe protecteur d'amine, et leurs sels d'addition avec des acides pharmaceutiquement acceptables.

Un groupe préféré de composés est représenté par les composés dans lequels R₁ = H et R₂ est un groupe de formule -NH-SO₂-R₇.

On préfère tout particulièrement les composés de ce type dans lesquels R₇ est un groupe choisi parmi les groupes naphtyle, naphtyle substitué, biphényle, phénylthiényle.

Comme exemple de groupe aryle on peut citer les groupes phényle, α-naphtyle, β-naphtyle, fluorényle, biphényle.

Les groupes alcoyle en C₁-C₅ peuvent être linéaires ou ramifiés. Comme exemples on peut citer les groupes méthyle, éthyle, propyle, isopropyle, butyle, isobutyle, *tert*iobutyle, pentyle.

Les groupes cycloalkyle monocycliques peuvent être par exempte des groupes cyclopentyle ou cyclohexyle.

Les groupes cycloalkyle polycycliques peuvent être par exemple des groupes adamantyle, norbornyle et camphoryle.

Les groupes alkynyle peuvent être par exemple les groupes éthynyle, propargyle, butynyle.

Les groupes alkényle peuvent être par exemple les groupes vinyle, pentényle et allyle.

Les groupes alcoxy en C₁-C₄ peuvent de même être linéaires ou ramifiés. Comme exemples on peut citer les groupes méthoxy, éthoxy, propoxy, isopropoxy, butoxy, isobutoxy et terbutoxy.

Les halogènes peuvent être choisis parmi le fluor, le chlore, le brome et l'iode.

Comme groupes protecteurs d'amine, on peut citer les groupes éthoxycarbonyle, benzyloxycarbonyle, p-nitrobenzyloxycarbonyle et t-butoxycarbonyle.

Les "sels d'addition avec des acides pharmaceutiquement acceptables" désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique ; des sels métalliques acides, tels que l'orthophosphate disodique et le sulfate monopotassique, et des acides organiques.

Les composés de formule (I) peuvent être préparés par :
a₁) réaction d'un acide de formule : 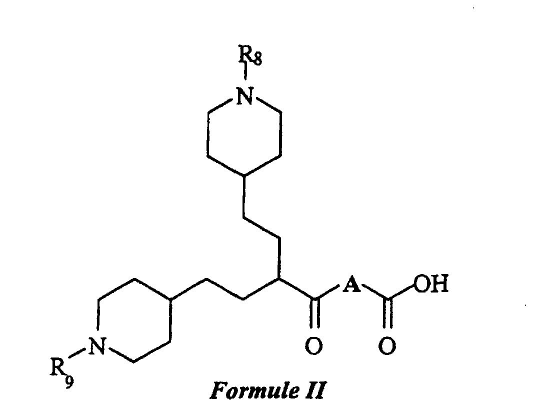 où R₈ et R₉ sont des groupes protecteurs, avec une amine de formule 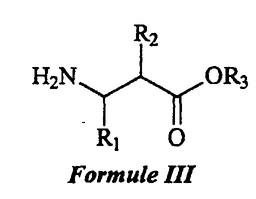 ou
a₂) réaction d'un acide de formule 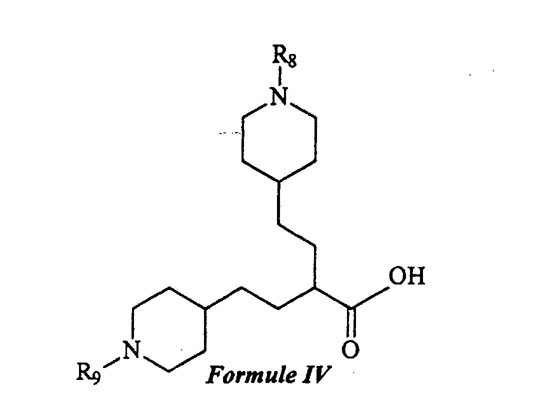 où R₈ et R₉ sont des groupes protecteurs, avec une amine de formule 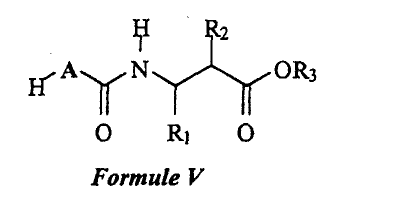 pour obtenir des composés de formule (I b) : 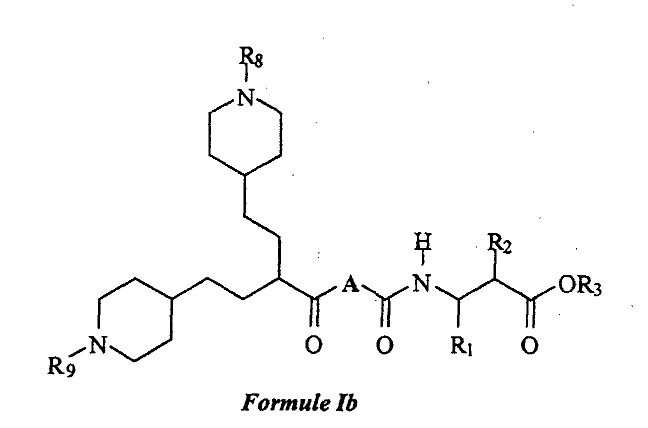
b) éventuellement conversion d'un groupe R₂ en un autre groupe R₂,
c) et éventuellement élimination des groupes protecteurs.

Les composés de formule (II) peuvent être préparés selon le schéma réactionnel suivant (dans le cas où R₈ et R₉ = Boc) :

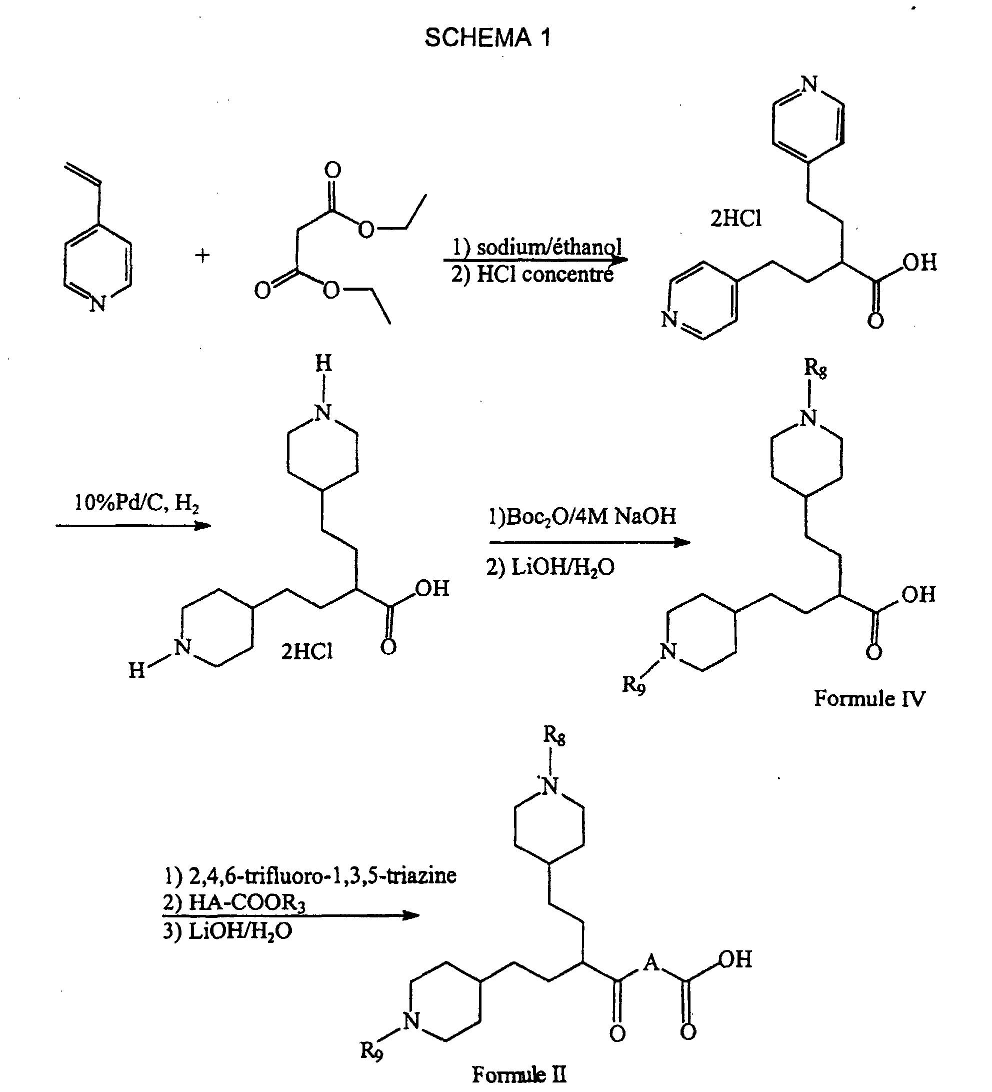

Les sels d'addition s'obtiennent de façon classique par réaction du composé de formule (I) avec un acide pharmaceutiquement acceptable dans un solvant approprié. Inversement, les bases peuvent être obtenues à partir des sels d'addition par traitement par une base forte.

Les exemples suivants illustrent la préparation des composés de formule (I)

### A - Préparation de l'acide de formule IV

### Synthèse de l'acide 4-[1-(tert-butoxycarbonyl)-4-pipéridinyl]-2-{2-[1-(tert-butoxycarbonyl)-4-pipéridinyl]éthyl} butanoïque (composé 3)

### A-1 Synthèse du dichlorhydrate de l'acide 4-(4-pyridinyl)-2-[2-(4-pyridinyl)éthyl] butanoïque (composé 1)

A une solution de 4-vinylpyridine (165 g, 1,49 mol) et de malonate de diéthyle (120 g, 0,75 mol) dans 400 ml d'éthanol, est ajouté du sodium (3,5 g, 0,15 mol). Le mélange est chauffé à reflux pendant 18 heures. On évapore la plupart de l'éthanol et le reste est repris par l'éther (environ 300 ml), puis lavé à la saumure. On évapore le solvant pour obtenir une huile qui est chauffée à reflux dans 400 ml d'acide chlorhydrique 12 N pendant 12 heures. On évapore à sec pour obtenir une huile rouge-brune qui est reprise dans environ 1 l d'isopropanol et laissée reposer à température ambiante. On filtre, rince à l'isopropanol et l'acétone, sèche sous vide pour obtenir 190 g d'un solide beige.
Rendement = 74%
Point de fusion = 172°C
¹H-RMN (400MHz, CD₃OD) : δ 2,1 (m, 4H), 2,5 (m, 1H), 3,0 (t, 4H), 8,0 (d, 4H), 8,7 (d, 4H).

### A-2 Synthèse du dichlorhydrate de l'acide 4-(4-pipéridinyl)-2-[2-(4-pipéridinyl)éthyl] butanoïque (composé 2)

Un mélange du dichlorhydrate de l'acide 4-(4-pyridinyl)-2-[2-(4-pyridinyl)éthyl] butanoïque (118 g, 0,344 mol) dans 1,5 I d'acide acétique est hydrogéné en présence de charbon palladié à 10% (10 g) sous 100 psi à 60°C pendant 24 heures. On filtre et évapore pour obtenir une huile que l'on triture dans de l'éther pour donner une suspension. On filtre, rince à l'éther et sèche pour obtenir 126 g d'un solide beige.
Rendement = 104% (contenant de l'acide acétique)
Point de fusion = 180°C
¹H-RMN (400MHz, CD₃OD) δ 1,35 (m, 8H), 1,6 (m, 6H), 2,0 (bd, 4H), 2,3 (m, 1H), 3,0 (bt, 4H), 3,4 (bd, 4H).

### A-3 Synthèse de l'acide 4-[1-(tert-butoxycarbonyl)-4-pipéridinyl]-2-{2-[1-(tert-butoxycarbonyl)-4-pipéridinyl]éthyl} butanoïque (composé 3)

A une solution du dichlorhydrate de l'acide 4-(4-pipéridinyl)-2-[2-(4-pipéridinyl)éthyl] butanoïque (71,5 g, 0,203 mol) dans 300 ml (1,2 mol) de NaOH 4M et 300 ml de *tert-*butanol, est ajouté du dicarbonate de di-*tert-*butyle (90 g, 0,413 mol) à température ambiante. L'agitation est maintenue pendant 4 heures. La phase organique est séparée, puis lavée à l' HCl 1 N et l'eau, séchée sur sulfate de sodium, évaporée pour donner le produit brut. On ajoute du cyclohexane et laisse cristalliser à environ 0°C. On filtre, rince au cyclohexane, sèche sous vide pour obtenir 71 g d'un solide blanc.
Rendement = 73%
Point de fusion = 162°C
¹H-RMN (400MHz, CDCl₃) : δ 1,05 (m, 4H), 1,25 (m, 4H), 1,35 (m, 3H), 1,45 (s, 19H), 1,6 (bd, 6H), 2,25 (m, 1H), 2,88 (bt, 4H), 4,05 (bs, 4H).

### B - Préparation des composés de formule II

### B-1 Synthèse de l'acide 2-[(4-[1-(tert-butoxycarbonyl)-4-pipéridinyl]-2-{2-[1-(tert-butoxycarbonyl)-4-pipéridinyl]éthyl}butanoyl)amino]acétique (composé 4)

A une solution d'acide 4-[1-(*tert*-butoxycarbonyl)-4-pipéridinyl]-2-{2-[1-(*tert*-butoxycarbonyl)-4-pipéridinyl]éthyl}butanoïque (18,6 g, 38,6 mmol) dans 150 ml de dichlorométhane et de pyridine (3,1 g, 39,2 mmol), on ajoute de la 2,4,6-trifluoro-1,3,5-triazine (3,6 g, 26,7 mmol) à température ambiante. Après 3 heures d'agitation, on ajoute de l'eau. La phase organique est lavée à l'eau, séchée sur sulfate de sodium, puis filtrée. Le filtrat est utilisé directement dans l'étape suivante.

La solution est ajoutée à un mélange de chlorhydrate du glycinate de méthyle (4,9 g, 39 mmol), de diisopropyléthylamine (11 g, 85,3 mmol) dans 50 ml de dichlorométhane. L'agitation est maintenue à température ambiante pendant une heure, puis de l'acide chlorhydrique 1N est ajouté. La phase organique est lavée à l'eau, séchée sur sulfate de sodium, évaporée pour donner une huile qui est hydrolysée directement.

Une solution du produit obtenu ci-dessus dans 150 ml de tétrahydrofurane, 30 ml d'eau et d' hydroxyde de lithium monohydraté (4,2 g, 100 mmol) est agitée à température ambiante pendant 30 minutes. On évapore le solvant organique, le résidu est repris dans l'eau, acidifié à pH 2 et extrait à l'acétate d'éthyle. Les extraits sont lavés à l'eau, séchés sur sulfate de sodium, évaporés pour donner 18,2 g d'un solide blanc.
Rendement = 88% (pour les trois étapes).
¹H-RMN (400MHz, CDCl₃) : δ 0,95∼1,65 (m, 36H), 2,04 (m, 1H), 2,65 (bt, 4H), 4,0 (bd, 6H), 6,39 (bs, 1H).

La synthèse B-1 a été utilisée pour la préparation des composés suivants :

### B-2 Acide 3-[(4-[1-(tert-butoxycarbonyl)-4-pipéridinyl]-2-{2-[1-(tert-butoxycarbonyl)-4-pipéridinyl]éthyl}butanoyl)amino] propanoïque (composé 5)

Produit de départ : le chlorhydrate du 3-amino-propanoate d'éthyle
Rendement = 86 %

### B-3 Acide 3-[(4-[1-(tert-butoxycarbonyl)-4-pipéridinyl]-2-{2-[1-(tert-butoxycarbonyl)-4-pipéridinyl]éthyl}butanoyl)amino]-3-méthyl-propanoïque (composé 6)

Produit de départ : le 3-amino-butanoate d'éthyle
Rendement = 49 %
¹H-RMN (400MHz, CDCl₃) : δ 1,03 (m, 4H), 1,15 (m, 4H), 1,25 (d, 3H), 1,35 (m, 2H), 1,45 (s, 20H), 1,63 (bd, 6H), 1,95 (m, 1H), 2,55 (dd, 2H), 2,65 (bt, 4H), 4,0 (bs, 4H), 4,35 (m, 1H), 6,25 (d, 1H).

### B-4 Acide 3-[(4-[1-(tert-butoxycarbonyl)-4-pipéridinyl]-2-{2-[1-(tert-butoxycarbonyl)-4-pipéridinyl]éthyl}butanoyl)amino]-3-phényl-propanoïque (composé 7)

Produit de départ : le chlorhydrate du 3-amino-3-phényl-propanoate d'éthyle Rendement = 68 %
¹H-RMN (400MHz, CDCl₃) : δ 0,9∼1,35 (m, 10H), 1,45 (s, 20H), 1,58 (m, 6H), 2,00 (m, 1H), 2,60 (bq, 4H), 2,90 (dq, 2H), 4,0 (bd, 4H), 5,45 (q, 1H), 6,78 (d, 1H), 7,25 (m, 5H).

### B-5 Acide (3R)-1-(4-[1-(tert-butoxycarbonyl)-4-pipéridinyl]-2-{2-[1-(tert-butoxycarbonyl)-4-pipéridinyl]éthyl}butanoyl)hexahydro-3-pyridinecarboxylique (composé 8)

Produit de départ : le L-tartrate du (R)-nipecotate d'éthyle
Rendement = 66 %

### C - Préparation des composés de formule Ib

### C-1 - Préparation des composés de formule Ib (R ₁ ≠ H, R ₂ =H)

### 1) 4-{3-{[1-(1,3-benzodioxol-5-yl)-3-éthoxy-3-oxopropyl]amino}-2-oxoéthyl)amino]carbonyl}-5-[1-(tert-butoxycarbonyl)-4-pipéridinyl]pentyl} tétrahydro-1(2H)-pyridinecarboxylate de tert-butyle (composé 9)

A une solution de l'acide 4-[1-(*tert*-butoxycarbonyl)-4-pipéridinyl]-2-{2-[1-(*tert*-butoxycarbonyl)-4-pipéridinyl]éthyl}butanoyl)amino]acétique (composé 4) (5,4 g, 10 mmol) dans 50 ml d'acétate d'éthyle et de la N-méthylmorpholine (2,2g, 22 mmol), on ajoute du chloroformiate d'isobutyle (1,5 g, 11 mmol) à température ambiante. Après 10 minutes d'agitation, le chlorhydrate du 3-amino-3-(1,3-benzodioxol-5-yl)propionate d'éthyle (2,8 g, 10 mmol) est additionné. L'agitation est maintenue à 50°C pendant 2 heures, puis de l'acide chlorhydrique 2N est ajouté. La phase organique est lavée à l'eau, séchée sur sulfate de sodium, évaporée et purifiée par chromatographie flash (dichlorométhane/méthanol, 20/1) pour donner 6,7g d'un solide blanc.
Rendement = 88%
¹H-RMN (400MHz, CDCl₃) : δ 0,9∼1,7 (m, 39H), 2,05 (m, 1 H), 2,6 (bs, 4H), 2,8 (dq, 2H), 4,0 (m, 8H), 5,3 (q, 1H), 6,55 (t, 1H), 6,75 (m, 3H), 7,55 (d, 1 H).

La méthode décrite ci-dessus a été utilisée pour préparer les composés suivants :

### 2) 4-(5-[1-(tert-butoxycarbonyl)-4-pipéridinyl]-3-{[(2-{[3-éthoxy-1-(4-isopropylphényl)-3-oxopropyl]amino}-2-oxoéthyl)amino]carbonyl}pentyl)tétrahydro-1(2H)-pyridinecarboxylate de tert-butyle (composé 10)

Produit de départ : le chlorhydrate du 3-amino-3-(4-isopropylphényl)propionate d'éthyle
Rendement = 82%
¹H-RMN (400MHz, CDCl₃) : δ 0,9∼1,7 (m, 45H), 2,05 (m, 1H), 2,6 (bs, 4H), 2,8 (m, 3H), 4,0 (m, 8H), 5,4 (q, 1H), 6,5 (t, 1H), 7,15 (d, 2H), 7,2 (d, 2H), 7,45 (d, 1H).

### 3) 4-(5-[1-(tert-butoxycarbonyl)-4-pipéridinyl]-3-{[(2-{[3-éthoxy-1-(4-méthoxyphényl)-3-oxopropyl]amino}-2-oxoéthyl)amino]carbonyl}pentyl) tétrahydro-1(2H)-pyridinecarboxylate de tert-butyle (composé 11)

Produit de départ: le chlorhydrate du 3-amino-3-(4-méthoxyphényl)propionate d'éthyle
Rendement = 59%
¹H-RMN (400MHz, CDCl₃) : δ 0,9∼1,7 (m, 39H), 2,05 (m, 1H), 2,6 (bs, 4H), 2,8 (dq, 2H), 3,75 (s, 3H), 4,0 (m, 8H), 5,38 (q, 1H), 6,55 (t, 1H), 6,85 (d, 2H), 7,2 (d, 2H), 7,45 (d, 1H).

### 4) 4-(5-[1-(tert-butoxycarbonyl)-4-pipéridinyl]-3-{[(2-{[3-éthoxy-1-(3,4-diméthoxyphényl)-3-oxopropyl]amino}-2-oxoéthyl)amino]carbonyl} pentyl)tétrahydro-1(2H)-pyridinecarboxylate de tert-butyle (composé 12)

Produit de départ : le chlorhydrate du 3-amino-3-(3,4-diméthoxyphényl)propionate d'éthyle
Rendement = 82%

### 5) 4-[5-[1-(tert-butoxycarbonyl)-4-pipéridinyl]-3-({[2-({3-éthoxy-1-[3-(2-éthoxy-2-oxoéthoxy)phényl]-3-oxopropyl}amino)-2-oxoéthyl]amino} carbonyl)pentyl]tétrahydro-1(2H)-pyridinecarboxylate de tert-butyle (composé 13)

Produit de départ : le chlorhydrate du 3-amino-3-(3-(2-éthoxy-2-oxoéthoxy)phényl)propionate d'éthyle
Rendement = 61%
¹H-RMN (400MHz, CDCl₃) δ 0,9∼1,7 (m, 42H), 2,05 (m, 1H), 2,6 (bs, 4H), 2,8 (dq, 2H), 4,0 (m, 8H), 4,28 (q, 2H), 4,6 (s, 2H), 5,4 (q, 1H), 6,5 (t, 1H), 6,8 (dd, 1H), 6,9 (m, 2H), 7,2 (d, 1H), 7,45 (d, 1H).

### 6) 4-(5-[1-(tert-butoxycarbonyl)-4-pipéridinyl]-3-{[(2-{[3-éthoxy-1-(3-méthoxyphényl)-3-oxopropyl]amino}-2-oxoéthyl)amino]carbonyl} pentyl)tétrahydro-1(2H)-pyridinecarboxylate de tert-butyle (composé 14)

Produit de départ : le chlorhydrate du 3-amino-3-(3-méthoxyphényl)propionate d'éthyle
Rendement = 78%
¹H-RMN (400MHz, CDCl₃) : δ 0,9∼1,7 (m, 39H), 2,05 (m, 1H), 2,6 (bs, 4H), 2,83 (dq, 2H), 3,8 (s, 3H), 4,0 (m, 8H), 5,4 (q, 1H), 6,5 (t, 1H), 6,8 (dd, 1H), 6,85 (m, 2H), 7,2 (t, 1H), 7,45 (d, 1H).

### 7) 4-{3-{[1-(2,3-dihydro-1,4-benzodioxin-6-yl)-3-éthoxy-3-oxopropyl]amino}-2-oxoéthyl)amino]carbonyl}-5-[1-(tert-butoxycarbonyl)-4-pipéridinyl]pentyl} tétrahydro-1(2H)-pyridinecarboxylate de tert-butyle (composé 15)

Produit de départ : le chlorhydrate du 3-amino-3-(2,3-dihydro-1,4-benzodioxin-6-yl) propionate d'éthyle
Rendement = 83%
¹H-RMN (400MHz, CDCl₃) : δ 0,9∼1,7 (m, 39H), 2,05 (m, 1H), 2,65 (bs, 4H), 2,8 (dq, 2H), 4,0 (m, 8H), 4,25 (s,4H), 5,3 (m, 1H), 6,55 (t, 1H), 6,79 (s, 1H), 6,81 (d, 2H), 7,5 (d, 1H).

### 8) 4-(5-[1-(tert-butoxycarbonyl)-4-pipéridinyl]-3-{[(2-{[3-éthoxy-1-(3-pyridinyl)-3-oxopropyl]amino}-2-oxoéthyl)amino]carbonyl}pentyl) tétrahydro-1(2H)-pyridinecarboxylate de tert-butyle (composé 16)

Produit de départ : le dichlorhydrate du 3-amino-3-(3-pyridinyl)propionate d'éthyle Rendement = 69%

### 9) 4-(5-[1-(tert-butoxycarbonyl)-4-pipéridinyl]-3-{[(2-{[3-éthoxy-3-oxopropyl]amino}-2-oxoéthyl)amino]carbonyl}pentyl)tétrahydro-1(2H)-pyridinecarboxylate de tert-butyle (composé 17)

Produit de départ : le chlorhydrate du 3-amino-propionate d'éthyle
Rendement = 69%
¹H-RMN (400MHz, CDCl₃) : δ 1,05 (m, 4H), 1,2 (m, 4H), 1,25 (t, 3H), 1,3 (m, 2H), 1,4 (s, 20H), 1,6 (m, 6H), 2,05 (m, 1H), 2,5 (t, 2H), 2,6 (bt, 4H), 3,5 (q, 2H), 3,9 (d, 2H), 4,0 (bs, 4H), 4,15 (q, 2H), 6,4 (bt, 1H), 6,65 (bt, 1H).

### 10) 4-(5-[1-(tert-butoxycarbonyl)-4-pipéridinyl]-3-{[(2-{[3-éthoxy-1-méthyl-3-oxopropyl]amino}-2-oxoéthyl)amino]carbonyl}pentyl)tétrahydro-1(2H)-pyridinecarboxylate de tert-butyle (composé 18)

Produit de départ : le chlorhydrate du 3-amino-butanoate d'éthyle
Rendement = 70%
¹H-RMN (400MHz, CDCl₃) : δ 1,0 (m, 4H), 1,1∼1,35 (m, 13H), 1,4 (s, 19H), 1,55
(m, 6H), 2,05 (m, 1H), 2,5 (m, 2H), 2,6 (bt, 4H), 3,85 (m, 2H), 4,0 (bs, 4H), 4,1 (q, 2H), 4,3 (m, 1H), 6,4 (t, 1H), 6,75 (d, 1H).

### 11) 4-(5-[1-(tert-butoxycarbonyl)-4-pipéridinyl]-3-{[(2-{[3-éthoxy-3-oxo-1-phénéthylpropyl]amino}-2-oxoéthyl)amino]carbonyl}pentyl) tétrahydro-1(2H)-pyridinecarboxylate de tert-butyle (composé 19)

Produit de départ : le chlorhydrate du 3-amino-5-phényl-pentanoate d'éthyle Rendement = 27%
¹H-RMN (400MHz, CDCl₃) δ 1,0 (m, 4H), 1,1∼1,3 (m, 10H), 1,4 (s, 19H), 1,55 (m, 6H), 1,85 (m, 2H), 2,0 (m, 1H), 2,5 (d, 2H), 2,6 (m, 6H), 3,85 (d, 2H), 4,0 (bs, 4H), 4,1 (q, 2H), 4,25 (m, 1H), 6,25 (t, 1H), 6,6 (d, 1H), 7,1 (m, 3H), 7,2 (t, 2H).

### 12) Synthèse du 4-{3-({[2-({1-[(1-adamantylamino)carbonyl]-(1S)-3-benzyloxy-3-oxopropyl}amino)-2-oxoéthyl]amino}carbonyl)-5-[1-(tert-butoxycarbonyl)-4-pipéridinyl]pentyl}tétrahydro-1(2H)-pyridine carboxylate de tert-butyle (composé 20)

A une solution d'acide 2-[(4-[1-(*tert*-butoxycarbonyl)-4-pipéridinyl]-2-{2-[(1-*tert*-butoxycarbonyl)-4-pipéridinyl]éthyl}butanoyl)amino]acétique (composé 4) (6,9 g, 11 mmol) dans 150 ml d'acétate d'éthyle et de N-méthylmorpholine (5 g, 49,5 mmol), on ajoute du chloroformiate d'isobutyle (1,7 g, 12,4 mmol) à température ambiante. On obtient une suspension blanche. Après 10 minutes d'agitation, une solution de trifluoroacétate de (3S)-3-amino-4-(1-adamantylamino)-4-oxobutanoate de benzyle (6,9 g, 11,2 mmol) dans 20 ml d'acétate d'éthyle est ajoutée. L'agitation est maintenue à température ambiante pendant 18 heures. On ajoute de l'acide chlorhydrique 2N. La phase organique est lavée à l'eau, séchée sur sulfate de sodium, évaporée pour donner le produit brut qui est purifié par flash-chromatographie (dichlorométhane/méthanol, 20/1) pour donner 6,6 g d'un solide beige. Rendement = 68%

La méthode décrite ci-dessus a été utilisée pour préparer les composés suivants :

### 13) 4-{3-({[2-({1-[[2-(1H-indol-4-yl)éthyl]amino]carbonyl]-(1S)-3-benzyloxy-3-oxopropyl}amino)-2-oxoéthyl]amino}carbonyl)-5-[1-(tert-butoxycarbonyl)-4-pipéridinyl]pentyl}tétrahydro-1(2H)-pyridinecarboxylate de tert-butyle (composé 21)

Produit de départ : le trifluoroacétate du (3S)-3-amino-4-[2-(1H-indol-4-yl)éthylamino]-4-oxobutanoate de benzyle
Rendement = 49%
¹H-RMN (400MHz, CDCl₃) : δ 1,05 (m, 4H), 1,25 (m, 6H), 1,45 (s, 20H), 1,6 (m, 6H), 2,05 (m, 1H), 2,65 (m, 5H), 2,95 (m, 3H), 3,55 (m, 2H), 3,78 (dq, 2H), 4,05 (bs, 4H), 4,8 (m, 1H), 5,05 (s, 2H), 6,3 (t, 1H), 6,8 (t, 1H), 7,0 (d, 1H), 7,1 (t, 1H), 7,2 (m, 2H), 7,35 (m, 6H), 7,6 (d, 1H), 8,2 (s, 1H).

### 14) 4-{3-({[2-({1-[{(4-méthoxyphénéthyl)amino}carbonyl]-(1S)-3-benzyloxy-3-oxopropyl}amino)-2-oxoéthyl]amino}carbonyl)-5-[1-(tert-butoxycarbonyl)-4-pipéridinyl]pentyl}tétrahydro-1(2H)-pyridinecarboxylate de tert-butyle (composé 22)

Produit de départ : le trifluoroacétate du (3S)-3-amino-4-[(4-méthoxyphénéthyl)amino]-4-oxobutanoate de benzyle
Rendement = 59%
¹H-RMN (400MHz, CDCl₃) : δ 1,05 (m, 4H), 1,25 (m, 6H), 1,45 (s, 20H), 1,6 (m, 6H), 2,05 (m, 1H), 2,65 (m, 7H), 3,05 (dd, 1H), 3,4 (m, 2H), 3,75 (s, 3H), 3,85 (d, 2H), 4,05 (bs, 4H), 4,78 (m, 1H), 5,1 (s, 2H), 6,4 (t, 1H), 6,8 (m, 3H), 7,1 (d, 2H), 7,2 (d, 1H), 7,4 (m, 5H).

### 15) 4-{3-({[2-({1-[{(3-phénylpropyl)amino}carbonyl]-(1S)-3-benzyloxy-3-oxopropyl}amino)-2-oxoéthyl]amino}carbonyl)-5-[1-(tert-butoxycarbonyl)-4 pipéridinyl]- pentyl}tétrahydro-1(2H)-pyridinecarboxylate de tert-butyle (composé 23)

Produit de départ : le trifluoroacétate du (3S)-3-amino-4-[(3-phénylpropyl)amino]-4-oxobutanoate de benzyle
Rendement = 79%

### 16) 4-{3-({[2-({1-[{(1,3-benzodioxol-5yl-méthyl)amino}carbonyl]-(1S)-3-benzyloxy-3-oxopropyl}amino)-2-oxoéthyl]amino}carbonyl)-5-[1-(tert-butoxycarbonyl)-4-pipéridinyl]pentyl}tétrahydro-1(2H)-pyridinecarboxylate de tert-butyle (composé 24)

Produit de départ : le trifluoroacétate du (3S)-3-amino-4-[(1,3-benzodioxol-5ylméthyl)amino]-4-oxobutanoate de benzyle
Rendement = 54%

### 17) 4-{3-({[2-({1-[{(3-méthoxyphénéthyl)amino}carbonyl]-(1S)-3-benzyloxy-3-oxopropyl}amino)-2-oxoéthyl]amino}carbonyl)-5-[1-(tert-butoxycarbonyl)-4-pipéridinyl]pentyl}tétrahydro-1(2H)-pyridinecarboxylate de tert-butyle (composé 25)

Produit de départ : le trifluoroacétate du (3S)-3-amino-4-[(3-méthoxyphénéthyl)amino]-4-oxobutanoate de benzyle
Rendement = 65%
¹H-RMN (400MHz, CDCl₃) : δ 0,95∼1,25 (m, 7H), 1,25∼1,5 (m, 23H), 1,6 (m, 6H), 2,05 (m, 1H), 2,65 (m, 4H), 2,75 (t, 2H), 3,05 (dd, 1H), 3,45 (q, 2H), 3,8 (s, 3H), 3,85 (d, 2H), 4,05 (bs, 4H), 4,78 (m, 1H), 5,1 (s, 2H), 6,48 (t, 1H), 6,75 (m, 3H), 6,9 (t, 1H), 7,4 (m, 6H).

### 18) 4-{3-({[2-({1-[{(2-hydroxy-1,1-diméthyléthyl)amino}carbonyl]-(1S)-3-benzyloxy-3-oxopropyl}amino)-2-oxoéthyl]amino}carbonyl)-5-[1-(tert-butoxycarbonyl)-4-pipéridinyl]pentyl}tétrahydro-1(2H)-pyridinecarboxylate de tert-butyle (composé 26)

Produit de départ : le trifluoroacétate du (3S)-3-amino-4-[(2-hydroxy-1,1-diméthyléthyl)amino] -4-oxobutanoate de benzyle
Rendement = 34%
¹H-RMN (400MHz, CDCl₃) : δ 1,05 (m, 5H), 1,15-1,35 (m, 12H), 1,45 (s, 20H), 1,65 (m, 7H), 2,1 (m, 1H), 2,62 (m, 6H), 3,08 (dd, 1H), 3,5 (q, 2H), 3,8 (dd, 2H), 4,0 (bs, 4H), 4,8 (m, 1H), 5,1 (s, 2H), 6,8 (s, 1H), 6,9 (t, 1H), 7,8 (m, 6H).

### 19) 4-{3-({[2-({1-[{(1-isopropyl-2-méthylpropyl)amino}carbonyl]-(1S)-3-benzyloxy-3-oxopropyl}amino)-2-oxoéthyl]amino}carbonyl)-5-[1-(tert-butoxycarbonyl)-4-pipéridinyl]pentyl}tétrahydro-1(2H)-pyridinecarboxylate de tert-butyle (composé 27)

Produit de départ : le trifluoroacétate du (3S)-3-amino-4-[(1-isopropyl-2-méthylpropyl)amino]-4-oxobutanoate de benzyle
Rendement = 59%
¹H-RMN (400MHz, CDCl₃) : δ 0,9 (m, 9H), 1,0∼1,35 (m, 11H), 1,4 (s, 20H), 1,6 (bs, 6H), 1,79 (m, 2H), 2,05 (m, 1H), 2,65 (m, 6H), 3,1 (dd, 1H), 3,55 (m, 1H), 3,9 (d, 2H), 4,05 (bs, 5H), 4,82 (m, 1H), 5,15 (dd, 2H), 6,3 (t, 1H), 6,45 (d, 1H), 7,3 (m, 6H).

### 20) 4-{3-({[2-({(1S)-3-(benzyloxy)-3-oxo-1-[(4-benzylpipéridino)carbonyl]-propyl}amino)-2-oxoéthyl]amino}carbonyl)-5-[1-(tert-butoxycarbonyl)-4-pipéridinyl]pentyl}tétrahydro-1(2H)-pyridinecarboxylate de tert-butyle (composé 28)

Produit de départ : le trifluoroacétate du (3S)-3-amino-4-oxo-4-(4-benzylpipéridino)butanoate de benzyle
Rendement = 55%

### C-2- Préparation des composés de formule Ib (R₁ = H, R₂ ≠ H)

### 1) Synthèse du 4-[(10S)-3-{2-[1-(tert-butoxycarbonyl)-4-pipéridinyl] éthyl}-10-(éthoxycarbonyl)-4,7,12-trioxo-14-phényl-13-oxa-5,8,11-triazatétradec-1-yl]tétrahydro-1(2H)-pyridinecarboxylate de tert-butyle (composé 29)

A une solution d'acide 2-[(4-[1-(*tert*-butoxycarbonyl)-4-pipéridinyl]-2-{2-[1-(*tert*-butoxycarbonyl)-4-pipéridinyl]éthyl}butanoyl)amino]acétique (composé 4) (46 g, 85,2 mmol) dans 550 ml d'acétate d'éthyle et de la N-méthylmorpholine (19 g, 188 mmol), on ajoute du chloroformiate d'isobutyle (13 g, 95,2 mmol) à température ambiante et obtient une suspension. Après 20 minutes d'agitation, le chlorhydrate du (2S)-3-amino-2-{[(benzyloxy)carbonyl]amino}propanoate d'éthyle (26,3 g, 86,9 mmol) est additionné. L'agitation est maintenue à température ambiante pendant 18 heures, puis le milieu réactionnel est lavé à l'eau, à l'acide chlorhydrique 1 N et à l'eau puis séché sur sulfate de sodium et évaporé pour donner le produit brut qui est purifié par flash-chromatographie (dichlorométhane/méthanol, 20/1) pour donner 61 g d'un solide blanc.
Rendement = 91 %
¹H-RMN (400MHz, CDCl₃) : δ 1,0 (m, 4H), 1,15 (m, 4H), 1,25 (m, 5H), 1,4 (s, 20H), 1,65 (m, 6H), 2,0 (m, 1H), 2,62 (bt, 4H), 3,62 (bs, 2H), 3,85 (m, 2H), 4,05 (bs, 4H), 4,2 (bt, 2H), 4,4 (m, 1H), 5,08 (s, 2H), 5,95 (d, 1H), 6,4 (bs, 1H), 6,9 (bs, 1H), 7,4 (bs, 5H).

La méthode décrite ci-dessus a été utilisée pour préparer les composés suivants :

### 2) 4-[5-[1-(tert-butoxycarbonyl)-4-pipéridinyl]-3-({[2-({2-[(cyclohexylsulfonyl)amino]-3-méthoxy-3-oxopropyl}amino)-2-oxoéthyl]amino}carbonyl) pentyl]tétrahydro-1(2H)-pyridinecarboxylate de tert-butyle (composé 30)

Produit de départ : le trifluoroacétate du 3-amino-2-[(cyclohexylsulfonyl)amino]propanoate de méthyle
Rendement = 81 %
¹H-RMN (400MHz, CDCl₃) : δ 1,05 (m; 4H), 1,1-1,5 (m, 31H), 1,6 (m, 6H), 1,7 (bd, 1H), 1,85 (bd, 2H), 2,05 (m, 1H), 2,2 (bt, 2H), 2,60 (bt, 4H), 2,85 (m, 1H), 3,55 (m, 1H), 3,7 (m, 1H), 3,80 (s, 3H), 3,95 (m, 2H), 4,05 (bs, 4H), 4,2 (m, 1H), 5,7 (d, 1H), 6,55 (t, 1H), 6,95 (t, 1H).

### 3) 4-[5-[1-(tert-butoxycarbonyl)-4-pipéridinyl]-3-({[2-({2-[(isopropylsulfonyl)amino]-3-éthoxy-3-oxopropyl}amino)-2-oxoéthyl]amino}carbonyl) pentyl]tétrahydro-1(2H)-pyridinecarboxylate de tert-butyle (composé 31)

Produit de départ : le trifluoroacétate du 3-amino-2-[(isopropylsulfonyl)amino]propanoate d'éthyle
Rendement = 60%
¹H-RMN (400MHz, CDCl₃) : δ 1,05 (m, 4H), 1,2 (m, 4H), 1,3 (t, 3H), 1,35 (dd, 6H), 1,45 (s, 21 H), 1,6 (m, 6H), 2,05 (m, 1H), 2,65 (bt, 4H), 3,15 (m, 1H), 3,55 (m, 1H), 3,75 (m, 1H), 3,95 (t, 2H), 4,05 (bs, 4H), 4,20 (m, 1H), 4,25 (q, 2H), 5,6 (d, 1H), 6,45 (t, 1H), 6,85 (t, 1H).

### 4) 4-{3-({[2-({2-[(1,3-benzothiazol-2-ylsulfonyl)amino]-3-éthoxy-3-oxopropyl}amino)-2-oxoéthyl]amino}carbonyl)-5-[1-(tert-butoxycarbonyl) 4-pipéridinyl]pentyl}tétrahydro-1(2H)-pyridinecarboxylate de tert-butyle (composé 32)

Produit de départ : le chlorhydrate du 3-amino-2-[(1,3-benzothiazol-2-ylsulfonyl)amino]propanoate d'éthyle
Rendement = 43%
¹H-RMN (400MHz, CDCl₃) : δ 1,05 (m, 4H), 1,15 (t, 3H), 1,20 (m, 4H), 1,3 (m, 2H), 1,45 (s, 20H), 1,60 (m, 6H), 2,10 (m, 1H), 2,65 (bt, 4H), 3,70 (m, 1H), 3,80 (m, 1H), 3,95 (d, 2H), 4,05 (m, 6H), 4,55 (dd, 1H), 6,50 (t, 1H), 6,85 (bs, 1H), 7,10 (t, 1H), 7,55 (m, 2H), 7,95 (dd, 1H), 8,10 (dd, 1H).

### 5) 4-[(11S)-3-{2-[1-(tert-butoxycarbonyl)-4-pipéridinyl]éthyl}-11-(éthoxycarbonyl)-4,8,13-trioxo-15-phényl-14-oxa-5,9,12-triazapentadec-1-yl]tétrahydro-1(2H)-pyridinecarboxylate de tert-butyle (composé 33)

Produits de départ : le composé 5 et le chlorhydrate du (2S)-3-amino-2-{[(benzyloxy)carbonyl]amino}propanoate d'éthyle
Rendement = 65 %
¹H-RMN (400MHz, CDCl₃) : δ 1,0 (m, 4H), 1,15 (m, 4H), 1,25 (t, 5H), 1,4 (s, 20H), 1,55 (m, 6H), 1,9 (m, 1H), 2,3 (bt, 2H), 2,60 (bq, 4H), 3,4 (m, 2H), 3,6 (t, 2H), 4,0 (bs, 4H), 4,2 (q, 2H), 4,4 (m, 1 H), 5,05 (s, 2H), 5,95 (d, 1H), 6,4 (t, 1H), 6,55 (t, 1H), 7,3 (s, 5H).

### 6) 4-[5-[1-(tert-butoxycarbonyl)-4-pipéridinyl]-3-({[3-({3-éthoxy-2-[(2-naphtylsulfonyl)amino]-3-oxopropyl}amino)-1-méthyl-3-oxopropyl]amino}carbonyl)pentyl]tétrahydro-1(2H)-pyridinecarboxylate de tert-butyle (composé 34)

Produits de départ : le composé 6 et le chlorhydrate du (2S)-3-amino-2-[(2-naphtylsulfonyl)amino]propanoate d'éthyle
Rendement = 75 %
¹H-RMN (400MHz, CDCl₃) : δ 0,9 (t, 3H), 1,05 (m, 3H), 1,20 (m, 8H), 1,30 (m, 2H), 1,45 (s, 20H), 1,60 (m, 6H), 1,95 (m, 1H), 2,40 (dq, 2H), 2,65 (bt, 4H), 3,50 (m, 1H), 3,65 (m, 1H), 3,85 (m, 2H), 4,05 (m, 5H), 4,30 (m, 1H), 6,05 (d, 1H), 6,65 (m, 2H), 7,65 (m, 2H), 7,80 (d, 1H), 7,90 (d, 1H), 8,00 (d, 2H), 8,40 (s, 1H).

### 7) 4-[5-[1-(tert-butoxycarbonyl)-4-pipéridinyl]-3-({[3-({3-éthoxy-2-[(2-naphtylsulfonyl)amino]-3-oxopropyl}amino)-3-oxo-1-phénylpropyl]amino}carbonyl)pentyl]tétrahydro-1(2H)-pyridinecarboxylate de tert-butyle (composé 35)

Produits de départ : le composé 7 et le chlorhydrate du (2S)-3-amino-2-[(2-naphtylsulfonyl)amino]propanoate d'éthyle
Rendement = 77 %
¹H-RMN (400MHz, CDCl₃) : δ 0,95 (t, 3H), 1,00 (m, 4H), 1,10 (m, 4H), 1,30 (m, 2H), 1,45 (s, 20H), 1,60 (m, 6H), 2,05 (m, 1H), 2,65 (bd, 4H), 2,70 (m, 2H), 3,40 (m, 1H), 3,60 (m, 1H), 3,80 (m, 2H), 4,00 (m, 6H), 5,40 (m, 1H), 5,85 (d, 1H), 6,40 (m, 1H), 7,25 (m, 5H), 7,65 (m, 2H), 7,76 (m, 1H), 7,90 (d, 1H), 7,95 (m, 2H), 8,40 (d, 1H).

### 8) 4-[5-[1-(tert-butoxycarbonyl)-4-pipéridinyl]-3-{[(3R)-3-[({3-éthoxy-2-[(2-naphthylsulfonyl)amino)-3-oxopropyl}amino)carbonyl]tétrahydro-1(2H) pyridinyl]carbonyl}pentyl)tétrahydro1(2H)-pyridinecarboxylate de tert-butyle (composé 36)

Produits de départ : le composé 8 et le chlorhydrate du (2S)-3-amino-2-[(2-naphthylsulfonyl)amino]propanoate d'éthyle
Rendement = 67%

### 9) Synthèse du chlorhydrate du 4-{3-{[(2-{[(2S)-2-amino-3-éthoxy-3-oxopropyl]amino}-2-oxoéthyl)amino]carbonyl}-5-[1-(tert-butoxycarbonyl)-4-pipéridinyl]pentyl}tétrahydro-1(2H)-pyridinecarboxylate de tert-butyle (composé 37)

Un mélange de 4-[(10S)-3-{2-[1-(*tert*-butoxycarbonyl)-4-pipéridinyl]éthyl}-10-(éthoxycarbonyl)-4,7,12-trioxo-14-phényl-13-oxa-5,8,11-triazatétradec-1-yl]tétrahydro-1(2H)-pyridinecarboxylate de *tert*-butyle (composé 29) (60 g, 76,1 mmol), du charbon palladié à 10% (5 g) dans 400 ml d'éthanol et 77 ml d'éthanol chlorhydrique 1 N est hydrogéné à température ambiante sous environ 25 psi pendant 30 minutes. On filtre et évapore pour obtenir 52 g d'un solide beige.
Rendement = 99%
¹H-RMN (400MHz, CDCl₃) : δ 1,0 (m, 4H), 1,18 (m, 4H), 1,27 (t, 5H), 1,42 (s, 20H), 1,58 (m, 6H), 2,0 (m, 1H), 2,6 (bt, 4H), 3,35 (m, 1H), 3,55 (m, 1H), 3,68 (m, 1H), 3,9 (d, 2H), 4,02 (bs, 4H), 4,18 (q, 2H), 6,38 (bt, 1H), 6,72 (bt, 1H).

La méthode décrite ci-dessus a été utilisée pour préparer les composés suivants :

### 10) Synthèse du 4-{3-{[(3-{[(2S)-2-amino-3-éthoxy-3-oxopropyl]amino}-3-oxopropyl)amino]carbonyl}-5-[1-(tert-butoxycarbonyl)-4-pipéridinyl]pentyl}tétrahydro-1(2H)-pyridinecarboxylate de tert-butyle (composé 38)

Produit de départ : le composé 33
Rendement = 97%

### 11) Synthèse du 4-[5-[1-(tert-butoxycarbonyl)-4-pipéridinyl]-3-({[2-({(2S)-3-éthoxy-3-oxo-2-[(phénylsulfonyl)amino]propyl}amino)-2-oxoéthyl]amino} carbonyl)pentyl]tétrahydro-1(2H)-pyridinecarboxylate de tert-butyle (composé 39)

Le 4-{3-{[(2-{[(2S)-2-amino-3-éthoxy-3-oxopropyl]amino}-2-oxoéthyl) amino]carbonyl}-5-[1-(*tert*-butoxycarbonyl)-4-pipéridinyl]pentyl}tétrahydro-1 (2H)-pyridinecarboxylate de *tert*-butyle (composé 37) (3,31 g, 4,8 mmol) est solubilisé dans 50 ml de dichlorométhane contenant de la triéthylamine (1,04 g, 5 mmol) puis le chlorure de benzènesulfonyle (0,9 g, 5 mmol) est ajouté à environ 5°C. Après 2 heures à température ambiante, on ajoute de l'eau. La phase organique est lavée à l' HCl 1N puis à l'eau, séchée sur sulfate de sodium puis évaporée pour donner le produit brut qui est purifié par flash-chromatographie (dichlorométhane/méthanol, 15/1) pour donner 2,8 g d'un solide blanc.
Rendement = 74%
¹H-RMN (400MHz, CDCl₃) : δ 1,05 (m, 7H), 1,25 (m, 6H), 1,45 (s, 20H), 1,6 (m, 6H), 2,08 (m, 1H), 2,6 (bs, 4H), 3,5 (m, 1H), 3,67 (m, 1H), 4,0 (m, 9H), 6,28 (t, 1H), 6,62 (t, 1H), 7,1 (t, 1H), 7,5 (m, 3H), 7,82 (d, 2H).

La méthode décrite ci-dessus est utilisée pour préparer les composés suivants :

### 12) 4-[5-[1-(tert-butoxycarbonyl)-4-pipéridinyl]-3-({[2-({(2S)-3-éthoxy-3-oxo-2-[(1,3-benzodioxol-5-ylcarbonyl)amino]propyl}amino)-2-oxoéthyl]amino} carbonyl) pentyl]tétrahydro-1(2H)-pyridinecarboxylate de tert-butyle (composé 40)

Produit de départ : le chlorure de l'acide pipéronylique
Rendement = 74%

### 13) 4-[5-[1-(tert-butoxycarbonyl)-4-pipéridinyl]-3-({[2-({(2S)-3-éthoxy-3-oxo-2-[(2-naphthylsulfonyl)amino]propyl}amino)-2-oxoéthyl]amino}carbonyl) pentyl]tétrahydro-1(2H)-pyridinecarboxylate de tert-butyle (composé 41)

Produit de départ : le chlorure de 2-naphthylsulfonyle
Rendement = 74%
¹H-RMN (400MHz, CDCl₃) : δ 0,95 (t, 3H), 1,05 (m, 4H), 1,2 (m, 4H), 1,35 (m, 2H), 1,45 (s, 20H), 1,6 (m, 6H), 2,1 (m, 1H), 2,63 (bt, 4H), 3,55 (m, 1H), 3,7 (m, 1H), 3,9 (q, 2H), 4,0 (m, 7H), 6,3 (bs, 1H), 6,55 (t, 1H), 7,05 (t, 1H), 7,65 (m, 2H), 7,8 (d, 1H), 7,9 (d, 1H), 7,95 (d, 2H), 8,4 (s, 1H).

### 14) 4-[5-[1-(tert-butoxycarbonyl)-4-pipéridinyl]-3-({[2-({(2S)-3-éthoxy-3-oxo-2-[(4-propylphénylsulfonyl)amino]propyl}amino)-2-oxoéthyl]amino}carbonyl) pentyl]tétrahydro-1(2H)-pyridinecarboxylate de tert-butyle (composé 42)

Produit de départ : le chlorure de 4-propylphénylsulfonyle
Rendement = 78%
¹H-RMN (400MHz, CDCl₃) : δ 0,95 (t, 3H), 1,05∼1,4 (m, 15H), 1,5 (s, 18H), 1,65 (m, 8H), 2,15 (m, 1H), 2,65 (bt, 6H), 3,45 (m, 1H), 3,8 (m, 1H), 4,0 (m, 9H), 6,0 (d, 1H), 6,55 (t, 1H), 6,9 (t, 1H), 7,35 (d, 2H), 7,75 (d, 2H).

### 15) 4-[5-[1-(tert-butoxycarbonyl)-4-pipéridinyl]-3-({[2-({(2S)-3-éthoxy-3-oxo-2-[((1,1'-biphényl)-4-ylsulfonyl)amino]propyl}amino)-2 oxoethyl]amino}carbonyl) pentyl]tetrahydro-1(2H)-pyridinecarboxylate de tert-butyle (composé 43)

Produit de départ : le chlorure de 4-biphénylsulfonyle
Rendement = 81%
¹H-RMN (400MHz, CDCl₃) : δ 1,05 (m, 7H), 1,15 (m, 4H), 1,35 (m, 2H), 1,4 (s, 20H), 1,55 (m, 6H), 2,05 (m, 1H), 2,6 (bt, 4H), 3,5 (m, 1H), 3,65 (m, 1H), 3,85∼4,1 (m, 9H), 6,25 (bs, 1H), 6,6 (bt, 1H), 7,05 (bt, 1H), 7,4 (m, 3H), 7,55 (d, 2H), 7,65 (d, 2H), 7,85 (d, 2H).

### 16) 4-[5-[1-(tert-butoxycarbonyl)-4-pipéridinyl]-3-({[2-({(2S)-3-éthoxy-3-oxo-2-[(1-naphthylsulfonyl)amino]propyl}amino)-2-oxoéthyl]amino}carbonyl) pentyl]tétrahydro-1(2H)-pyridinecarboxylate de tert-butyle (composé 44)

Produit de départ : le chlorure de 1-naphthylsulfonyle
Rendement = 92%
¹H-RMN (400MHz, CDCl₃) : δ 0,9 (t, 3H), 1,05 (m, 4H), 1,2 (m, 4H), 1,3 (m, 2H), 1,45 (s, 20H), 1,6 (m, 6H), 2,05 (m, 1H), 2,6 (bt, 4H), 3,45 (m, 1H), 3,6 (m, 1H), 3,75 (m, 2H), 3,8 (m, 2H), 4,0 (m, 5H), 6,3 (d, 1H), 6,4 (bt, 1H), 6,75 (bt, 1H), 7,5 (t, 1H), 7,6 (t, 1H), 7,7 (t, 1H), 7,9 (d, 1H), 8,05 (d, 1H), 8,2 (d, 1H), 8,65 (d, 1H).

### 17) 4-[5-[1-(tert-butoxycarbonyl)-4-pipéridinyl]-3-({[2-({(2S)-3-éthoxy-3-oxo-2-[(4-(méthylsulfonyl)phénylsulfonyl)amino]propyl}amino)-2-oxoéthyl]amino}carbonyl) pentyl]tétrahydro-1(2H)-pyridinecarboxylate de tert-butyle (composé 45)

Produit de départ : le chlorure de 4-(méthylsulfonyl)phénylsulfonyle
Rendement = 80%
¹H-RMN (400MHz, CDCl₃) : δ 1,0 (m, 4H), 1,1 (t, 3H), 1,2 (m, 4H), 1,3 (m, 2H), 1,4 (m, 20H), 1,6 (m, 6H), 2,05 (m, 1H), 2,65 (bt, 4H), 3,1 (s, 3H), 3,55 (m, 1H), 3,65 (m, 1H), 3,9 (d, 2H), 4,0 (m, 7H), 6,6 (bt, 2H), 7,05 (bt, 1H), 8,05 (dd, 4H).

### 18) 4-[5-[1-(tert-butoxycarbonyl)-4-pipéridinyl]-3-({[2-({(2S)-3-éthoxy-3-oxo-2-[(2-thienylsulfonyl)amino]propyl}amino)-2-oxoéthyl]amino}carbonyl) pentyl]tétrahydro-1(2H)-pyridinecarboxylate de tert-butyle (composé 46)

Produit de départ : le chlorure de 2-thiènylsulfonyle
Rendement = 78%
¹H-RMN (400MHz, CDCl₃) : δ 1,0 (m, 4H), 1,15 (m, 7H), 1,3 (m, 2H), 1,4 (s, 20H), 1,6 (m, 6H), 2,05 (m, 1H), 2,6 (bt, 4H), 3,55 (m, 1H), 3,7 (m, 1H), 3,9 (dq, 2H), 4,05 (m, 7H), 6,25 (bs, 1H), 6,55 (bt, 1H), 6,95 (bt, 1H), 7,05 (dd, 1H), 7,55 (dd, 2H).

### 19) 4-{5-[1-(tert-butoxycarbonyl)-4-pipéridinyl]-3-[({2-[((2S)-2-{[(4-chlorophényl)sulfonyl]amino}-3-éthoxy-3-oxopropyl)amino]-2-oxoéthyl}amino)carbonyl] pentyl}tétrahydro-1(2H)-pyridinecarboxylate de tert-butyle (composé 47)

Produit de départ : le chlorure de 4-chlorophénylsulfonyle
Rendement = 63%
¹H-RMN (400MHz, CDCl₃) : δ 1,05 (m, 4H), 1,15 (t, 3H), 1,2 (m, 4H), 1,3 (m, 2H), 1,4 (s, 20H), 1,6 (m, 6H), 2,05 (m, 1H), 2,6 (bt, 4H), 3,45 (m, 1H), 3,7 (m, 1H), 4,0 (m, 9H), 6,1 (bd, 1H), 6,45 (bt, 1H), 6,95 (bt, 1H), 7,45 (d, 2H), 7,75 (d, 2H).

### 20) 4-{5-[1-(tert-butoxycarbonyl)-4-pipéridinyl]-3-[({2-[((2S)-2-{[(4-fluorophényl)sulfonyl]amino}-3-éthoxy-3-oxopropyl)amino]-2-oxoéthyl}amino)carbonyl] pentyl}tétrahydro-1(2H)-pyridinecarboxylate de tert-butyle (composé 48)

Produit de départ : le chlorure de 4-fluorophénylsulfonyle
Rendement = 82%
¹H-RMN (400MHz, CDCl₃) : δ 1,05 (m, 4H), 1,15 (t, 3H), 1,2 (m, 4H), 1,3 (m, 2H), 1,45 (s, 20H), 1,6 (m, 6H), 2,1 (m, 1H), 2,65 (bt, 4H), 3,5 (m, 1H), 3,7 (m, 1 H), 4,1 (m, 9H), 6,2 (bs, 1H), 6,55 (bt, 1H), 7,0 (bt, 1H), 7,2 (t, 2H), 7,85 (dd, 2H).

### 21) 4-[5-[1-(tert-butoxycarbonyl)-4-pipéridinyl]-3-({[2-({(2S)-3-éthoxy-3-oxo-2-[(6-méthoxy-2-naphtylsulfonyl)amino]propyl}amino)-2-oxoéthyl]amino}carbonyl) pentyl]tétrahydro-1(2H)-pyridinecarboxylate de tert-butyle (composé 49)

Produit de départ : le chlorure de 6-méthoxy-2-naphtylsulfonyle
Rendement = 71%
¹H-RMN (400MHz, CDCl₃) : δ 0,95 (t, 3H), 1,0 (m, 4H), 1,15 (m, 4H), 1,25 (m, 2H), 1,45 (s, 20H), 1,6 (m, 6H), 2,05 (m, 1H), 2,6 (bt, 4H), 3,45 (m, 1H), 3,7 (m, 1H), 3,75 (q, 2H), 3,9 (s, 3H), 4,0 (m, 8H), 6,05 (bd, 1H), 6,45 (bt, 1H), 6,75 (bt, 1H), 7,1 (d, 1H), 7,2 (d, 1H), 7,75 (m, 3H), 8,25 (s, 1H).

### 22) 4-[5-[1-(tert-butoxycarbonyl)-4-pipéridinyl]-3-({[2-({(2S)-3-éthoxy-3-oxo-2-[(mésitylsulfonyl)amino]propyl}amino)-2-oxoéthyl]amino}carbonyl) pentyl]tétrahydro-1(2H)-pyridinecarboxylate de tert-butyle (composé 50)

Produit de départ : le chlorure de mésitylsulfonyle
Rendement = 67%
¹H-RMN (400MHz, CDCl₃) : δ 1,0 (m, 4H), 1,10 (t, 3H), 1,20 (m, 4H), 1,35 (m, 2H), 1,45 (s, 20H), 1,65 (m, 6H), 2,05 (m, 1H), 2,25 (s, 3H), 2,65 (s, 6H), 2,67 (m, 4H), 3,50 (m, 1H), 3,65 (m, 1 H), 3,85∼4,15 (m, 9H), 5,90 (d, 1H), 6,40 (bt, 1H), 6,70 (bt, 1H), 6,90 (s, 2H).

### 23) 4-[5-[1-(tert-butoxycarbonyl)-4-pipéridinyl]-3-({[2-({(2S)-3-éthoxy-3-oxo-2-[(butylsulfonyl)amino]propyl}amino)-2-oxoéthyl]amino}carbonyl) pentyl]tétrahydro-1(2H)-pyridinecarboxylate de tert-butyle (composé 51)

Produit de départ : le chlorure de n-butylsulfonyle
Rendement = 66%
¹H-RMN (400MHz, CDCl₃) : δ 0,9 (t, 3H), 1,0 (m, 4H), 1,15 (m, 4H), 1,25-1,4 (s, 27H), 1,6 (m, 6H), 1,75 (m, 2H), 2,05 (m, 1H), 2,65 (bt, 4H), 3,0 (t, 2H), 3,5 (m, 1H), 3,75 (m, 1H), 3,9 (t, 2H), 4,05 (bs, 4H), 4,2 (m, 3H), 5,85 (d, 1H), 6,55 (bt, 1H), 7,0 (bt, 1H).

### 24) 4-[5-[1-(tert-butoxycarbonyl)-4-pipéridinyl]-3-({[2-({(2S)-3-éthoxy-3-oxo-2-[(4-méthylphénylsulfonyl)amino]propyl}amino)-2-oxoéthyl]amino}carbonyl) pentyl]tétrahydro-1(2H)-pyridinecarboxylate de tert-butyle (composé 52)

Produit de départ : le chlorure de 4-méthylphénylsulfonyle
Rendement = 68%
¹H-RMN (400MHz, CDCl₃) : δ 1,0 (m, 4H), 1,15 (t, 3H), 1,2 (m, 4H), 1,35 (m, 2H), 1,45 (s, 20H), 1,6 (m, 6H), 2,1 (m, 1H), 2,4 (s, 3H), 2,65 (bt, 4H), 3,45 (m, 1H), 3,75 (m, 1H), 3,85∼4,15 (m, 9H), 5,95 (bd, 1H), 6,5 (bt, 1H), 6,85 (bt, 1H), 7,25 (d, 2H), 7,7 (d, 2H).

### 25) 4-[5-[1-(tert-butoxycarbonyl)-4-pipéridinyl]-3-({[2-({(2S)-3-éthoxy-3-oxo-2-[(3-méthylphénylsulfonyl)amino]propyl}amino)-2-oxoéthyl]amino}carbonyl) pentyl]tétrahydro-1(2H)-pyridinecarboxylate de tert-butyle (composé 53)

Produit de départ : le chlorure de 3-méthylphénylsulfonyle
Rendement = 86%
¹H-RMN (400MHz, CDCl₃) : δ 1,0 (m, 4H), 1,05 (t, 3H), 1,1 (m, 4H), 1,3 (m, 2H), 1,4 (s, 20H), 1,6 (m, 6H), 2,1 (m, 1H), 2,4 (s, 3H), 2,65 (bt, 4H), 3,4 (m, 1H), 3,75 (m, 1H), 3,85∼4,1 (m, 9H), 5,9 (bs, 1H), 6,45 (bt, 1H), 6,8 (bt, 1H), 7,4 (d, 2H), 7,6 (m, 2H).

### 26) 4-[(10S)-3-{2-[1-(tert-butoxycarbonyl)-4-pipéridinyl]éthyl}-10-(éthoxycarbonyl)-4,7,12,12-tétraoxo-13-phényl-12λ⁶-thia-5,8,11-triazatridec-1-yl]tétrahydro-1(2H)-pyridinecarboxylate de tert-butyle (composé 54)

Produit de départ : le chlorure de 4-benzylsulfonyle
Rendement = 49%
¹H-RMN (400MHz, CDCl₃) : δ 1,0 (m, 4H), 1,2 (m, 4H), 1,25 (m, 5H), 1,45 (s, 20H), 1,6 (m, 6H), 2,05 (m, 1H), 2,6 (bt, 4H), 3,5 (m, 2H), 3,9 (m, 2H), 4,0 (bs, 4H), 4,2 (q, 2H), 4,3 (q, 2H), 5,75 (bd, 1H), 6,4 (bt, 1H), 6,75 (bt, 1H), 7,55 (m, 5H).

### 27) 4-[(10S, 13E)-3-{2-[1-(tert-butoxycarbonyl)-4-pipéridinyl]éthyl}-10-(éthoxycarbonyl)-4,7,12,12-tétraoxo-14-phényl-12λ⁶-thia-5,8,11-triaza-13-tétradecèn-1-yl]tétrahydro-1(2H)-pyridinecarboxylate de tert-butyle (composé 55)

Produit de départ: le chlorure de *trans*-β-styrènesulfonyle
Rendement = 57%
¹H-RMN (400MHz, CDCl₃) : δ 1,0 (m, 4H), 1,1∼1,2 (m, 7H), 1,3 (m, 2H), 1,45 (m, 20H), 1,6 (m, 6H), 2,05 (m, 1H), 2,6 (bt, 4H), 3,5 (m, 1H), 3,8 (m, 1H), 3,85∼4,15 (m, 9H), 5,95 (bs, 1H), 6,5 (bt, 1H), 6,75 (d, 1H), 6,95 (bt, 1H), 7,35∼7,5 (m, 6H).

### 28) 4-[(10S)-3-{2-[1-(tert-butoxycarbonyl)-4-pipéridinyl]éthyl}-10-(éthoxycarbonyl)-4,7,12,12-tétraoxo-14-phényl-12λ⁶-thia-5,8,11-triazatétradec-1-yl]tétrahydro-1(2H)-pyridinecarboxylate de tert-butyle (composé 56)

Produit de départ : le produit de l'hydrogénation du composé 55
Rendement = 96%
¹H-RMN (400MHz, CDCl₃) : δ 1,0 (m, 4H), 1,15 (m, 4H), 1,25 (m, 5H), 1,4 (s, 20H), 1,55 (m, 6H), 2,05 (m, 1H), 2,6 (bt, 4H), 3,0∼3,3 (m, 3H), 3,55 (m, 1H), 3,75 (m, 1H), 3,9 (d, 2H), 4,05 (bs, 5H), 4,25 (d, 2H), 6,0 (bs, 1H), 6,55 (bt, 1H), 7,0 (bt, 1H), 7,1∼7,5 (m, 5H).

### 29) 4-[5-[1-(tert-butoxycarbonyl)-4-pipéridinyl]-3-{[(2-{[(2S)-3-éthoxy-3-oxo-2-({[3-(trifluorométhyl)phényl]sulfonyl}amino)propyl]amino}-2-oxoéthyl)amino]carbonyl}pentyl)tétrahydro-1(2H)-pyridinecarboxylate de tert-butyle (composé 57)

Produit de départ : le chlorure de 3-trifluorométhylphénylsulfonyle
Rendement = 77%
¹H-RMN (400MHz, CDCl₃) : δ 1,0 (m, 7H), 1,15 (m, 4H), 1,25 (m, 2H), 1,4 (s, 20H), 1,55 (m, 6H), 2,05 (m, 1H), 2,55 (bt, 4H), 3,5 (m, 1H), 3,65 (m, 1H), 3,85∼4,05 (m, 9H), 6,4 (bs, 1H), 6,55 (bt, 1H), 7,0 (bt, 1H), 7,6 (t, 1H), 7,75 (d, 1H), 7,95 (d, 1H), 8,05 (s, 1H).

### 30) 4-[5-[1-(tert-butoxycarbonyl)-4-pipéridinyl]-3-{[(2-{[(2S)-3-éthoxy-2-({[3-nitrophényl]sulfonyl}amino)-3-oxopropyl]amino}-2-oxoéthyl)amino]carbonyl}pentyl)tétrahydro-1(2H)-pyridinecarboxylate de tert-butyle (composé 58)

Produit de départ: le chlorure de 3-nitrophénylsulfonyle
Rendement = 55%
¹H-RMN (400MHz, CDCl₃) : δ 1,0 (m, 4H), 1,15(t, 3H), 1,2 (m, 4H), 1,35 (m, 2H), 1,45 (s, 20H), 1,6 (m, 6H), 2,1 (m, 1H), 2,65 (bt, 4H), 3,65 (m, 2H), 3,9∼4,2 (m, 9H), 6,65 (bt, 1H), 7,15 (t, 1H), 7,7 (t, 1H), 8,2 (d, 1H), 8,4 (d, 1H), 8,7 (s, 1H).

### 31) 4-[5-[1-(tert-butoxycarbonyl)-4-pipéridinyl]-3-{[(2-{[(2S)-3-éthoxy-2-({[3-méthoxyphényl]sulfonyl}amino)-3-oxopropyl]amino}-2-oxoéthyl)amino]carbonyl}pentyl)tétrahydro-1(2H)-pyridinecarboxylate de tert-butyle (composé 59)

Produit de départ : le chlorure de 4-méthoxyphénylsulfonyle
Rendement = 55%
¹H-RMN (400MHz, CDCl₃) : δ 1,0 (m, 4H), 1,15 (t, 3H), 1,2 (m, 4H), 1,35 (m, 2H), 1,45 (s, 20H), 1,65 (m, 6H), 2,05 (m, 1H), 2,65 (bt, 4H), 3,45 (m, 1H), 3,75 (m, 1H), 3,85 (s, 3H), 3,9∼4,1 (m, 9H), 5,9 (bs, 1H), 6,5 (bt, 1H), 6,85 (bt, 1H), 6,95 (d, 2H), 7,75 (d, 2H).

### 32) 4-[5-[1-(tert-butoxycarbonyl)-4-pipéridinyl]-3-({[2-({(2S)-3-éthoxy-3-oxo-2-[(8-quinolinylsulfonyl)amino]propyl}amino)-2-oxoéthyl]amino}carbonyl) pentyl]tétrahydro-1(2H)-pyridinecarboxylate de tert-butyle (composé 60)

Produit de départ : le chlorure de 8-quinolinylsulfonyle
Rendement = 55%
¹H-RMN (400MHz, CDCl₃) : δ 0,9 (t, 3H), 1,05 (m, 4H), 1,2 (m, 4H), 1,3 (m, 2H), 1,45 (m, 20H), 1,6 (m, 6H), 2,05 (m, 1H), 2,65 (bt, 4H), 3,5 (m, 1 H), 3,65∼4,2 (m, 10H), 6,35 (bt, 1H), 6,65 (bt, 1H), 7,2 (bs, 1H), 7,55 (q, 1H), 7,65 (t, 1H), 8,05 (d, 1H), 8,25 (d, 1H), 8,35 (d, 1H), 9,05 (d, 1H).

### 33) 4-{5-[1-(tert-butoxycarbonyl)-4-pipéridinyl]-3-[({2-[((2S)-2-{[(3,5-diméthyl-4-isoxazolyl)sulfonyl]amino}-3-éthoxy-3-oxopropyl)amino]-2-oxoéthyl}amino)carbonyl] pentyl}tétrahydro-1(2H)-pyridinecarboxylate de tert-butyle (composé 61)

Produit de départ : le chlorure de 3,5-diméthyl-4-isoxazolylsulfonyle
Rendement = 81 %
¹H-RMN (400MHz, CDCl₃) : δ 1,05 (m, 4H), 1,2 (m, 7H), 1,3 (m, 2H), 1,45 (m, 20H), 1,65 (m, 6H), 2,05 (m, 1H), 2,4 (s, 3H), 2,6 (s, 3H), 2,65 (bt, 4H), 3,5 (m, 1H), 3,7 (m, 1H), 3,85∼4,1 (m, 9H), 6,4 (bs, 1H), 6,55 (bt, 1H), 7,0 (bt, 1H).

### 34) 4-{5-[1-(tert-butoxycarbonyl)-4-pipéridinyl]-3-[({2-[((2S)-2-{([5-(diméthylamino)-1-naphtyl]sulfonyl)amino}-3-éthoxy-3-oxopropyl)amino]-2-oxoéthyl}amino)carbonyl] pentyl}tétrahydro-1(2H)-pyridinecarboxylate de tert-butyle (composé 62)

Produit de départ : le chlorure de 5-diméthylamino-1-naphtylsulfonyle
Rendement = 73%
¹H-RMN (400MHz, CDCl₃) : δ 0,9 (t, 3H), 1,15 (m, 4H), 1,3 (m, 2H), 1,45 (m, 20H), 1,6 (m, 6H), 2,05 (m, 1H), 2,6 (bt, 4H), 2,85 (s, 6H), 3,45 (m, 1H), 3,6 (m, 1H), 3,8 (m, 4H), 3,9∼4,1 (m, 5H), 6,15 (bs, 1H), 6,35 (bt, 1H), 6,6 (bt, 1H), 7,2 (d, 1H), 7,5 (t, 1H), 7,6 (t, 1H), 8,2 (d, 1H), 8,25 (d, 1H), 8,55 (d, 1H).

### 35) 4-{3-{[(2-{[(2S)-2-({[2-(acétylamino)-4-méthyl-1,3-thiazol-5-yl]sulfonyl}amino)-3-éthoxy-3-oxopropyl]amino}-2-oxoéthyl)amino]carbonyl}-5-[1-(tert-butoxycarbonyl)-4-pipéridinyl]pentyl}tétrahydro-1(2H)-pyridinecarboxylate de tert-butyle (composé 63)

Produit de départ : le chlorure de 2-(acétylamino)-4-méthyl-1,3-thiazol-5-ylsulfonyle
Rendement = 64%
¹H-RMN (400MHz, CDCl₃ : δ 1,05 (m, 4H), 1.15∼1,4 (m, 9H), 1,45 (s, 20H), 1,6 (bd, 6H), 2,1 (m, 1H), 2,3 (s, 3H), 2,45 (s, 3H), 2,65 (bt, 4H), 3,55 (m, 1H), 3,7 (m, 1H), 3,9∼4,2 (m, 9H), 6,85 (bt, 1H), 7,2 (bt, 1H).

### 36) 4-{5-[1-(tert-butoxycarbonyl)-4-pipéridinyl]-3-[({2-[((2S)-2-{([3-chloropropyl]sulfonyl)amino}-3-éthoxy-3-oxopropyl)amino]-2-oxoéthyl}amino)carbonyl] pentyl}tétrahydro-1(2H)-pyridinecarboxylate de tert-butyle (composé 64)

Produit de départ : le chlorure de 3-chloropropylsulfonyle
Rendement = 68%
¹H-RMN (400MHz, CDCl₃) : δ 1,05 (m, 4H), 1,2 (m, 4H), 1,35 (m, 5H), 1,45 (s, 20H), 1,6 (m, 6H), 2,05 (m, 1H), 2,3 (m, 2H), 2,65 (bt, 4H), 3,2 (t, 2H), 3,6 (m, 1H), 3,65 (t, 2H), 3,75 (m, 1H), 3,95 (d, 2H), 4,05 (bs, 4H), 4,25 (q, 3H), 6,1 (bd, 1H), 6,6 (bt, 1H), 7,1 (bt, 1H).

### 37) 4-{5-[1-(tert-butoxycarbonyl)-4-pipéridinyl]-3-[({2-[((2S)-2-{([4-méthoxy-1-naphtyl]sulfonyl)amino}-3-éthoxy-3-oxopropyl)amino]-2-oxoéthyl}amino)carbonyl] pentyl}tétrahydro-1(2H)-pyridinecarboxylate de tert-butyle (composé 65)

Produit de départ : le chlorure de 4-méthoxy-1-naphtylsulfonyle
Rendement = 71 %
¹H-RMN (400MHz, CDCl₃) : δ 0,9 (t, 3H), 1,05 (m, 4H), 1,15 (m, 4H), 1,35 (m, 2H), 1,45 (s, 20H), 1,6 (m, 6H), 2,05 (m, 1H), 2,65 (bt, 4H), 3,45 (m, 1H), 3,6 (m, 1H), 3,75-4,05 (m, 9H), 4,1 (s, 3H), 6,2 (d, 1H), 6,35 (bt, 1H), 6,65 (bt, 1H), 6,8 (d, 1H), 7,6 (t, 1H), 7,7 (t, 1H), 8,15 (d, 1H), 8,35 (d, 1H), 8,6 (d, 1H).

### 38) 4-{5-(1-(tert-butoxycarbonyl)-4-pipéridinyl]-3-[({2-[((2S)-2-{([6,7-diméthoxy-2-naphtyl]sulfonyl)amino}-3-éthoxy-3-oxopropyl)amino]-2-oxoéthyl}amino)carbonyl] pentyl}tétrahydro-1(2H)-pyridinecarboxylate de tert-butyle (composé 66)

Produit de départ : le chlorure de 6,7-diméthoxy-2-naphtylsulfonyle
Rendement = 63%
¹H-RMN (400MHz, CDCl₃) : δ 1,0 (t, 3H), 1,05 (m, 4H), 1,2 (m, 4H), 1,35 (m, 2H), 1,45 (s, 20H), 1,6 (m, 6H), 2,1 (m, 1H), 2,65 (bt, 4H), 3,45 (m, 1H), 3,75 (m, 1H), 3,8∼4,1 (m, 15H), 6,0 (d, 1H), 6,5 (bt, 1H), 6,9 (bt, 1H), 7,15 (s, 1H), 7,2 (s, 1H), 7,3 (s, 1H), 7,7 (d, 1H), 7,8 (d, 1H), 8,25 (s, 1H).

### 39) 4-{5-[1-(tert-butoxycarbonyl)-4-pipéridinyl]-3-{[(2-{[(2S)-2-({[2,4-diméthyl-1,3-thiazol-5-yl]sulfonyl}amino)-3-éthoxy-3-oxopropyl]amino}-2-oxoéthyl)amino]carbonyl}pentyl}tétrahydro-1(2H)-pyridinecarboxylate de tert-butyle (composé 67)

Produit de départ : le chlorure de 2,4-diméthyl-1,3-thiazol-5-ylsulfonyle
Rendement = 54%
¹H-RMN (400MHz, CDCl₃) : δ 1,05 (m, 4H), 1,2 (m, 7H), 1,35 (m, 2H), 1,45 (s, 20H), 1,6 (m, 6H), 2,1 (m, 1H), 2,6 (s, 3H), 2,7 (m, 7H), 3,55 (m, 1H), 3,7 (m, 1H), 3,85-4,15 (m, 9H), 6,35 (d, 1H), 6,5 (bt, 1H), 6,95 (bt, 1H).

### 40) 4-{5-[1-(tert-butoxycarbonyl)-4-pipéridinyl]-3-{[(2-{[(2S)-2-({[3,5-diméthyl-1H-pyrazol-4-yl]sulfonyl)amino)-3-éthoxy-3-oxopropyl]amino)-2-oxoéthyl)amino]carbonyl}pentyl}tétrahydro-1(2H)-pyridinecarboxylate, de tert-butyle (composé 68)

Produit de départ : le chlorure de 3,5-diméthyl-1H-pyrazol-4-ylsulfonyle
Rendement = 50%
¹H-RMN (400MHz, CDCl₃) : δ 1,05 (m, 4H), 1,2 (m, 7H), 1,3 (m, 2H), 1,45 (s, 20H), 1,65 (m, 6H), 2,1 (m, 1H), 2,4 (s, 6H), 2,65 (bt, 4H), 3,5 (m, 1H), 3,7 (m, 1H), 3,9 (bs, 2H), 4,05 (m, 7H), 6,4 (bd, 1H), 6,75 (bt, 1H), 7,15 (bs, 1H), 11,8 (bs, 1H).

### 41) 4-[5-[1-(tert-butoxycarbonyl)-4-pipéridinyl]-3-({[2-({(2S)-3-éthoxy-3-oxo-2-[(3-pyridinylsulfonyl)amino]propyl}amino)-2-oxoéthyl]amino}carbonyl) pentyl]tétrahydro-1(2H)-pyridinecarboxylate de tert-butyle (composé 69)

Produit de départ : le chlorure de 3-pyridinylsulfonyle
Rendement = 61%
¹H-RMN (400MHz, CDCl₃) : δ 1,05 (m, 4H), 1,15 (t, 3H), 1,2 (m, 4H), 1,35 (m, 2H), 1,45 (m, 20H), 1,65 (m, 6H), 2,1 (m, 1H), 2,65 (bt, 4H), 3,6 (m, 1H), 3,7 (m, 1H), 3,9∼4,15 (m, 9H), 6,65 (bt, 1H), 6,7 (d, 1H), 7,15 (bt, 1H), 7,45 (q, 1H), 8,15 (dd, 1H), 8,8 (d, 1H), 9,05 (s, 1H).

### 42) 4-{3-{[(2-{[(2S)-2-({1,3-benzodioxol-5-ylsulfonyl}amino)-3-éthoxy-3-oxopropyl]amino}-2-oxoéthyl)amino]carbonyl}-5-[1-(tert-butoxycarbonyl)-4-pipéridinyl]pentyl}tétrahydro-1(2H)-pyridinecarboxylate de tert-butyle (composé 70)

Produit de départ : le chlorure de 1,3-benzodioxol-5-ylsulfonyle
Rendement = 61 %
¹H-RMN (400MHz, CDCl₃) : δ 1,05 (m, 4H), 1,2 (m, 7H), 1,3 (m, 2H), 1,45 (s, 20H), 1,65 (m, 6H), 2,1 (m, 1H), 2,65 (bt, 4H), 3,5 (m, 1H), 3,7 (m, 1H), 3,85-4,1 (m, 9H), 6,05 (d, 1H), 6,1 (s, 2H), 6,55 (bt, 1H), 6,85 (d, 1H), 6,9 (bt, 1H), 7,25 (d, 1H), 7,4 (d, 1H).

### 43) 4-{5-[1-(tert-butoxycarbonyl)-4-pipéridinyl]-3-{[(2-{[(2S)-2-({2,3-dihydro-1,4-benzodioxin-6-ylsulfonyl}amino)-3-éthoxy-3-oxopropyl]amino}-2-oxoéthyl)amino]carbonyl}pentyl}tétrahydro-1(2H)-pyridinecarboxylate de tert-butyle (composé 71)

Produit de départ : le chlorure de 2,3-dihydro-1,4-benzodioxin-6-ylsulfonyle Rendement = 61%
¹H-RMN (400MHz, CDCl₃) : δ 1,05 (m, 4H), 1,2 (m, 7H), 1,35 (m, 2H), 1,45 (s, 20H), 1,65 (m, 6H), 2,1 (m, 1H), 2,65 (bt, 4H), 3,45 (m, 1H), 3,75 (m, 1H), 3,9∼4,1 (m, 9H), 4,3 (dd, 4H), 5,95 (bs, 1H), 6,5 (bt, 1H), 6,85 (d, 1H), 6,95 (d, 1H), 7,3 (dd, 1H), 7,35(d, 1H).

### 44) 4-{3-{[(2-{[(2S)-2-({1-benzothiophèn-2-ylsulfonyl}amino)-3-éthoxy-3-oxopropyl]amino}-2-oxoéthyl)amino]carbonyl}-5-[1-(tert-butoxycarbonyl)-4-pipéridinyl]pentyl}tétrahydro-1(2H)-pyridinecarboxylate de tert-butyle (composé 72)

Produit de départ : le chlorure de 1-benzothiophèn-2-ylsulfonyle
Rendement = 74%
¹H-RMN (400MHz, CDCl₃) : δ 0,95 (t, 3H), 1,05 (m, 4H), 1,2 (m, 4H), 1,3 (m, 2H), 1,45 (s, 20H), 1,6 (m, 6H), 2,1 (m, 1H), 2,65 (bt, 4H), 3,5 (m, 1H), 3,7 (m, 1H), 3,85 (m, 4H), 4,05 (m, 5H), 6,45 (bt, 2H), 6,9 (bt, 1H), 7,5 (m, 2H), 7,9 (d, 1H), 8,2 (d, 1H), 8,25 (s, 1H).

### 45) 4-{5-[1-(tert-butoxycarbonyl)-4-pipéridinyl]-3-{[(2-{[(2S)-2-({[2,5-diméthyl-3-furyl]sulfonyl}amino)-3-éthoxy-3-oxopropyl]amino}-2-oxoéthyl)amino]carbonyl}pentyl}tétrahydro-1(2H)-pyridinecarboxylate de tert-butyle (composé 73)

Produit de départ : le chlorure de 2,5-diméthyl-3-furylsulfonyle
Rendement = 78%
¹H-RMN (400MHz, CDCl₃) : δ 1,05 (m, 4H), 1,2 (m, 7H), 1,3 (m, 2H), 1,45 (s, 20H), 1,6 (m, 6H), 2,1 (m, 1H), 2,25 (s, 3H), 2,5 (s, 3H), 2,65 (bt, 4H), 3,45 (m, 1H), 3,75 (m, 1H), 3,85∼4,15 (m, 9H), 5,9 (bs, 1H), 6,1 (s, 1H), 6,45 (bt, 1H), 6,85 (bt, 1H).

### 46) 4-{5-[1-(tert-butoxycarbonyl)-4-pipéridinyl]-3-{[(2-{[(2S)-2-({[4-cyclohexylphényl]sulfonyl}amino)-3-éthoxy-3-oxopropyl]amino}-2-oxoéthyl)amino]carbonyl}pentyl}tétrahydro-1(2H)-pyridinecarboxylate de tert-butyle (composé 74)

Produit de départ : le chlorure de 4-cyclohexylphénylsulfonyle
Rendement = 64%
¹H-RMN (400MHz, CDCl₃) : δ 1,1 (m, 7H), 1,15∼1,5 (m, 30H), 1,6 (m, 6H), 1,8 (m, 6H), 2,1 (m, 1H), 2,65 (m, 5H), 3,45 (m, 1H), 3,75 (m, 1H), 3,85-4,15 (m, 9H), 5,9 (bs, 1H), 6,45 (bt, 1H), 6,85 (bt, 1H), 7,35 (d, 2H), 7,75 (d, 2H).

### 47) 4-[5-[1-(tert-butoxycarbonyl)-4-pipéridinyl]-3-{[(2-{[(2S)-3-éthoxy-2-({[4-fluoro-1-naphtyl]sulfonyl}amino)-3-oxopropyl]amino}-2-oxoéthyl)amino]carbonyl}pentyl)tétrahydro-1(2H)-pyridinecarboxylate de tert-butyle (composé 75)

Produit de départ : le chlorure de 4-fluoro-1-naphtylsulfonyle
Rendement = 64%
¹H-RMN (400MHz, CDCl₃) : δ 0,9 (t, 3H), 1,05 (m, 4H), 1,2 (m, 4H), 1,35 (m, 2H), 1,45 (s, 20H), 1,6 (m, 6H), 2,1 (m, 1H), 2,65 (bt, 4H), 3,5 (m, 1H), 3,65 (m, 1H), 3,8 (q, 2H), 3,85 (bd, 2H), 4,05 (m, 5H), 6,3 (bs, 1H), 6,45 (bt, 1H), 6,8 (bt, 1H), 7,2 (t, 1H), 7,7 (t, 1H), 7,75 (t, 1H), 8,23 (t, 1H), 8,65 (d, 1H).

### 48) 4-[5-[1-(tert-butoxycarbonyl)-4-pipéridinyl]-3-{[(2-{[(2S)-3-éthoxy-2-({[4-chloro-1-naphtyl]sulfonyl}amino)-3-oxopropyl]amino}-2-oxoéthyl)amino]carbonyl}pentyl)tétrahydro-1(2H)-pyridinecarboxylate de tert-butyle (composé 76)

Produit de départ : le chlorure de 4-chloro-1-naphtylsulfonyle
Rendement = 63%
¹H-RMN (400MHz, CDCl₃) : δ 0,9 (t, 3H), 1,1 (m, 4H), 1,2 (m, 4H), 1,35 (m, 2H), 1,45 (s, 20H), 1,6 (m, 6H), 2,1 (m, 1H), 2,65 (bt, 4H), 3,5 (m, 1H), 3,6 (m, 1H), 3,8 (q, 2H), 3,85 (bd, 2H), 3,95∼4,15 (m, 5H), 6,4 (bt, 2H), 6,8( bt, 1H), 7,65 (d, 1H), 7,75 (m, 2H), 8,4 (d, 1H), 8,7 (d, 1H).

### 49) 4-{5-[1-(tert-butoxycarbonyl)-4-pipéridinyl]-3-{[(2-{[(2S)-2-({[2,3-dihydro-1-benzofuran-5-yl]sulfonyl}amino)-3-éthoxy-3-oxopropyl]amino}-2-oxoéthyl)amino]carbonyl}pentyl}tétrahydro-1(2H)-pyridinecarboxylate de tert-butyle (composé 77)

Produit de départ : le chlorure de 2,3-dihydro-1-benzofuran-5-ylsulfonyle Rendement = 74%
¹H-RMN (400MHz, CDCl₃) : δ 1,05 (m, 4H), 1,2 (m, 7H), 1,35 (m, 2H), 1,45 (s, 20H), 1,6 (m, 6H), 2,1 (m, 1 H), 2,65 (bt, 4H), 3,25 (t, 2H), 3,45 (m, 1H), 3,75 (m, 1H), 3,85∼4,1 (m, 9H), 4,65 (t, 2H), 5,8 (d, 1H), 6,45 (bt, 1H), 6,8 (d, 2H), 7,6 (d, 1H), 7,65 (s, 1H).

### 50) 4-[5-[1-(tert-butoxycarbonyl)-4-pipéridinyl]-3-({[2-({(2S)-3-éthoxy-3-oxo-2-{[4-(2-thiényl)phénylsulfonyl]amino}propyl}amino)-2-oxoéthyl]amino}carbonyl) pentyl]tétrahydro-1(2H)-pyridinecarboxylate de tert-butyle (composé 78)

Produit de départ : le chlorure de 4-(2-thiényl)-phénylsulfonyle
Rendement = 78%
¹H-RMN (400MHz, CDCl₃) : δ 1.1 (m, 7H), 1,2 (m, 4H), 1,35 (m, 2H), 1,45 (s, 20H), 1,6 (m, 6H), 2,1 (m, 1H), 2,65 (bq, 4H), 3,5 (m, 1H), 3,75 (m, 1H), 3,9∼4,1 (m, 9H), 6,1 (d, 1H), 6,5 (bt, 1H), 6,9 (bt, 1H), 7,1 (t, 1H), 7,4 (dd, 2H), 7,7 (d, 2H), 7,85 (d, 2H).

### 51) 4-[5-[1-(tert-butoxycarbonyl)-4-pipéridinyl)-3-({[2-({(2S)-3-éthoxy-3-oxo-2-{[2-(2-thiényl)phénylsulfonyl]amino}propyl}amino)-2 tert-butyle (composé 79)

Produit de départ : le chlorure de 2-(2-thiényl)-phénylsulfonyle
Rendement = 63%
¹H-RMN (400MHz, CDCl₃) : δ 1,05 (m, 4H), 1,2 (m, 7H), 1,3 (m, 2H), 1,45 (s, 20H), 1,65 (m, 6H), 2,05 (m, 1H), 2,65 (bt, 4H), 3,5 (m, 2H), 3,8∼4,1 (m, 9H), 5,3 (d, 1H), 6,25 (bt, 1H), 6,4 (bt, 1H), 7,15 (t, 1H), 7,5 (m, 4H), 7,6 (t, 1H), 8,1 (d, 1 H).

### 52) 4-[5-[1-(tert-butoxycarbonyl)-4-pipéridinyl]-3-({[2-({(2S)-3-éthoxy-3-oxo-2-{[4-(2-furyl)phénylsulfonyl]amino}propyl}amino)-2-oxoéthyl]amino}carbonyl) pentyl]tétrahydro-1(2H)-pyridinecarboxylate de tert-butyle (composé 80)

Produit de départ : le chlorure de 4-(2-furyl)-phénylsulfonyle
Rendement = 62%
¹H-RMN (400MHz, CDCl₃) : δ 1,1 (m, 7H), 1,2 (m, 4H), 1,35 (m, 2H), 1,45 (s, 20H), 1,65 (m, 6H), 2,1 (m, 1H), 2,65 (bt, 4H), 3,5 (m, 1H), 3,75 (m, 1H), 3,85∼4,1 (m, 9H), 6,05 (bs, 1H), 6,5 (bt, 1H), 6,55 (d, 1H), 7,5 (s, 1H), 7,75 (d, 2H), 7,8 (d, 2H).

### 53) 4-{3-{[(2-{[(2S)-2-({1-benzofuran-2-ylsulfonyl}amino)-3-éthoxy-3-oxopropyl]amino}-2-oxoéthyl)amino]carbonyl}-5-[1-(tert-butoxycarbonyl)-4-pipéridinyl]pentyl}tétrahydro-1(2H)-pyridinecarboxylate de tert-butyle (composé 81)

Produit de départ : le chlorure de 1-benzofuran-2-ylsulfonyle
Rendement = 52%
¹H-RMN (400MHz, CDCl₃) : δ 1,1 (m, 7H), 1,2 (m, 4H), 1,3 (m, 2H), 1,45 (s, 20H), 1,6 (m, 6H), 2,1 (m, 1H), 2,65 (bt, 4H), 3,65 (m, 1H), 3,75 (m, 1H), 3,9∼4,15 (m, 8H), 4,25 (m, 1H), 6,45 (bt, 1H), 6,9 (bt, 1H), 7,35 (m, 2H), 7,48 (t, 1H), 7,65 (d, 1H), 7,7 (d, 1H).

### 54) 4-[5-[7-(tert-butoxycarbonyl)-4-piperidinyl]-3-({[2-({(2S)-3-ethoxy-3-oxo-2-[(2-naphtylméthylsulfonyl)amino]propyl}amino)-2-oxoéthyl]amino}carbonyl) pentyl]tétrahydro-1(2H)-pyridinecarboxylate de tert-butyle (composé 82)

Produit de départ : le chlorure de 2-naphtylméthanesulfonyle
Rendement = 61 %
¹H-RMN (400MHz, CDCl₃) : δ 1,05 (m, 4H), 1,1 (t, 3H), 1,2 (m, 4H), 1,3 (m, 2H), 1,45 (s, 20H), 1,6 (m, 6H), 2,05 (m, 1H), 2,65 (bt, 4H), 3,4 (m, 1H), 3,5 (m, 1H), 3,75∼4,1 (m, 9H), 4,4 (q, 2H), 5,7 (bs, 1H), 6,4 (bt, 1H), 6,7 (bt, 1H), 7,5 (m, 3H), 7,8 (m, 4H).

### 55) 4-{5-[1-(tert-butoxycarbonyl)-4-pipéridinyl]-3-{[(2-{[(2S)-2-({[2,3-dihydro-1H-indèn-5-yl]sulfonyl}amino)-3-éthoxy-3-oxopropyl]amino}-2-oxoéthyl)amino]carbonyl}pentyl}tétrahydro-1(2H)-pyridinecarboxylate de tert-butyle (composé 83)

Produit de départ : le chlorure de 5-indanesulfonyle
Rendement = 62%
¹H-RMN (400MHz, CDCl₃) : δ 1,05 (m, 4H), 1,1 (t, 3H), 1,2 (m, 4H), 1,35 (m, 2H), 1,45 (s, 20H), 1,6 (m, 6H), 2,05 (m, 1H), 2,1 (t, 2H), 2,65 (bt, 4H), 2,95 (t, 4H), 3,45 (m, 1H), 3,75 (m, 1H), 3,85∼4,1 (m, 9H), 5,85 (d, 1H), 6,45 (bt, 1H), 6,8 (bt, 1H), 7,3 (d, 1 H), 7,6 (d, 1H), 7,65 (s, 1H).

### 56) 4-[5-[1-(tert-butoxycarbonyl)-4-pipéridinyl]-3-({[2-({(2S)-3-éthoxy-3-oxo-2-{[(5-phényl-2-thiényl)sulfonyl]amino}propyl}amino)-2-oxoéthyl]amino}carbonyl) pentyl]tétrahydro-1(2H)-pyridinecarboxylate de tert-butyle (composé 84)

Produit de départ : le chlorure de 5-phényl-2-thiophènesulfonyle
Rendement = 60%
¹H-RMN (400MHz, CDCl₃) : δ 1,05 (m, 4H), 1,15 (t, 3H), 1,2 (m, 4H), 1,35 (m, 2H), 1,45 (s, 20H), 1,6 (m, 6H), 2,05 (m, 1H), 2,65 (bt, 4H), 3,55 (m, 1H), 3,8 (m, 1H), 3,9∼4,15 (m, 9H), 6,4 (bt, 1H), 6,8 (bt, 1H), 7,25 (d, 1H), 7,4 (m, 3H), 7,55 (m, 3H).

### 57) 4-[5-[1-(tert-butoxycarbonyl)-4-pipéridinyl]-3-({[2-({(2S)-3-éthoxy-3-oxo-2-[(5,6,7,8-tétrahydro-2-naphthènylsulfonyl)amino]propyl}amino)-2-oxoéthyl]amino}carbonyl) pentyl]tétrahydro-1(2H)-pyridinecarboxylate de tert-butyle (composé 85)

Produit de départ : le chlorure de 5,6,7,8-tétrahydro-2-naphthalènesulfonyle
Rendement =12%
¹H-RMN (400MHz, CDCl₃) : δ 1,05 (m, 4H), 1,1 (t, 3H), 1,2 (m, 4H), 1,3 (m, 2H), 1,45 (s, 20H), 1,6 (m, 6H), 1,8 (bs, 4H), 2,1 (m, 1H), 2,65 (bt, 4H), 2,8 (bs, 4H), 3,45 (m, 1H), 3,75 (m, 1H), 3,85∼4,15 (m, 9H), 5,9 (bs, 1H), 6,5 (bt, 1H), 6,85 (bt, 1H), 7,2 (d, 1H), 7,49 (d, 1H), 7,65 (s, 1H).

### 58) 4-[(10S, 13E)-3-{2-[1-(tert-butoxycarbonyl)-4-pipéridinyl]éthyl}-10-(éthoxycarbonyl)-4,7,12,12-tétraoxo-12λ⁶-thia-5,8,11-triaza-13-heptadècen-1-yl]tétrahydro-1(2H)-pyridinecarboxylate de tert-butyle (composé 86)

Produit de départ : le chlorure de (E)-1-pentènylsulfonyle
Rendement = 21%
¹H-RMN (400MHz, CDCl₃) δ 0,9 (t, 3H), 1,05 (m, 4H), 1,2 (m, 4H), 1,27 (t, 3H), 1,30 (m, 2H), 1,45 (s, 20H), 1,6 (m, 6H), 2,05 (m, 1H), 2,2 (q, 2H), 2,65 (bt, 4H), 3,45 (m, 1H), 3,75 (m, 1H), 3,85-4,10 (m, 7H), 4,2 (q, 2H), 5,65 (d, 1H), 6,15 (d, 1H), 6,45 (bt, 1H), 6,75 (dt, 1H), 6,8 (bt, 1H).

### 59) 4-[5-[1-(tert-butoxycarbonyl)-4-pipéridinyl]-3-({[3-({(3S)-3-éthoxy-2-[(2-naphtylsulfonyl)amino]-3-oxopropyl}amino)-3-oxopropyl]amino}carbonyl)pentyl]tétrahydro-1(2H)-pyridinecarboxylate de tert-butyle (composé 87)

Produits de départ : le composé 38 et le chlorure de 2-naphtylsulfonyle
Rendement = 76%
¹H-RMN (400MHz, CDCl₃) : δ 0,95 (t, 3H), 1,05 (m, 4H), 1,2 (m, 4H), 1,30 (m, 2H), 1,45 (s, 20H), 1,6 (m, 6H), 2,0 (m, 1H), 2,4 (m, 2H), 2,65 (bq, 4H), 3,45 (m, 1H), 3,55 (m, 2H), 3,7 (m, 1H), 3,85 (q, 2H), 4,0 (bs, 4H), 4,1 (bs, 1H), 6,0 (bd, 1H), 6,75 (m, 2H), 7,65 (m, 2H), 7,8 (d, 1H), 7,9 (d, 1H), 7,95 (d, 2H), 8,4 (s, 1H).

### 60) 4-[5-[1-(tert-butoxycarbonyl)-4-pipéridinyl]-3-({[3-({(3S)-3-éthoxy-3-oxo-2-[(phénylsulfonyl)amino]propyl}amino)-3-oxopropyl]amino}carbonyl) pentyl]tétrahydro-1(2H)-pyridinecarboxylate de tert-butyle (composé 88)

Produits de départ : le composé 38 et le chlorure de benzènesulfonyle
Rendement = 76%
¹H-RMN (400MHz, CDCl₃) : δ 1,05 (m, 4H), 1,1 (t, 3H), 1,2 (m, 4H), 1,30 (m, 2H), 1,45 (s, 20H), 1,6 (m, 6H), 2,0 (m, 1H), 2,4 (t, 2H), 2,65 (bt, 4H), 3,4 (m, 1H), 3,55 (m, 2H), 3,7 (m, 1H), 4,0 (q, 2H), 4,05 (bs, 4H), 5,9 (d, 1H), 6,5 (m, 2H), 7,5 (t, 2H), 7,6 (d, 1H), 7,85 (d, 1H)

### 61) 4-[(10S)-3-{2-[1-(tert-butoxycarbonyl)-4-pipéridinyl]éthyl}-13-(7,7-diméthyl-2-oxobicyclo[2.2.1]hept-1-yl)-10-(éthoxycarbonyl)-4,7,12,12-tétraoxo-12λ⁶-thia-5,8,11-triazatridec-1-yl]tétrahydro-1(2H)-pyridinecarboxylate de tert-butyle (composé 89)

En suivant le mode opératoire décrit pour le composé 29, à partir du chlorhydrate de (2S)-3-amino-2-({[(7,7-diméthyl-2-oxobicyclo[2.2.1]hept-1-yl)méthyl]sulfonyl)amino)propanoate d'éthyle et du composé 4.
Rendement = 78%
¹H-RMN (400MHz, CDCl₃) : δ 0,90 (s, 3H), 1,00 (s, 3H), 1,05 (m, 4H), 1,20 (m, 4H), 1,30 (t, 5H), 1,45 (s, 20H), 1,55 (m, 6H), 1,95 (t, 3H), 2,05 (m, 2H), 2,15 (t, 1H), 2,20 (m, 1H), 2,40 (m, 1H), 2,65 (bt, 4H), 3,00 (d, 1H), 3,50 (m, 2H), 3,80 (m, 1H), 3,95 (dq, 2H), 4,05 (bs, 4H), 4,20 (q, 2H), 4,30 (m, 1H), 6,40 (t, 1H), 6,50 (d, 1H), 6,70 (t, 1H).

### 62) Acide (2R)-3-({2-[(4-[1-(tert-butoxycarbonyl)-4-pipéridinyl]-2-{2-[1-(tert-butoxycarbonyl)-4-pipéridinyl]éthyl}butanoyl)amino]acétyl}amino)-2-[(2-naphtylsulfonyl)amino]propanoïque (composé 90)

A une solution d'acide 2-[(4-[1-(*tert*-butoxycarbonyl)-4-pipéridinyl]-2-{2-[1-(*tert*-butoxycarbonyl)-4-pipéridinyl]éthyl}butanoyl)amino]acétique (composé 4) (10,8 g, 20 mmol) dans 200 ml de THF et de N-méthylmorpholine (5 g, 49,5 mmol), on ajoute du chloroformiate d'isobutyle (3,3 g, 24,2 mmol) à température ambiante. On obtient une suspension. Après 20 minutes d'agitation, on additionne, à 0°C, un mélange d'acide (2R)-3-amino-2-[(2-naphtylsulfonyl)amino]propanoïque (8 g, 25 mmol) et de l'eau (80 ml). L'agitation est maintenue à 0∼5°C pendant 30 minutes puis à température ambiante pendant 18 heures. On évapore le THF et acidifie la solution aqueuse à pH 2 avec de l'acide chlorhydrique 1N. On extrait à l'éther. Les extraits sont lavés à l'eau, séchés sur sulfate de sodium, évaporés pour donner le produit brut qui est purifié par flash-chromatographie (dichlorométhane/méthanol/acide acétique, 10/0,5/0,5) pour donner 8,3 g d'un solide beige.
Rendement = 51 %
¹H-RMN (400MHz, CDCl₃) : δ 1,0 (m, 4H), 1,15 (m, 4H), 1,25 (m, 2H), 1,4 (s, 20H), 1,55 (bd, 6H), 2,1 (m, 1H), 2,6 (bq, 4H), 3,6 (m, 1H), 3,75 (m, 1H), 3,8∼4,1 (m, 7H), 6,6 (d, 1H), 6,95 (bt, 1H), 7,25 (bt, 1H), 7,6 (m, 2H), 7,85 (t, 2H), 7,95 (t, 2H), 8,45 (s, 1H).

### D - Préparation des composés de formule Ib selon Ia voie a₂: réaction d'un acide de formule IV avec une amine de formule V

### 63) 4-[5-[1-(tert-butoxycarbonyl)-4-pipéridinyl]-3-({[2-({3-éthoxy-2-[(2-naphtylsulfonyl)amino]-3-oxopropyl}amino)-2-oxo-1-phényléthyl]amino}carbonyl)pentyl]tétrahydro-1(2H)-pyridinecarboxylate de tert-butyle (composé 91)

A une solution du fluorure de 4-[1-(*tert*-butoxycarbonyl)-4-pipéridinyl]-2-{2-[1-(*tert*-butoxycarbonyl)-4-pipéridinyl]éthyl}butanoyle (ref. la synthèse B-1) (1,55 g, 3 mmol) dans 50 ml de dichlorométhane, on ajoute du chlorhydrate de 3-[(2-amino-2-phényléthanoyl)amino]-2-[(2-naphtylsulfonyl)amino]propanoate d'éthyle (1,7 g, 3 mmol) et de la diisopropyléthylamine (0,8 g, 6,2 mmol) à température ambiante. Après 3 heures d'agitation, on ajoute de l'eau. La phase organique est lavée à l'eau, séchée sur sulfate de sodium, évaporée pour donner le produit brut qui est purifié par flash-chromatographie (dichlorométhane/méthanol 20/1) pour donner 1,2 g d'un solide blanc.
Rendement = 44%
¹H-RMN (400MHz, CDCl₃) : δ 0,95 (m, 3H), 1,00 (m, 4H), 1,20 (m, 4H), 1,40 (m, 2H), 1,47 (s, 20H), 1,60 (m, 6H), 2,05 (m, 1H), 2,60 (bd, 4H), 3,50 (m, 1H), 3,58 (m, 1H), 3,65 (m, 1H), 3,80 (m, 2H), 3,99 (m, 4H), 5,45 (m, 1H), 5,75 (dd, 1H), 6,40 (dt, 1H), 6,85 (dd, 1H), 7,35 (m, 5H), 7,52 (m, 2H), 7,75 (d, 1H), 7,95 (m, 3H), 8,37 (d, 1H).

Cette méthode a été utilisée pour préparer le composé suivant :

### 64) 4-[5-[1-(tert-butoxycarbonyl)-4-pipéridinyl]-3-({[2-({3-éthoxy-2-[(2-naphtylsulfonyl)amino]-3-oxopropyl}amino)-1-méthyl-2-oxoéthyl]amino}carbonyl)pentyl]tétrahydro-1(2H)-pyridinecarboxylate de tert-butyle (composé 92)

Produit de départ : le chlorhydrate 3-[(2-aminopropanoyl)amino]-2-[(2-naphtylsulfonyl)amino]propanoate d'éthyle
Rendement = 71 %
¹H-RMN (400MHz, CDCl₃) : δ 0,95 (t, 3H), 1,05 (m, 4H), 1,20 (m, 4H), 1,30 (d, 5H), 1,45 (s, 20H), 1,60 (m, 6H), 2,00 (m, 1H), 2,65 (bs, 4H), 3,50 (m, 1H), 3,70 (m, 1H), 3,88 (m, 2H), 4,08 (m, 4H), 4,50 (m, 1H), 6,10 (bd, 1H), 6,30 (d, 1H), 6,90 (t, 1H), 7,65 (m, 2H), 7,80 (d, 1H), 7,90 (d, 1H), 8,00 (d, 2H), 8,40 (s, 1H).

### EXEMPLE 1 :

### Dichlorhydrate du 3-(1,3-benzodioxol-5-yl)-3-{[2-({4-(4-pipéridinyl)-2-[2-(4-pipéridinyl)éthyl]butanoyl}amino)acétyl]amino}propanoate d'éthyle (CRL42725)

Le 4-{3-{[1-(1,3-benzodioxol-5-yl)-3-éthoxy-3-oxopropyl]amino}-2-oxoéthyl)amino]carbonyl}-5-[1-(*tert*-butoxycarbonyl)-4-pipéridinyl]pentyl} tétrahydro-1(2H)-pyridinecarboxylate de *tert*-butyle (composé 9) (3,3 g, 4,35 mmol) est solubilisé dans 5 ml de dioxane, ensuite 10 ml d'une solution de dioxane-acide chlorhydrique 4 N sont ajoutés. On maintient l'agitation à température ambiante pendant 20 minutes puis décante le dioxane. On rajoute de l'éther et décante à nouveau puis évapore à sec. Une poudre blanche est obtenue qui est solubilisée dans environ 150 ml d'eau puis filtrée, le filtrat est lyophilisé pour donner 2,4 g d'un solide blanc.
Rendement = 87%
MS (ES): *m*/*z* 559 (M + H)⁺
¹H-RMN (400MHz, CD₃OD) : δ 1,1 (m, 7H), 1,25 (m, 6H), 1,45 (m, 4H), 1,75 (m, 4H), 2,15 (m, 1H), 2,75 (m, 6H), 3,2 (m, 4H), 3,62 (m, 2H), 4,0 (q, 2H), 5,12 (q, 1H), 6,0 (s, 2H), 6,8 (dd, 2H), 6,95 (s, 1H), 8,1 (t, 1H), 8,45 (d, 1H), 8,8 (bs, 2H), 9,1 (bs, 2H).

La méthode décrite dans l'exemple 1 a été utilisée pour préparer les composés suivants :

### EXEMPLE 2 :

### Dichlorhydrate du 3-[3-(2-éthoxy-2-oxoéthoxy)phényl]-3-{[2-({4-(4-pipéridinyl)-2-[2-(4-pipéridinyl)éthyl]butanoyl}amino) acétyl]amino} propanoate d'éthyle (CRL42640)

Produit de départ : le composé 13
Rendement = 92%
¹H-RMN (400MHz, CD₃OD) : δ 1,3 (t, 3H), 1,45 (m, 13H), 1,7 (m, 4H), 1,95 (m, 4H), 2,35 (m, 1H), 3,0 (m, 6H), 3,4 (m, 4H), 3,75 (s, 2H), 3,95 (s, 2H), 4,18 (q, 2H), 4,35 (q, 2H), 4,8 (s, 2H), 5,42 (t, 1H), 6,92 (d, 1H), 7,05 (m, 2H), 7,35 (t, 1H).

### EXEMPLE 3 :

### Dichlorhydrate du 3-[3-(3-méthoxyphényl)]-3-{[2-({4-(4-pipéridinyl)-2-[2-(4-pipéridinyl)éthyl]butanoyl}amino)acétyl]amino}propanoate d'éthyle (CRL42661)

Produit de départ : le composé 14
Rendement = 93%
¹H-RMN (400MHz, CD₃OD) : δ 1,3 (t, 3H), 1,5 (m, 17H), 2,0 (m, 4H), 2,33 (m, 1H), 3,0 (m, 6H), 3,4 (m, 4H), 3,9 (s, 3H), 3,98 (s, 2H), 4,2 (m, 2H), 5,42 (t, 1H), 6,95 (m, 2H), 7,05 (bs, 2H), 7,32 (t, 1H).

### EXEMPLE 4 :

### Dichlorhydrate de (2S)-2-[(2-naphtylsulfonyl)amino]-3-{[2-({4-(4-pipéridinyl)-2-[2-(4-pipéridinyl)éthyl]butanoyl}amino) acétyl]amino}propanoate d'éthyle (CRL42968)

Produit de départ : le composé 41
Rendement = 100%
[α]_{D} -16,5 (C = 0,97, H₂O)
MS (ES) : *m*/*z* 644 (M + H)⁺
¹H-RMN (400MHz, DMSO-d₆) : δ 0,85 (t, 3H), 1,1 (m, 4H), 1,25 (m, 6H), 1,45 (m, 4H), 1,7 (bd, 4H), 2,15 (m, 1H), 2,75 (bq, 4H), 3,2 (m, 5H), 3,3 (m, 1H), 3,6 (m, 4H), 3,95 (m, 1H), 7,7 (m, 2H), 7,8 (d, 1H), 8,0∼8,2 (m, 5H), 8,4 (s, 1H), 8,55 (d, 1H), 8,85 (bd, 2H), 9,1 (bd, 2H).

### EXEMPLE 5

### Dichlorhydrate de l'acide 3-(1,3-benzodioxol-5-yl)-3-{[2-({4-(4-pipéridinyl)-2-[2-(4-pipéridinyl)éthyl]butanoyl}amino)acétyl]amino}propanoïque (CRL42630)

A une solution de 4-{3-{[1-{1,3-benzodioxol-5-yl)-3-éthoxy-3-oxopropyl]amino}-2-oxoéthyl)amino]carbonyl}-5-*[1*-*(tert*-butoxycarbonyl)-4-pipéridinyl]pentyl}tétrahydro-1(2H)-pyridinecarboxylate de *tert*-butyle (composé 9) (7,6 g, 10 mmol) dans 80 ml de tétrahydrofuranne et 20 ml d'eau, on ajoute 1 g (23,8 mmol) d' hydroxyde de lithium monohydraté. Après 4 heures à température ambiante, le solvant organique est évaporé. On ajoute de l'eau, acidifie à pH 2, puis extrait à l'acétate d'éthyle. Les extraits sont lavés à l'eau, séchés sur sulfate de sodium. Le filtrat est évaporé pour donner 6,3 g d'acide.

L'acide ainsi obtenu est solubilisé dans 10 ml d'acétate d'éthyle, puis on ajoute 50 ml d'une solution d'acétate d'éthyle-acide chlorhydrique 3 N. L'agitation est maintenue pendant 30 minutes à température ambiante. Le mélange est décanté. Après addition d'eau (environ 200 ml), puis lyophilisation, 4,7g d'un solide blanc sont obtenus.
Rendement = 78%
MS (ES) : *m*/*z* 531 (M + H)⁺
¹H-RMN (400MHz, CD₃OD) : δ 1,5 (m, 12H), 1,95 (m, 4H), 2,25 (m, 1H), 2,75 (bs, 2H), 2,9 (m, 4H), 3,3 (m, 4H), 3,85 (s, 2H), 5,29 (bs, 1H), 5,95 (s, 2H), 6,8 (d, 2H), 6,85 (m, 3H).

La méthode décrite dans l'exemple 5 a été utilisée pour préparer les composés suivants :

### EXEMPLE 6 :

### Dichlorhydrate de l'acide 3-(4-isopropylphényl)-3-{[2-({4-(4-pipéridinyl)-2-[2-(4-pipéridinyl)éthyl]butanoyl}amino)acétyl]amino}propanoïque (CRL42548)

Produit de départ : le composé 10
Rendement = 98%
MS-Cl : *m*/*z* 529 (M + H)⁺
¹H-RMN (400MHz, CD₃OD) : δ 1,5 (m, 16H), 1,65 (m, 4H), 1,95 (m, 5H), 2,35 (m, 1H), 3,0 (m, 6H), 3,4 (m, 4H), 3,95 (m, 2H), 5,4 (t, 1H), 7,25 (d, 2H), 7,38 (d, 2H).

### EXEMPLE 7 :

### Dichlorhydrate de l'acide 3-(4-méthoxyphényl)-3-{[2-({4-(4-pipéridinyl)-2-[2-(4-pipéridinyl)éthyl]butanoyl}amino)acétyl]amino}propanoïque (CRL42549)

Produit de départ : le composé 11
Rendement = 98%
MS-Cl: *m*/*z* 517 (M + H)⁺
¹H-RMN (400MHz, CD₃OD): δ 1,5 (m, 14H), 1,95 (m, 4H), 2,16 (m, 1H), 2,9 (dq, 2H), 3,0 (m, 4H), 3,4 (m, 4H), 3,85 (s, 3H), 3,95 (s, 2H), 5,35 (t, 1H), 6,92(d, 2H), 7,35 (d, 2H).

### EXEMPLE 8 :

### Dichlorhydrate de l'acide 3-(3,4-diméthoxyphényl)-3-{[2-({4-(4-pipéridinyl)-2-[2-(4-pipéridinyl)éthyl]butanoyl}amino)acétyl]amino}propanoïque (CRL42590)

Produit de départ : le composé 12
Rendement = 80%
MS-CI: *m*/*z*: 547 (M + H)⁺
¹H-RMN (400MHz, CD₃OD) δ 1,15∼1,65 (m, 14H), 1,8 (m, 4H), 2,25 (m, 1H), 2,85 (m, 6H), 3,3 (bs, 4H), 3,78 (s, 3H), 3,8 (s, 3H), 3,85 (m, 2H), 5,25 (t, 1H), 6,85 (d, 2H), 6,95 (d, 1H).

### EXEMPLE 9 :

### Dichlorhydrate de l'acide 3-[(3-carboxyméthoxy)phényl]-3-{[2-({4-(4-pipéridinyl)-2-[2-(4-pipéridinyl)éthyl]butanoyl}amino)acétyl]amino} propanoïque (CRL42639)

Produit de départ : le composé 13
Rendement = 79%
¹H-RMN (400MHz, CD₃OD) : δ 1,2∼1,65 (m, 14H), 1,85 (m, 4H), 2,25 (m, 1H), 2,7∼3,0 (m, 6H), 3,3 (bs, 4H), 3,78 (d, 2H), 4,68 (s, 2H), 5,3 (t, 1H), 6,82 (d, 1H), 6,95 (m, 2H), 7,25 (t, 1H).

### EXEMPLE 10 :

### Dichlorhydrate de l'acide 3-(3-méthoxyphényl)-3-{[2-({4-(4-pipéridinyl)-2-[2-(4-pipéridinyl)éthyl]butanoyl}amino)acétyl]amino}propanoïque (CRL42660)

Produit de départ : le composé 14
Rendement = 81%
MS (ES) : *m*/*z* 517 (M + H)⁺
¹H-RMN (400MHz, CD₃OD) : δ 1,4 (m, 8H), 1,55 (m, 2H), 1,68 (m, 4H), 1,95 (bs, 4H), 2,3 (m, 1H), 2,9 (dd, 2H), 3,0 (m, 4H), 3,4 (m, 4H), 3,86 (s, 3H), 3,95 (s, 2H), 5,4 (m, 1H), 6,9 (m, 1H), 7,0 (m, 2H), 7,3 (t, 1H).

### EXEMPLE 11 :

### Dichlorhydrate de l'acide 3-(2,3-dihydro-1,4-benzodioxin-6-yl)-3-{[2-({4-(4-pipéridinyl)-2-[2-(4-pipéridinyl)éthyl]butanoyl}amino)acétyl]amino} propanoïque (CRL42718)

Produit de départ : le composé 15
Rendement = 84%
MS (ES) : *m*/*z* 545 (M + H)⁺
¹H-RMN (400MHz, DMSO-d₆ + D₂O) : δ 1,1 (m, 4H), 1,25 (m, 6H), 1,4 (m, 4H), 1,7 (m, 4H), 2,15 (m, 1H), 2,6 (t, 2H), 2,75 (m, 4H), 3,15 (bd, 4H), 3,7 (s, 2H), 4,05 (s, 4H), 5,05 (m, 1H), 6,72 (s, 1H), 6,78 (d, 2H).

### EXEMPLE 12 :

### Trlchlorhydrate de l'acide 3-(3-pyridinyl)-3-{[2-({4-(4-pipéridinyl)-2-[2-(4-pipéridinyl)éthyl]butanoyl}amino)acétyl]amino}propanoïque (CRL42722)

Produit de départ : le composé 16
Rendement = 95%
MS (ES) : *m*/*z* 488 (M + H)⁺
¹H-RMN (400MHz, DMSO-d₆) : δ 1,1 (m, 4H), 1,25 (m, 6H), 1,4 (m, 4H), 1,65 (m, 4H), 2,15 (m, 1H), 2,85 (m, 4H), 2,9 (bd, 2H), 3,15 (m, 4H), 3,7 (m, 2H), 5,25 (m, 1H), 8,1 (bs, 1H), 8,25 (bs, 2H), 8,65 (d, 1H), 8,85 (bs, 1H), 8,95 (m, 4H), 9,2 (bs, 2H).

### EXEMPLE 13 :

### Dichlorhydrate de l'acide 3-{[2-({4-(4-pipéridinyl)-2-[2-(4-pipéridinyl)éthyl]butanoyl}amino)acétyl]amino}propanoïque (CRL43040)

Produit de départ : le composé 17
Rendement = 100%
MS (ES) : *m*/*z* 411 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,35 (t, 2H), 2,75 (bq, 4H), 3,15 (bd, 4H), 3,25 (q, 2H), 3,65 (d, 2H), 7,9 (t, 1H), 8,15 (t, 1H), 8,85 (bq, 2H), 9,15 (bd, 2H).

### EXEMPLE 14 :

### Dichlorhydrate de l'acide 3-{[2-({4-(4-pipéridinyl)-2-[2-(4-pipéridinyl)éthyl]butanoyl}amino)acétyl]amino}butanoïque (CRL43041)

Produit de départ : le composé 18
Rendement = 100%
MS (ES) : *m*/*z* 425 (M + H)⁺
¹H-RMN (400MHz, DMSO-d₆) : δ 1,1 (d, 3H), 1,15 (m, 4H), 1,3 (m, 6H), 1,4 (m, 4H), 1,7 (bd, 4H), 2,15 (m, 1H), 2,25 (q, 1H), 2,45 (q, 1H), 2,75 (bq, 4H), 3,15 (bd, 4H), 3,6 (m, 2H), 4,05 (m, 1H), 7,85 (d, 1H), 8,1 (t, 1H), 8,8 (bq, 2H), 9,1 (bd, 2H).

### EXEMPLE 15 :

### Dichlorhydrate de l'acide 5-phényl-3-{[2-({4-(4-pipéridinyl)-2-[2-(4-pipéridinyl)éthyl]butanoyl}amino)acétyl]amino}pentanoique (CRL43042)

Produit de départ : le composé 19
Rendement = 100%
MS (ES) : *m*/*z* 515 (M + H)⁺
¹H-RMN (400MHz, DMSO-d₆) : δ 1,15 (m, 4H), 1,25 (m, 6H), 1,45 (m, 4H), 1,75 (m, 6H), 2,2 (m, 1H), 2,4 (m, 2H), 2,5 (m, 1H), 2,65 (m, 1H), 2,75 (bq, 4H), 3,15 (bd, 4H), 3,7 (bq, 2H), 4,05 (m, 1H), 7,2 (m, 3H), 7,25 (t, 2H), 7,95 (d, 1H), 8,2 (t, 1H), 8,8 (bs, 2H), 9,15 (bd, 2H).

### EXEMPLE 16 :

### Dichlorhydrate de l'acide (3S)-4-(1-adamantylamino)-4-oxo-3-{[2-({4-(4-pipéridinyl)-2-[2-(4-pipéridinyl)éthyl]butanoyl}amino)acétyl]amino} butanoïque (CRL42592)

Produit de départ : le composé 20
Rendement = 94%
MS (ES) : *m*/*z* 588 (M + H)⁺
¹H-RMN (400MHz, CD₃OD) : δ 1,25 (m, 10H), 1,5 (m, 4H), 1,6 (s, 6H), 1,82 (bd, 4H), 1,9 (bs, 9H), 2,2 (m, 1H), 2,6 (m, 2H), 2,86 (bt, 4H), 3,25 (bd, 4H), 3,7 (d, 2H), 4,5 (t, 1H).

### EXEMPLE 17 :

### Dichlorhydrate de l'acide (3S)-4-{[2-(1H-indol-4-yl)éthyl]amino}-4-oxo-3-{[2-({4-(4-pipéridinyl)-2-[2-(4-pipéridinyl)éthyl]butanoyl}amino)acétyl]amino} butapoïque (CRL42678)

Produit de départ : le composé 21
Rendement = 100%
MS (ES) : *m*/*z* 597 (M + H)⁺
¹H-RMN (400MHz, DMSO-d₆) : δ 1,15 (m, 4H), 1,3 (m, 6H), 1,45 (m, 4H), 1,75 (m, 4H), 2,2 (m, 1H), 2,55 (m, 2H), 2,75 (m, 6H), 3,15 (m, 4H), 3,35 (m, 2H), 3,75 (bs, 2H), 4,6 (m, 1H), 6,98 (t, 1H), 7,08 (t, 1H), 7,16 (s, 1H), 7,35 (m, 1H), 7,55 (d, 1H), 8,1 (d, 1H), 8,2 (bs, 2H), 8,9 (bs, 2H), 9,2 (bs, 2H), 10,9 (bs, 1H).

### EXEMPLE 18 :

### Dichlorhydrate de l'acide (3S)-4-[(4-méthoxyphényl)éthylamino]-4-oxo-3-{[2-({4-(4-pipéridinyl)-2-[2-{4-pipéridinyl)éthyl]butanoyl}amino)acétyl]amino} butanoïque (CRL42694)

Produit de départ : le composé 22
Rendement = 97%
1H-RMN (400MHz, DMSO-d₆) : δ 1,15 (m, 4H), 1,25 (m, 6H), 1,45 (m, 4H), 1,8 (bd, 4H), 2,15 (m, 1H), 2,5 (m, 2H), 2,65 (t, 2H), 2,75 (bd, 4H), 3,15 (bd, 6H), 3,7 (s, 5H), 4,55 (m, 1H), 6,85 (d, 2H), 7,15 (d, 2H), 8,1 (d, 1H), 8,15 (m, 2H), 8,95 (bd, 2H), 9,2 (bd, 2H).

### EXEMPLE 19 :

### Dichlorhydrate de l'acide (3S)-4-(3-phénylpropylamino)-4-oxo-3-{[2-({4-(4-pipéridinyl)-2-[2-(4-pipéridinyl)éthyl]butanoyl}amino)acétyl]amino}butanoïque (CRL42719)

Produit de départ : le composé 23
Rendement = 100%
MS (ES) : *m*/*z* 572 (M + H)⁺
¹H-RMN (400MHz, DMSO-d₆) : δ 1,15 (m, 4H), 1,3 (m, 6H), 1,4 (m, 4H), 1,7 (m, 6H), 2,15 (m, 1H), 2,55 (m, 4H), 2,75 (bd, 4H), 3,05 (m, 2H), 3,2 (bd, 4H), 3,7 (d, 2H), 4,55 (m, 1H), 7,2 (m, 5H), 8,15 (m, 3H), 8,9 (bs, 2H), 9,15 (bs, 2H).

### EXEMPLE 20 :

### Dichlorhydrate de l'acide (3S)-4-[(1,3-benzodioxol-5-ylméthyl)amino]-4-oxo-3-{[2-({4-(4-pipéridinyl)-2-[2-(4-pipéridinyl)éthyl]butanoyl}amino)acétyl]amino}butanoïque (CRL42720)

Produit de départ : le composé 24
Rendement = 90%
MS (ES) : *m*/*z* 588 (M + H)⁺
¹H-RMN (400MHz, DMSO-d₆) : δ 1,15 (m, 4H), 1,3 (m, 6H), 1,4 (m, 4H), 1,7 (m, 4H), 2,15 (m, 1H), 2,55 (bs, 2H), 2,75 (bd, 4H), 3,15 (d, 4H), 3,7 (d, 2H), 4,15 (d, 2H), 4,6 (m, 1H), 5,95 (s, 2H), 6,7 (d, 1H), 6,8 (s, 1H), 6,81 (d, 1H), 8,2 (m, 2H), 8,55 (t, 1H), 8,9 (bs, 2H), 9,15 (bs, 2H).

### EXEMPLE 21 :

### Dichlorhydrate de l'acide (3S)-4-[(3-méthoxyphényl)amino]-4-oxo-3-{[2-({4-(4-pipéridinyl)-2-[2-(4-pipéridinyl)éthyl]butanoyl}amino)acétyl]amino}butanoïque (CRL42721)

Produit de départ : le composé 25
Rendement = 100%
¹H-RMN (400MHz, DMSO-d₆) : δ 1,15 (m, 4H), 1,3 (m, 6H), 1,4 (m, 4H), 1,7 (m, 4H), 2,15 (m, 1H), 2,5 (m, 4H), 2,7 (m, 6H), 3,15 (m, 6H), 3,7 (s, 5H), 4,55 (m, 1H), 6,75 (s, 3H), 7,15 (t, 1H), 8,1 (d, 1H), 8,2 (bs, 2H), 8,9 (bs, 2H), 9,15 (bs, 2H).

### EXEMPLE 22 :

### Dichlorhydrate de l'acide (3S)-4-[(2-hydroxy-1,1-diméthyléthyl)amino]-4-oxo-3-{[2-({4-(4-pipéridinyl)-2-[2-(4-pipéridinyl)éthyl]butanoyl}amino)acétyl]amino}butanoïque (CRL42726)

Produit de départ : le composé 26
Rendement = 100%
MS (ES) : *m*/*z* 526 (M + H)⁺
¹H-RMN (400MHz, DMSO-d₆) : δ 1,1 (m, 7H), 1,25 (m, 7H), 1,4 (m, 4H), 1,7 (m, 4H), 2,15 (m, 1H), 2,5 (m, 2H), 2,6 (m, 1H), 2,7 (bd, 4H), 3,1 (bd, 4H), 3,3 (q, 1H), 3,7 (bs, 2H), 4,05 (m, 1H), 4,45 (m, 1H), 4,65 (m, 1H), 8,05∼8,5 (m, 3H), 8,9 (bs, 2H), 9,15 (bs, 2H).

### EXEMPLE 23 :

### Dichlorhydrate de l'acide (3S)-4-[(1-isopropyl-2-méthylpropyl)amino]-4-oxo-3-{[2-({4-(4-pipérldinyl)-2-[2-(4-pipéridinyl)éthyl]butanoyl}amino)acétyl] amino}butanoïque (CRL42727)

Produit de départ : le composé 27
Rendement = 89%
MS (ES) : *m*/*z* 552 (M + H)⁺
¹H-RMN (400MHz, DMSO-d₆) : δ 0,8 (m, 9H), 1,15 (m, 11H), 1,25 (m, 4H), 1,85 (m, 6H), 2,15 (m, 1H), 2,6 (m, 1H), 2,75 (m, 4H), 3,15 (m, 4H), 3,4 (m, 4H), 3,7 (m, 2H), 4,55 (m, 1H), 7,3 (d, 1H), 8,3 (m, 2H), 8,95 (bs, 2H), 9,2 (bs, 2H).

### EXEMPLE 24 :

### Dichlorhydrate de l'acide (2S)-2-{[(benzyloxy)carboyl]amino}-3-{[2-({4-(4-pipéridinyl)-2-[2-(4-pipéridinyl)éthyl]butanoyl}amino)acétyl]amino} propanoïque (CRL42717)

Produit de départ : le composé 29
Rendement = 97%
[α]_{D} -8 (C = 2,2, H₂O)
MS (ES) : *m*/*z* 560 (M + H)⁺
¹H-RMN (400MHz, DMSO-d₆) : δ 1,15 (m, 4H), 1,3 (m, 6H), 1,45 (m, 4H), 1,75 (m, 4H), 2,15 (m, 1H), 2,8 (bd, 4H), 3,2 (,bd, 4H), 3,3 (m, 1H), 3,5 (m, 1H), 3,7 (m, 2H), 4,1 (q, 1H), 5,05 (s, 2H), 7,35 (m, 4H), 7,55 (d, 1H), 8,05 (s, 1H), 8,15 (s, 1H), 8,9 (bs, 2H), 9,2 (bs, 2H).

### EXEMPLE 25 :

### Dichlorhydrate de l'acide 2-[(1,3-benzodioxol-5-ylcarbonyl)amino]-3-{[2-({4-(4-pipéridinyl)-2-[2-(4-pipéridinyl)éthyl]butanoyl}amino)acétyl]amino}propanoïque (CRL42731)

Produit de départ : le composé 40
Rendement = 100%
MS (ES) : *m*/*z* 574 (M + H)⁺
¹H-RMN (400MHz, DMSO-d₆) : δ 1,15 (m, 4H), 1,25 (m, 6H), 1,45 (m, 4H), 1,75 (m, 4H), 2,2 (m, 1H), 2,75 (m, 4H), 3,2 (bd, 4H), 3,48 (m, 1H), 3,65 (m, 3H), 4,45 (m, 1H), 6,1 (s, 1H), 7,0 (d, 1H), 7,5 (s, 1H), 7,55 (d, 1H), 8,25 (bd, 2H), 8,65 (d, 1H), 8,95 (bs, 2H), 9,2 (bs, 2H).

### EXEMPLE 26 :

### Dichlorhydrate de l'acide 2-[(phénylsulfonyl)amino]-3-{[2-({4-(4-pipéridinyl)-2-[2-(4-pipéridinyl)éthyl]butanoyl}amino)acétyl]amino}propanoïque (CRL42724)

Produit de départ : le composé 39
Rendement = 95%
MS-CI : *m*/*z* 566 (M)⁺
¹H-RMN (400MHz, DMSO-d₆) : δ 1,15 (m, 4H), 1,25 (m, 6H), 1,45 (m, 4H), 1,75 (m, 4H), 2,2 (m, 1H), 2,75 (m, 4H), 3,15 (bd, 4H), 3,25 (m, 1H), 3,5 (m, 2H), 3,8 (m, 1H), 7,6 (m, 3H), 7,8 (m, 2H), 8,05 (bd, 2H), 8,25 (d, 1H), 9,0 (bd, 2H), 9,25 (bd, 2H).

### EXEMPLE 27 :

### Dichlorhydrate de l'acide (2S)-2-[(2-naphtylsulfonyl)amino]-3-{[2-({4-(4-pipéridinyl)-2-[2-(4-pipéridinyl)éthyl]butanoyl}amino)acétyl]amino} propanoïque (CRL42796)

Produit de départ : le composé 41
Rendement = 91%
[α]_{D} -10,1 (C = 0,86, H₂O)
MS (ES) : *m*/*z* 616 (M + H)⁺
¹H-RMN (400MHz, DMSO-d₆) : δ 1,1 (m, 4H), 1,25 (m, 6H), 1,45 (m, 4H), 1,7 (m, 4H), 2,15 (m, 1 H), 2,75 (bd, 4H), 3,2 (bd, 5H), 3,4 (m, 1H), 3,55 (dq, 2H), 3,95 (q, 1H), 7,7 (m, 2H), 7,8 (d, 1H), 8,0 (m, 3H), 8,15 (dd, 2H), 8,35 (d, 1H), 8,4 (s, 1H), 8,7 (bs, 2H), 8,95 (bd, 2H).

### EXEMPLE 28 :

### Dichlorhydrate de l'acide 2-{[(4-propylphényl)sulfonyl]amino}-3-{[2-({4-(4-pipéridinyl)-2-[2-(4-pipéridinyl)éthyl]butanoyl}amino)acétyl]amino} propanoïque (CRL42811)

Produit de départ : le composé 42
Rendement = 94%
MS (ES) : *m*/*z* 608 (M + H)⁺
¹H-RMN (400MHz, DMSO-d₆) : δ 0,85 (t, 3H), 1,15 (m, 4H), 1,25 (m, 6H), 1,35 (m, 4H), 1,6 (q, 2H), 1,7 (m, 4H), 2,1 (m, 1H), 2,65 (t, 2H), 2,75 (m, 4H), 3,15 (m, 4H), 3,25 (m,1H), 3,5 (m, 2H), 3,8 (m, 1H), 7,35 (d, 2H), 7,65 (d, 2H), 8,0 (t, 1H), 8,05 (m, 1H), 8,1 (d, 1H), 8,8 (bd, 2H), 9,05 (bd, 2H).

### EXEMPLE 29 :

### Dichlorhydrate de l'acide (3S)-4-oxo-4-(4-benzylpipéridino)-3-{[2-({4-(4-pipéridinyl)-2-[2-(4-pipéridinyl)éthyl]butanoyl}amino)acétyl]amino}-butanoïque (CRL42591)

Produit de départ: le composé 28
Rendement = 89%
MS (ES) : *m*/*z* 612 (M + H)⁺
1H-RMN( 400Mhz, CD₃OD) : δ 1,3-1,6 (m, 12H), 1,9 (m, 6H), 2,26 (m, 1H), 2,55 (m, 4H), 2,92 (m, 6H), 3,45 (m, 6H), 3,85 (bd, 2H), 4 (bt, 1H), 4,4 (bd, 1H), 5,2 (bs, 1H), 7,15 (m, 3H), 7,25 (t, 2H).

### EXEMPLE 30 :

### Dichlorhydrate de l'acide 2-[([1,1'biphényl]-4-ylsulfonyl)amino]-3-{[2-({4-(4-pipéridinyl)-2-[2-(4-pipéridinyl)éthyl]butanoyl}amino)acétyl]amino}propanoïque (CRL 42913)

Produit de départ : le composé 43
Rendement = 96%
MS (ES) : *m*/*z* 642 (M + H)⁺
¹H-RMN (400MHz, DMSO-d₆) : δ 1,15 (m, 4H), 1,30 (m, 6H), 1,45 (m, 4H), 1,75 (bd, 4H), 2,15 (m, 1H), 2,75 (bq, 4H), 3,20 (bd, 5H), 3,40 (m, 1H), 3,60 (dq, 2H), 3,9 (m, 1H), 7,45 (t, 1H), 7,5 (t, 2H), 7,75 (d, 2H), 7,85 (s, 4H), 8,05 (bs, 2H), 8,3 (d, 1H), 8,9 (bq, 2H), 9,15 (bd, 2H).

### EXEMPLE 31 :

### Dichlorhydrate de l'acide 2-[(1-naphtylsulfonyl)amino]-3-{[2-({4-(4-pipéridinyl)-2-[2-(4-pipéridinyl)éthyl]butanoyl}amino)acétyl]amino} propanoïque (CRL42914)

Produit de départ : le composé 44
Rendement = 96%
MS (ES) : *m*/*z* 616 (M + H)⁺
¹H-RMN (400MHz, DMSO-d₆) : δ 1,05 (m, 4H), 1,2 (m, 6H), 1,4 (m, 4H), 1,7 (bd, 4H), 2,1 (m, 1H), 2,75 (bq, 4H), 3,15 (bd, 5H), 3,3 (m, 1H), 3,45 (dq, 2H), 3,85 (q, 1H), 7,65 (m, 3H), 7,95 (m, 2H), 8,05 (d, 1H), 8,1 (d, 1H), 8,2 (d, 1H), 8,5 (d, 1H), 8,65 (d, 1H), 8,75 (bs, 2H), 9,0 (bd, 2H).

### EXEMPLE 32 :

### Dichlorhydrate de l'acide 2-({[4-(méthylsulfonyl)phényl]sulfonyl}amino)-3-{[2-({4-(4-pipéridinyl)-2-[2-(4-pipéridinyl)éthyl]butanoyl}amino)acétyl]amino} propanoïque (CRL42969)

Produit de départ : le composé 45
Rendement = 91 %
MS (ES) : *m*/*z* 644 (M + H)⁺
¹H-RMN (400MHz, DMSO-d₆) : δ 1,1 (m, 4H), 1,25 (m, 6H), 1,4 (m, 4H), 1,7 (bd, 4H), 2,1 (m, 1H), 2,7 (bq, 4H), 3,15 (bd, 5H), 3,25 (s, 3H), 3,35 (m, 1H), 3,55 (dq, 2H), 3,9 (m, 1H), 7,95∼8,1 (m, 6H), 8,55 (d, 1H), 8,8 (bq, 2H), 9,05 (bd, 2H).

### EXEMPLE 33 :

### Dichlorhydrate de l'acide 2-[(2-thiénylsulfonyl)amino]-3-{[2-({4-(4-pipéridinyl)-2-[2-(4-pipéridinyl)éthyl]butanoyl}amino)acétyl]amino} propanoïque (CRL 42985)

Produit de départ : le composé 46
Rendement = 97%
MS (ES) : *m*/*z* 572 (M + H)⁺
¹H-RMN (400MHz, DMSO-d₆) : δ 1,05 (m, 4H), 1,25 (m, 6H), 1,4 (m, 4H), 1,7 (bd, 4H), 2,15 (m, 1H), 2,7 (bq, 4H), 3,15 (bd, 5H), 3,35 (m, 1H), 3,55 (dq, 2H), 3,85 (q, 1H), 7,15 (dd, 1H), 7,5 (t, 1H), 7,9 (t, 1H), 8,0 (t, 1H), 8,05 (t, 1H), 8,4 (d, 1H), 8,8 (bq, 2H), 9,1 (bd, 2H).

### EXEMPLE 34 :

### Dichlorhydrate de l'acide 2-{[(4-chlorophényl)sulfonyl]amino}-3-{[2-({4-(4-pipéridinyl)-2-[2-(4-pipéridinyl)éthyl]butanoyl}amino)acétyl]amino} propanoïque (CRL42986)

Produit de départ : le composé 47
Rendement = 92%
MS (ES) : *m*/*z* 600 (M + H)⁺
¹H-RMN (400MHz, DMSO-d₆) δ 1,1 (m, 4H), 1,25 (m, 6H), 1,4 (m, 4H), 1,7 (bd, 4H), 2,1 (m, 1H), 2,7 (bq, 4H), 3,15 (bd, 5H), 3,3 (m, 1H), 3,55 (dq, 2H), 3,8 (q, 1H), 7,6 (d, 2H), 7,75 (d, 2H), 8,05 (m, 2H), 8,35 (d, 1H), 8,85 (bq, 2H), 9,1 (bd, 2H).

### EXEMPLE 35 :

### Dichlorhydrate de l'acide 2-{[(4-fluorophényl)sulfonyl]amino}-3-{[2-({4-(4-pipéridinyl)-2-[2-(4-pipéridinyl)éthyl]butanoyl}amino)acétyl]amino} propanoïque (CRL42999)

Produit de départ : le composé 48
Rendement = 97%
MS (ES) : *m*/*z* 584 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,75 (bq, 4H), 3,2 (bd, 5H), 3,35 (m, 1H), 3,6 (dq, 2H), 3,85 (m, 1H), 7,4 (t, 2H), 7,85 (dd, 2H), 8,05 (m, 2H), 8,30 (d, 1H), 8,8 (bq, 2H), 9,1 (bd, 2H).

### EXEMPLE 36 :

### Dichlorhydrate de l'acide (2S)-2-{[(6-méthoxy-2-naphtyl)sulfonyl]amino}-3-{[2-({4-(4-pipéridinyl)-2-[2-(4-pipéridinyl)éthyl]butanoyl}amino)acétyl]amino}propanoïque (CRL43000)

Produit de départ : le composé 49
Rendement = 90%
[α]_{D} -10 (C = 1, H₂O)
MS (ES) : *m*/*z* 646 (M + H)⁺
¹H-RMN (400MHz, DMSO-d₆) : δ 1,1 (m, 4H), 1,25 (m, 6H), 1,4 (m, 4H), 1,7 (bd, 4H), 2,15 (m, 1H), 2,75 (bq, 4H), 3,2 (bd, 5H), 3,35 (m, 1H), 3,6 (dq, 2H), 3,85 (m, 1H), 3,9 (s, 3H), 7,3 (d, 1H), 7,45 (s, 1H), 7,75 (d, 1H), 8,0 (d, 1H), 8,05 (m, 3H), 8,25 (d, 1H), 8,3 (s, 1H), 8,8 (bq, 2H), 9,1 (bd, 2H).

### EXEMPLE 37 :

### Dichlorhydrate de l'acide 2-[(mésitylsulfonyl)amino]-3-{[2-({4-(4-pipéridinyl)-2-[2-(4-pipéridinyl)éthyl]butanoyl}amino)acétyl]amino} propanoïque (CRL43031)

Produit de départ : le composé 50
Rendement = 98%
MS (ES) : *m*/*z* 608 (M + H)⁺
¹H-RMN (400MHz, DMSO-d₆) : δ 1,15 (m, 4H), 1,3 (m, 6H), 1,4 (m, 4H), 1,7 (bd, 4H), 2,15 (m, 1H), 2,25 (s, 3H), 2,55 (s, 6H), 2,75 (bq, 4H), 3,2 (bd, 5H), 3,35 (m, 1H), 3,55 (dq, 2H), 3,8 (q, 1H), 7,0 (s, 2H), 8,0 (m, 3H), 8,85 (bq, 2H), 9,15 (bd, 2H).

### EXEMPLE 38 :

### Dichlorhydrate de l'acide (2S)-2-[(butylsulfonyl)amino]-3-{[2-({4-(4-pipéridinyl)-2-[2-(4-pipéridinyl)éthyl]butanoyl}amino)acétyl]amino}propanoïque (CRL43032)

Produit de départ : le composé 51
Rendement = 90%
[α]_{D} -10,5 (C = 1, H₂O)
MS (ES) : *m*/*z* 546 (M + H)⁺
¹H-RMN (400MHz, DMSO-d₆) : δ 0,9 (t, 3H), 1,15 (m, 4H), 1,25∼1,5 (m, 12H), 1,6∼1,75 (m, 6H), 2,15 (m, 1H), 2,75 (bq, 4H), 3,0 (m, 2H), 3,15 (bd, 4H), 3,25 (m, 1H), 3,45 (m, 1H), 3,7 (dq, 2H), 3,95 (m, 1H), 7,6 (d, 1H), 8,05 (t, 1H), 8,15 (t, 1H), 8,85 (bq, 2H), 9,1 (bd, 2H).

### EXEMPLE 39 :

### Dichlorhydrate de l'acide 2-{[(4-méthylphényl)sulfonyl]amino}-3-{[2-({4-(4-pipéridinyl)-2-[2-(4-pipéridinyl)éthyl]butanoyl}amino)acétyl]amino} propanoïque (CRL43033)

Produit de départ : le composé 52
Rendement = 87%
MS (ES) : *m*/*z* 580 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,4 (s, 3H), 2,75 (bq, 4H), 3,15 (bd, 5H), 3,35 (m, 1H), 3,55 (dq, 2H), 3,8 (q, 1H), 7,35 (d, 2H), 7,7 (d, 2H), 8,0 (t, 1H), 8,05 (t, 1H), 8,1 (d, 1H), 8,8 (bq, 2H), 9,1 (bd, 2H).

### EXEMPLE 40 :

### Dichlorhydrate de l'acide 2-{[(3-méthylphényl)sulfonyl]amino}-3-{[2-({4-(4-pipéridinyl)-2-[2-(4-pipéridinyl)éthyl]butanoyl}amino)acétyl]amino} propanoïque (CRL43043)

Produit de départ : le composé 53
Rendement = 99%
MS (ES) : *m*/*z* 580 (M + H)⁺
¹H-RMN (400MHz, DMSO-d₆) : δ 1,1 (m, 4H), 1,25 (m, 6H), 1,4 (m, 4H), 1,7 (bd, 4H), 2,1 (m, 1H), 2,35 (s, 3H), 2,7 (bq, 4H), 3,15 (bd, 5H), 3,3 (m, 1H), 3,5 (dq, 2H), 3,8 (m, 1H), 7,4 (m, 2H), 7,55 (m, 2H), 8,0 (m, 2H), 8,15 (d, 1H), 8,8 (bq, 2H), 9,1 (bd, 2H).

### EXEMPLE 41 :

### Dichlorhydrate de l'acide 2-[(benzylsulfonyl)amino]-3-{[2-({4-(4-pipéridinyl)-2-[2-(4-pipéridinyl)éthyl]butanoyl}amino)acétyl]amino} propanoïque (CRL43055)

Produit de départ : le composé 54
Rendement = 99%
MS (ES) : m/z 580 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,75 (bq, 4H), 3,15 (bd, 4H), 3,35 (m, 1H), 3,45 (m, 1H), 3,7 (d, 2H), 3,95 (m, 1H), 4,35 (s, 2H), 7,35 (m, 5H), 7,6 (d, 1H), 8,1 (t, 1H), 8,15 (t, 1H), 8,85 (bq, 2H), 9,1 (bd, 2H).

### EXEMPLE 42 :

### Dichlorhydrate de l'acide 2-({[(E)-2-phényléthényl]sulfonyl}amino)-3-{[2-({4-(4-pipéridinyl)-2-[2-(4-pipéridinyl)éthyl]butanoyl}amino)acétyl]amino} propanoïque (CRL43057)

Produit de départ : le composé 55
Rendement = 100%
MS (ES) : m/z 592 (M + H)⁺
¹H-RMN (400MHz, DMSO-d₆) : δ 1,15 (m, 4H), 1,3 (m, 6H), 1,45 (m, 4H), 1,75
(bd, 4H), 2,15 (m, 1H), 2,75 (bq, 4H), 3,15 (bd, 4H), 3,3 (m, 1H), 3,45 (m, 1H), 3,65 (m, 2H), 3,9 (m, 1H), 7,15 (d, 1H), 7,35 (d, 1H), 7,45 (m, 3H), 7,7 (m, 2H), 7,9 (d, 1H), 8,1 (m, 2H), 8,9 (bq, 2H), 9,15 (bd, 2H).

### EXEMPLE 43 :

### Dichlorhydrate de l'acide 2-[(2-phénéthylsulfonyl)amino]-3-{[2-({4-(4-pipéridinyl)-2-[2-(4-pipéridinyl)éthyl]butanoyl}amino)acétyl]amino} propanoïque (CRL43056)

Produit de départ : le composé 56
Rendement = 96%
MS (ES) : *m*/*z* 594 (M + H)⁺
¹H-RMN (400MHz, DMSO-d₆) : δ 1,15 (m, 4H), 1,3 (m, 6H), 1,45 (m, 4H), 1,7 (bd, 4H), 2,15 (m, 1H), 2,75 (bq, 4H), 2,95 (m, 1H), 3,1 (m, 1H), 3,15 (bd, 5H), 3,3 (m, 2H), 3,5 (m, 1H), 3,7 (m, 2H), 4,05 (m, 1H), 7,3 (m, 5H), 7,8 (d, 1H), 8,1 (m, 2H), 8,9 (bq, 2H), 9,15 (bd, 2H).

### EXEMPLE 44 :

### Dichlorhydrate de l'acide 2-({[3-(trifluorométhyl)phényl]sulfonyl}amino)3-{[2-({4-(4-pipéridinyl)-2-[2-(4-pipéridinyl)éthyl)butanoyl}amino)acétyl]amino} propanoïque (CRL43058)

Produit de départ : le composé 57
Rendement = 93%
MS (ES) : *m*/*z* 634 (M + H)⁺
¹H-RMN (400MHz, DMSO) : δ 1,15 (m, 4H), 1,3 (m, 6H), 1,4 (m, 4H), 1,75 (bd, 4H), 2,15 (m, 1H), 2,75 (bq, 4H), 3,2 (bd, 5H), 3,4 (m, 1H), 3,6 (dq, 2H), 3,95 (m, 1H), 7,85 (t, 1H), 8,0 (d, 1H), 8,05 (m, 4H), 8,6 (t, 1H), 8,85 (bq, 2H), 9,1 (bd, 2H).

### EXEMPLE 45 :

### Dichlorhydrate de l'acide 2-{[(3-nitrophényl)sulfonyl]amino}-3-{[2-({4-(4-pipéridinyl)-2-[2-(4-pipéridinyl)éthyl]butanoyl}amino)acétyl]amino} propanoïque (CRL43059)

Produit de départ : le composé 58
Rendement = 90%
MS (ES) : *m*/*z* 611 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,75 (bq, 4H), 3,2 (bd, 5H), 3,35 (m, 1H), 3,6 (dq, 2H), 3,9 (m, 1H), 7,9 (t, 1H), 8,1 (m, 2H), 8,2 (d, 1H), 8,45 (dd, 1H), 8,55 (s, 1H), 8,75 (d, 1H), 8,85 (bq, 2H), 9,1 (bd, 2H).

### EXEMPLE 46 :

### Dichlorhydrate de l'acide 2-{[(4-méthoxyphényl)sulfonyl]amino}-3-{[2-({4-(4-pipéridinyl)-2-[2-(4-pipéridinyl)éthyl]butanoyl}amino)acétyl]amino} propanoïque (CRL43060)

Produit de départ : le composé 59
Rendement = 95%
MS (ES): *m*/*z* 596 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,75 (bq, 4H), 3,2 (bd, 5H), 3,35 (m, 1H), 3,6 (dq, 2H), 3,8 (m, 1H), 3,85 (s, 3H), 7,1 (d, 2H), 7,7 (d, 2H), 8,05 (m, 3H), 8,85 (bq, 2H), 9,1 (bd, 2H).

### EXEMPLE 47 :

### Trichlorhydrate de l'acide 2-[(8-quinolinylsulfonyl)amino] 3-{[2-({4-(4-pipéridinyl)-2-[2-(4-pipéridinyl)éthyl]butanoyl}amino)acétyl]amino} propanoïque (CRL43061)

Produit de départ : le composé 60
Rendement = 100%
MS (ES) : *m*/*z* 617 (M + H)⁺
¹H-RMN (400MHz, DMSO) : δ 1,15 (m, 4H), 1,25 (m, 6H), 1,45 (m, 4H), 1,75 (bd, 4H), 2,15 (m, 1H), 2,75 (bq, 4H), 3,2 (bd, 4H), 3,35 (m, 2H), 3,55 (dq, 2H), 4,25 (m, 1H), 7,75 (m, 3H), 8,0 (t, 1H), 8,1 (t, 1H), 8,3 (m, 2H), 8,6 (d, 1H), 8,9 (bd, 2H), 9,1 (m, 1H), 9, (bd, 2H).

### EXEMPLE 48 :

### Dichlorhydrate de l'acide 2-{[(3,5-diméthyl-4-isoxazolyl)sulfonyl]amino}-3-{[2-({4-(4-pipéridinyl)-2-[2-(4-pipéridinyl)éthyl]butanoyl}amino)acétyl]amino} propanoïque (CRL43068)

Produit de départ : le composé 61
Rendement = 85%
MS (ES) : *m*/*z* 585 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,35 (s, 3H), 2,6 (s, 3H), 2,75 (bq, 4H), 3,2 (bd, 5H), 3,4 (m, 1H), 3,6 (dq, 2H), 3,85 (m, 1H), 8,1 (t, 2H), 8,6 (d, 1H), 8,9 (bq, 2H), 9,15 (bd, 2H).

### EXEMPLE 49 :

### Trichlorhydrate de l'acide 2-({[5-(diméthylamino)-1-naphtyl)sulfonyl}amino)-3-{[2-({4-(4-pipéridinyl)-2-[2-(4-pipéridinyl)éthyl]butanoyl}amino)acétyl]amino} propanoïque (CRL43069)

Produit de départ : le composé 62
Rendement = 100%
MS (ES) : *m*/*z* 659 (M + H)⁺
¹H-RMN (400MHz, DMSO-d₆) : δ 1,1 (m, 4H), 1,3 (m, 6H), 1,45 (m, 4H), 1,7 (bd, 4H), 2,15 (m, 1H), 2,75 (bq, 4H), 3,2 (bs, 11H), 3,35 (m, 2H), 3,5 (q, 1H), 3,9 (q, 1H), 7,75 (q, 2H), 7,9 (bs, 1H), 7,95 (t, 1H), 8,05 (t, 1H), 8,7 (dd, 2H), 8,9 (bq, 2H), 9,15 (bd, 2H).

### EXEMPLE 50 :

### Dichlorhydrate de l'acide 2-({[2-(acétylamino)-4-méthyl-1,3-thiazol-5-yl]sulfonyl}amino)-3-{[2-({4-(4-pipéridinyl)-2-[2-(4-pipéridinyl)éthyl]butanoyl}amino)acétyl]amino} propanoïque (CRL43070)

Produit de départ : le composé 63
Rendement = 100%
MS (ES) : *m*/*z* 644 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,2 (s, 3H), 2,45 (s, 3H), 2,8 (bq, 4H), 3,2 (bd, 5H), 3,4 (m, 1H), 3,6 (dq, 2H), 3,9 (m, 1H), 8,1 (m, 2H), 8,45 (d, 1H), 8,85 (bq, 2H), 9,1 (bd, 2H).

### EXEMPLE 51 :

### Dichlorhydrate de l'acide 2-{[(3-chloropropyl)sulfonyl]amino}-3-{[2-({4-(4-pipéridinyl)-2-[2-(4-pipéridinyl)éthyl]butanoyl}amino)acétyl]amino} propanoïque (CRL43078)

Produit de départ : le composé 64
Rendement = 100%
MS (ES) : *m*/*z* 566 (M + H)⁺
¹H-RMN (400MHz, DMSO-d₆) : δ 1,15 (m, 4H), 1,25 (m, 6H), 1,45 (m, 4H), 1,75 (bd, 4H), 2,15 (m, 3H), 2,75 (bq, 4H), 3,15 (m, 7H), 3,45 (m, 1H), 3,65 (m, 2H), 3,75 (t, 2H), 3,95 (m, 1H), 7,75 (d, 1H), 8,1 (m, 2H), 8,8 (bq, 2H), 9,1 (bd, 2H).

### EXEMPLE 52 :

### Dichlorhydrate de l'acide 2-{[(4-méthoxy-1-naphtyl)sulfonyl]amino}-3-{[2-({4-(4-pipéridinyl)-2-[2-(4-pipéridinyl)éthyl]butanoyl}amino)acétyl]amino}propanoïque (CRL43079)

Produit de départ : le composé 65
Rendement = 95%
MS (ES) : *m*/*z* 646 (M + H)⁺
¹H-RMN (400MHz, DMSO-d₆) : δ 1,15 (m, 4H), 1,3 (m, 6H), 1,45 (m, 4H), 1,7 (bd, 4H), 2,1 (m, 1H), 2,75 (bq, 4H), 3,15 (bd, 5H), 3,3 (m, 1H), 3,45 (dq, 2H), 3,8 (m, 1H), 4,05 (s, 3H), 7,1 (d, 2H), 7,60( t, 1H), 7,7 (t, 1H), 7,9 (t, 1H), 8,0 (t, 1H), 8,1 (d, 2H), 8,3 (d, 1H), 8,35 (d, 1H), 8,6 (d, 1H), 8,85 (bs, 2H), 9,1 (bs, 2H).

### EXEMPLE 53 :

### Dichlorhydrate de l'acide 2-{[(6,7-diméthoxy-2-naphtyl)sulfonyl]amino}-3-{[2-({4-(4-pipéridinyl)-2-[2-(4-pipéridinyl)éthyl]butanoyl}amino)acétyl]amino}propanoïque (CRL43080)

Produit de départ : le composé 66
Rendement = 86%
MS (ES) : *m*/*z* 676 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,75 (bq, 4H), 3,15 (bd, 5H), 3,35 (m, 1H), 3,55 (dq, 2H), 3,85 (m, 1H), 3,9 (s, 3H), 3,92 (s, 3H), 7,45 (s, 1H), 7,55 (s, 1H), 7,65 (d, 1H), 7,9 (d, 1H), 8,05 (bs, 2H), 8,20 (d, 1H), 8,25 (s, 1H), 8,85 (bq, 2H), 9,1 (bd, 2H).

### EXEMPLE 54 :

### Dichlorhydrate de l'acide 2-{[(2,4-diméthyl-1,3-thiazol-5-yl)sulfonyl]amino}-3-{[2-({4-(4-pipéridinyl)-2-[2-(4-pipéridinyl)éthyl]butanoyl}amino)acétyl]amino} propanoïque (CRL43120)

Produit de départ : le composé 67
Rendement = 94%
MS (ES) : *m*/*z* 601 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,5 (s, 3H), 2,65 (s, 3H), 2,75 (bq, 4H), 3,2 (bd, 5H), 3,4 (m, 1H), 3,6 (dq, 2H), 3,9 (q, 1H), 8,1 (m, 2H), 8,7 (d, 1H), 8,9 (bq, 2H), 9,15 (bd, 2H).

### EXEMPLE 55 :

### Dichlorhydrate de l'acide 2-{[(3,5-diméthyl-1H-pyrazol-4-yl)sulfonyl]amino}-3-{[2-({4-(4-pipéridinyl)-2-[2-(4-pipéridinyl)éthyl]butanoyl}amino)acétyl]amino} propanoïque (CRL43121)

Produit de départ : le composé 68
Rendement = 98%
MS (ES) : *m*/*z* 584 (M + H)⁺
¹H-RMN (400MHz, DMSO-d₆) : δ 1,15 (m, 4H), 1,25 (m, 6H), 1,4 (m, 4H), 1,7 (bd, 4H), 2,15 (m, 1H), 2,3 (s, 6H), 2,75 (bq, 4H), 3,15 (bd, 5H), 3,3 (m, 1H), 3,6 (dq, 2H), 3,75 (m, 1H), 7,8 (d, 1H), 8,0 (bt, 1H), 8,1 (bt, 1H), 8,8 (bq, 2H), 9,15 (bd, 2H).

### EXEMPLE 56 :

### Trichlorhydrate de l'acide 2-[(3-pyridinylsulfonyl)amino] 3-{[2-({4-(4-pipéridinyl)-2-[2-(4-pipéridinyl)éthyl]butanoyl}amino)acétyl]amino} propanoïque (CRL43122)

Produit de départ : le composé 69
Rendement = 69%
MS (ES) : *m*/*z* 567 (M + H)⁺
1H-RMN (400MHz, DMSO) : δ 1,1 (m, 4H), 1,3 (m, 6H), 1,45 (m, 4H), 1,7 (bd, 4H), 2,15 (m, 1H), 2,75 (bd, 4H), 3,15 (bd, 5H), 3,45 (m, 1H), 3,55 (dq, 2H), 3,95 (q, 1H), 7,75 (t, 1H), 8,15 (bd, 2H), 8,35 (d, 1H), 8,75 (d, 1H), 8,9 (bd, 1H), 8,95 (bd, 2H), 9,0 (s, 1H), 9,2 (bd, 2H).

### EXEMPLE 57 :

### Dichlorhydrate de l'acide 2-[(1,3-benzodioxol-5-ylsulfonyl)amino]-3-{[2-({4-(4-pipéridinyl)-2-[2-(4-pipéridinyl)éthyl]butanoyl}amino)acétyl]amino} propanoïque (CRL43123)

Produit de départ : le composé 70
Rendement = 91 %
MS (ES) : *m*/*z* 610 (M + H)⁺
¹H-RMN (400MHz, DMSO-d₆) : δ 1,1 (m, 4H), 1,25 (m, 6H), 1,4 (m, 4H), 1,7 (bd, 4H), 2,15 (m, 1H), 2,75 (bq, 4H), 3,15 (bd, 5H), 3,3 (m, 1H), 3,6 (dq, 2H), 3,75 (m, 1H), 6,1 (d, 2H), 7,0 (d, 1H), 7,2 (s, 1H), 7,25 (d, 1H), 8,05 (m, 3H), 8,75 (bd, 2H), 9,05 (bd,2H).

### EXEMPLE 58 :

### Dichlorhydrate de l'acide 2-[(2,3-dihydro-1,4-benzodioxin-6-ylsulfonyl)amino]-3-{[2-({4-(4-pipéridinyl)-2-[2-(4-pipéridinyl)éthyl]butanoyl}amino)acétyl]amino} propanoïque (CRL43124)

Produit de départ : le composé 71
Rendement = 91 %
MS (ES) : *m*/*z* 624 (M + H)⁺
¹H-RMN (400MHz, DMSO-d₆) : δ 1,1 (m, 4H), 1,3 (m, 6H), 1,4 (m, 4H), 1,7 (bd, 4H), 2,15 (m, 1H), 2,75 (bq, 4H), 3,15 (bd, 5H), 3,3 (m, 1H), 3,6 (dq, 2H), 3,75 (q, 1H), 4,3 (dd, 2H), 6,95 (d, 1H), 7,2 (d, 1H), 7,22 (s, 1H), 8,05 (m, 3H), 8,85 (bq, 2H), 9,1 (bd, 2H).

### EXEMPLE 59 :

### Dichlorhydrate de l'acide 2-[(1-benzothiophèn-2-ylsulfonyl)amino]-3-{[2-({4-(4-pipéridinyl)-2-[2-(4-pipéridinyl)éthyl]butanoyl}amino)acétyl]amino}propanoïque (CRL 43125)

Produit de départ : le composé 72
Rendement = 99%
MS (ES) : *m*/*z* 622 (M + H)⁺
¹H-RMN (400MHz, DMSO-d₆) : δ 1,1 (m, 4H), 1,25 (m, 6H), 1,4 (m, 4H), 1,7 (bd, 4H), 2,15 (m, 1H), 2,75 (bq, 4H), 3,15 (bd, 5H), 3,3 (m, 1H) , 3,45 (dq, 2H), 3,9 (q, 1H), 7,5 (m, 2H), 8,0 (m, 2H), 8,1 (d, 1H), 8,2 (d, 1H), 8,45 (s, 1H), 8,55 (d, 1H), 8,9 (bq, 2H), 9,15 (bd, 2H).

### EXEMPLE 60 :

### Dichlorhydrate de l'acide 2-{[(2,5-diméthyl-3-furyl)sulfonyl]amino}-3-{[2-({4-(4-pipéridinyl)-2-[2-(4-pipéridinyl)éthyl]butanoyl}amino)acétyl]amino}propanoïque (CRL 43131)

Produit de départ : le composé 73
Rendement = 99%
MS (ES) : *m*/*z* 584 (M + H)⁺
¹H-RMN (400MHz, DMSO-d₆) : δ 1,15 (m, 4H), 1,3 (m, 6H), 1,45 (m, 4H), 1,75 (bd, 4H), 2,15 (m, 1H), 2,25 (s, 3H), 2,4 (s, 3H), 2,75 (bq, 4H), 3,2 (bd, 5H), 3,35 (m, 1H), 3,6 (dq, 2H), 3,8 (m, 1H), 6,2 (s, 1H), 8,05 (m, 3H), 8,8 (bq, 2H), 9,05 (bd, 2H).

### EXEMPLE 61 :

### Dichlorhydrate de l'acide 2-{[(4-cyclohexylphényl)sulfonyl]amino}-3-{[2-({4-(4-pipéridinyl)-2-[2-(4-pipéridinyl)éthyl]butanoyl}amino)acétyl]amino}propanoïque (CRL 43132)

Produit de départ : le composé 74
Rendement = 94%
MS (ES) : *m*/*z* 648 (M + H)⁺
¹H-RMN (400MHz, DMSO-d₆) : δ 1,0-1,5 (m, 20H), 1,75 (m, 8H), 2,15 (m, 1H), 2,6 (bt, 1H), 2,75 (bq, 4H), 3,2 (bd, 5H), 3,35 (m, 1H), 3,5 (dq, 2H), 3,85 (q, 1H), 7,15 (d, 2H), 7,7 (d, 2H), 7,98 (t, 1H), 8,04 (t, 1H), 8,1 (d, 1H), 8,8 (bq, 2H), 9,05 (bd, 2H).

### EXEMPLE 62 :

### Dichlorhydrate de l'acide 2-{[(4-fluoro-1-naphtyl)sulfonyl]amino}-3-{[2-({4-(4-pipéridinyl)-2-[2-(4-pipéridinyl)éthyl]butanoyl}amino)acétyl]amino} propanoïque (CRL 43133)

Produit de départ : le composé 75
Rendement = 90%
MS (ES) : m/z 634 (M + H)⁺
¹H-RMN (400MHz, DMSO-d₆) : δ 1,1 (m, 4H), 1,25 (m, 6H), 1,4 (m, 4H), 1,7 (bd, 4H), 2,1 (m, 1H), 2,75 (bq, 4H), 3,15 (bd, 5H), 3,3 (m, 1H), 3,5 (dq, 2H), 3,85 (q, 1H), 7,5 (t, 1H), 7,8 (m, 2H), 8,0 (m, 2H), 8,15 (m, 2H), 8,65 (d, 1H), 8,7 (d, 1H), 8,8 (bq, 2H), 9,05 (bd, 2H).

### EXEMPLE 63 :

### Dichlorhydrate de l'acide 2-{[(4-chloro-1-naphtyl)sulfonyl]amino}-3-{[2-({4-(4-pipéridinyl)-2-[2-(4-pipéridinyl)éthyl]butanoyl}amino)acétyl]amino} propanoïque (CRL 43134)

Produit de départ : le composé 76
Rendement = 91 %
MS (ES) : *m*/*z* 650 (M + H)⁺
¹H-RMN (400MHz, DMSO-d₆) : δ 1,1 (m, 4H), 1,25 (m, 6H), 1,4 (m, 4H), 1,7 (bd, 4H), 2,15 (m, 1H), 2,75 (bq, 4H), 3,15 (bd, 5H), 3,35 (m, 1H) , 3,5 (dq, 2H), 3,85 (q, 1H), 7,85 (m, 3H), 8,0 (m, 2H), 8,1 (t, 1H), 8,75 (m, 4H), 9,0 (bd, 2H).

### EXEMPLE 64 :

### Dichlorhydrate de l'acide 2-[(2,3-dihydro-1-benzofuran-5-ylsulfonyl)amino]-3-{[2-({4-(4-pipéridinyl)-2-[2-(4-pipéridinyl)éthyl]butanoyl}amino)acétyl]amino}propanoïque (CRL 43142)

Produit de départ : le composé 77
Rendement = 94%
MS (ES) : *m*/*z* 608 (M + H)⁺
¹H-RMN (400MHz, DMSO-d₆) : δ 1,1 (m, 4H), 1,25 (m, 6H), 1,4 (m, 4H), 1,7 (bd, 4H), 2,15 (m, 1H), 2,75 (bq, 4H), 3,2 (m, 7H), 3,3 (m, 1H), 3,6 (dq, 2H), 3,75 (q, 1H), 4,65 (t, 2H), 6,9 (d, 1H), 7,52 (d, 1H), 7,6 (s, 1H), 8,0 (d, 1H), 8,05 (t, 1H), 8,10 (t, 1H), 8,85 (bq, 2H), 9,1 (bd, 2H).

### EXEMPLE 65 :

### Dichlorhydrate de l'acide 2-({[4-(2-thiényl)phényl]sulfonyl}amino) 3-{[2-({4-(4-pipéridinyl)-2-[2-(4-pipéridinyl)éthyl]butanoyl}amino)acétyl]amino} propanoïque (CRL 43143)

Produit de départ : le composé 78
Rendement = 66%
MS (ES) : *m*/*z* 648 (M + H)⁺
¹H-RMN (400MHz, DMSO-d₆) : δ 1,1 (m, 4H), 1,25 (m, 6H), 1,35 (m, 4H), 1,7 (bd, 4H), 2,15 (m, 1H), 2,75 (bq, 4H), 3,1 (bd, 5H), 3,3 (m, 1H) , 3,55 (dq, 2H), 3,85 (m, 1H), 7,2 (bs, 1H), 7,65 (bs, 2H), 7,75 (dd, 4H), 8,05 (m, 2H), 8,30 (d, 1H), 8,85 (bq, 2H), 9,1 (bd, 2H).

### EXEMPLE 66 :

### Dichlorhydrate de l'acide 2-({[2-(2-thiényl)phényl]sulfonyl}amino) 3-{[2-({4-(4-pipéridinyl)-2-[2-(4-pipéridinyl)éthyl]butanoyl}amino)acétyl]amino} propanoïque (CRL 43144)

Produit de départ : le composé 79
Rendement = 97%
MS (ES) : *m*/*z* 648 (M + H)⁺
¹H-RMN (400MHz, DMSO-d₆) : δ 1,1 (m, 4H), 1,25 (m, 6H), 1,4 (m, 4H), 1,7 (bd, 4H), 2,1 (m, 1H), 2,75 (bq, 4H), 3,15 (bd, 4H), 3,3 (m, 1H), 3,35 (m, 1H), 3,65 (dq, 2H), 3,8 (q, 1H), 7,1 (t, 1H), 7,35 (d, 1H), 7,5 (d, 2H), 7,6 (m, 3H), 7,85 (d, 1H), 8,0 (d, 1H), 8,1 (m, 2H), 8,8 (bq, 2H), 9,1 (bd, 2H).

### EXEMPLE 67 :

### Dichlorhydrate de l'acide 2-({[4-(2-furyl)phényl]sulfonyl}amino)-3-{[2-({4-(4-pipéridinyl)-2-[2-(4-pipéridinyl)éthyl]butanoyl}amino)acétyl]amino}propanoïque (CRL 43146)

Produit de départ : le composé 80
Rendement = 84%
MS (ES) : *m*/*z* 632 (M + H)⁺
¹H-RMN (400MHz, DMSO-d₆) : δ 1,1 (m, 4H), 1,25 (m, 6H), 1,45 (m, 4H), 1,75 (bd, 4H), 2,15 (m, 1H), 2,75 (bq, 4H), 3,2 (bd, 5H), 3,35 (m, 1H), 3,6 (dq, 2H), 3,85 (q, 1H), 6,7 (d, 1H), 7,2 (d, 1H), 7,8 (d, 2H), 7,85 (m, 3H), 8,1 (m, 2H), 8,25 (d, 1H), 8,85 (bq, 2H), 9,1 (bd, 2H).

### EXEMPLE 68 :

### Dichlorhydrate de l'acide 2-[(1-benzofuran-Z-ylsulfonyl)amino]-3-{[2-({4-(4-pipéridinyl)-2-[2-(4-pipéridinyl)éthyl]butanoyl}amino)acétyl]amino}propanoïque (CRL 43147)

Produit de départ : le composé 81
Rendement = 94%
MS (ES): *m*/*z* 606 (M + H)⁺
¹H-RMN (400MHz, DMSO-d₆) : δ 1,1 (m, 4H), 1,2 (m, 6H), 1,4 (m, 4H), 1,75 (bd, 4H), 2,15 (m, 1H), 2,75 (bq, 4H), 3,15 (bd, 5H), 3,5 (m, 1H), 3,55 (dq, 2H), 4,05 (m, 1H), 7,4 (t, 1H), 7,5 (m, 2H), 7,7 (d, 1H), 7,75 (d, 1H), 8,05 (bs, 2H), 8,8 (bd, 3H), 9,1 (bd, 2H).

### EXEMPLE 69 :

### Dichlorhydrate de l'acide 2-{[(2-naphtylméthyl)sulfonyl]amino}-3-{[2-({4-(4-pipéridinyl)-2-[2-(4-pipéridinyl)éthyl]butanoyl}amino)acétyl]amino}-propanoïque (CRL 43158)

Produit de départ : le composé 82
Rendement = 88%
MS (ES) : *m*/*z* 630 (M + H)⁺
¹H-RMN (400MHz, DMSO-d₆) : δ 1,2 (m, 7H), 1,45 (m, 4H), 1,7 (m, 4H), 2,15 (m, 1H), 2,75 (bq, 4H), 3,1 (bd, 4H), 3,4 (m, 2H), 3,7 (d, 2H), 4,05 (q, 1H), 4,5 (s, 2H), 7,55 (d, 2H), 7,65 (d, 1H), 7,95 (m, 4H), 8,05 (t, 1H), 8,1 (t, 1H), 8,6 (bq, 2H), 8,85 (bd, 2H).

### EXEMPLE 70 :

### Dichlorhydrate de l'acide 2-[(2,3-dihydro-1H-indèn-5-ylsulfonyl)amino]-3-{[2-({4-(4-pipéridinyl)-2-[2-(4-pipéridinyl)éthyl]butanoyl}amino)acétyl]amino}propanoïque (CRL 43159)

Produit de départ : le composé 83
Rendement = 94%
MS (ES) : *m*/*z* 606 (M + H)⁺
¹H-RMN (400MHz, DMSO-d₆) : δ 1,15 (m, 4H), 1,25 (m, 6H), 1,4 (m, 4H), 1,7 (bd, 4H), 2,05 (m, 2H), 2,15 (m, 1H), 2,75 (bq, 4H), 2,9 (t, 4H), 3,15 (bd, 5H), 3,35 (m, 1H), 3,5 (dq, 2H), 3,8 (q, 1H), 7,4 (d, 1H), 7,55 (d, 1H), 7,6 (s, 1H), 8,0 (m, 4H), 8,75 (bq, 2H), 9,0 (bd, 2H).

### EXEMPLE 71 :

### Dichlorhydrate de l'acide 2-{[(5-phényl-2-thiényl))sulfonyl]amino}-3-{[2-({4-(4-pipéridinyl)-2-[2-(4-pipéridinyl)éthyl]butanoyl}amino)acétyl]amino}propanoïque (CRL 43160)

Produit de départ : le composé 84
endement = 97%
MS (ES) : *m*/*z* 648 (M + H)⁺
¹H-RMN (400MHz, DMSO-d₆) : δ 1,1 (m, 4H), 1,25 (m, 6H), 1,4 (m, 4H), 1,7 (bd, 4H), 2,15 (m, 1H), 2,75 (bq, 4H), 3,15 (bd, 5H), 3,4 (m, 1H), 3,6 (dq, 2H), 3,95 (q, 1 H), 7,4 (m, 3H), 7,55 (s, 2H), 7,75 (d, 2H), 8,05 (bs, 2H), 8,55 (d, 1 H), 8,85 (bq, 2H), 9,1 (bd, 2H).

### EXEMPLE 72 :

### Dichlorhydrate de l'acide 2-[(5,6,7,8-tétrahydro-2-naphthalenylsulfonyl)amino]3-{[2-({4-(4-pipéridinyl)-2-[2-(4-pipéridinyl)éthyl]butanoyl}amino)acétyl]amino} propanoïque (CRL 43161)

Produit de départ : le composé 85
Rendement = 97%
MS (ES) : *m*/*z* 620 (M + H)⁺
¹H-RMN (400MHz, DMSO-d₆) : δ 1,1 (m, 4H), 1,25 (m, 6H), 1,45 (m, 4H), 1,75 (bs, 8H), 2,15 (m, 1H), 2,75 (bs, 8H), 3,2 (bd, 5H), 3,35 (m, 1H), 3,55 (dq, 2H), 3,8 (q, 1H), 7,2 (d, 1H), 7,45 (d, 2H), 8,0 (m, 3H), 8,75 (bq, 2H), 9,0 (bd, 2H).

### EXEMPLE 73 :

### Dichlorhydrate de l'acide 2-{[(E)-1-pentènylsulfonyl]amino}-3-([2-({4-(4-pipéridinyl)-2-[2-(4-pipéridinyl)éthyl]butanoyl}amino)acétyl]amino} propanoïque (CRL43176)

Produit de départ : le composé 86
Rendement = 93%
MS (ES) : *m*/*z* 558 (M + H)⁺
¹H-RMN (400MHz, DMSO-d₆) : δ 0,9 (t, 3H), 1,15 (m, 4H), 1,25 (m, 6H), 1,45 (m, 6H), 1,75 (bd, 4H), 2,15 (q, 3H), 2,75 (bq, 4H), 3,2 (bd, 5H), 3,4-3,75 (m, 4H), 6,3 (d, 1H), 6,5 (dt, 1H), 7,65 (d, 1H), 8,0 (t, 1H), 8,1 (t, 1H), 8,75 (bq, 2H), 9,0 (bd, 2H).

### EXEMPLE 74 :

### Dichlorhydrate de l'acide 2-[(cyclohexylsulfonyl)amino]-3-{[2-({4-(4-pipéridinyl)-2-[2-(4-pipéridinyl)éthyl]butanoyl}amino)acétyl]amino}propanoïque (CRL43190)

Produit de depart : le compose 30
Rendement = 93%
MS (ES): *m*/*z* 572 (M + H)⁺
¹H-RMN (400MHz, DMSO-d₆) : δ 1,05∼1,40 (m, 15H), 1,45 (m, 4H), 1,60 (bd, 1H), 1,75 (m, 6H), 2,0∼2,25 (m, 3H), 2,75 (bq, 4H), 2,85 (m, 1H), 3,15 (bd, 4H), 3,25 (m, 1H), 3,45 (m, 1H), 3,70 (m, 2H), 3,95 (m, 1H), 7,5 (d, 1H), 8,10 (m, 2H), 8,85 (bd, 2H), 9,1 (bd, 2H).

### EXEMPLE 75 :

### Dichlorhydrate de l'acide 2-[(isopropylsulfonyl)amino]-3-[[2-({4-(4-pipéridinyl)-2-[2-(4-pipéridinyl)éthyl]butanoyl}amino)acétyl]amino}propanoïque (CRL43191)

Produit de départ : le composé 31
Rendement = 97%
MS (ES) : *m*/*z* 532 (M + H)⁺
¹H-RMN (400MHz, DMSO-d₆) : δ 1,1∼1,35 (m, 16H), 1,45 (m, 4H), 1,75 (bd, 4H), 2,15 (m, 1H), 2,75 (bq, 4H), 3,1 (m, 1H), 3,2 (bd, 4H), 3,3 (m, 1H), 3,45 (m, 1H), 3,70 (m, 2H), 3,95 (m, 1H), 7,5 (d, 1H), 8,05 (t, 1H), 8,10 (t, 1H), 8,8 (bq, 2H), 9,05 (bd, 2H).

### EXEMPLE 76 :

### Dichlorhydrate de l'acide 2-[(1,3-benzothiazol-2-ylsulfonyl)amino]-3-{[2-({4-(4-pipéridinyl)-2-[2-(4-pipéridinyl)éthyl]butanoyl}amino)acétyl]amino}propanoïque (CRL43194)

Produit de départ : le composé 32
Rendement = 98%
MS (ES) : *m*/*z* 623 (M + H)⁺
¹H-RMN (400MHz, DMSO-d₆) : δ 1,15 (m, 4H), 1,30 (m, 6H), 1,45 (m, 4H), 1,70 (m, 4H), 2,15 (m, 1H), 2,75 (bd, 4H), 3,15 (bd, 4H), 3,25 (m, 1H), 3,60 (m, 3H), 4,20 (q, 1H), 7,70 (m, 2H), 8,05 (bd, 2H), 8,20 (d, 1H), 8,30 (d, 1H), 8,90 (bd, 2H), 9,20 (bd, 2H), 9,25 (d, 1H).

### EXEMPLE 77 :

### Dichlorhydrate de l'acide (2S)-2-{[(benzyloxy)carboyl]amino}-3-{[3-({4-(4-pipéridinyl)-2-[2-(4-pipéridinyl)éthyl]butanoyl}amino)propanoyl]amino}propanoïque (CRL43022)

Produit de départ : le composé 33
Rendement = 88%
MS (ES) : *m*/*z* 574 (M + H)⁺
¹H-RMN (400MHz, DMSO-d₆) : δ 1,10 (m, 4H), 1,25 (m, 6H), 1,45 (m, 4H), 1,75 (bd, 4H), 2,05 (m, 1H), 2,25 (t, 2H), 2,75 (bq, 4H), 3,15 (bd, 6H), 3,3 (m, 1H), 3,45 (m, 1H), 4,1 (m, 1H), 5,05 (s, 2H), 7,35 (s, 5H), 7,55 (d, 1H), 7,95 (t, 1H), 8,15 (t, 1H), 8,95 (bq, 2H), 9,15 (bs, 2H).

### EXEMPLE 78 :

### Dichlorhydrate de l'acide 2-[(2-naphtylsulfonyl)amino]-3-{[3-({4-(4-pipéridinyl)-2-[2-(4-pipéridinyl)éthyl]butanoyl}amino)propanoyl]amino}propanoïque (CRL43021)

Produit de départ : le composé 87
Rendement = 84%
MS (ES) : *m*/*z* 630 (M + H)⁺
¹H-RMN (400MHz, DMSO-d₆) : δ 1,05 (m, 4H), 1,25 (m, 6H), 1,40 (m, 4H), 1,75 (bd, 4H), 2,05 (m, 3H), 2,75 (bq, 4H), 3,15 (m, 7H), 3,3 (m, 1H), 3,95 (q, 1H), 7,7 (m, 2H), 7,8 (d, 1H), 7,9 (t, 1H), 8,05 (d, 1H), 8,1 (m, 3H), 8,3 (d, 1H), 8,4 (s, 1H), 9,0 (bd, 2H), 9,25 (bd, 2H).

### EXEMPLE 79 :

### Dichlorhydrate de l'acide 2-[(phénylsulfonyl)amino]-3-{[3-({4-(4-pipéridinyl)-2-[2-(4-pipéridinyl)éthyl]butanoyl}amino)propanoyl]amino}propanoïque (CRL43145)

Produit de départ : le composé 88
Rendement = 86%
MS (ES) : *m*/*z* 580 (M + H)⁺
¹H-RMN (400MHz, DMSO-d₆) : δ 1,05 (m, 4H), 1,25 (m, 6H), 1,40 (m, 4H), 1,70 (bd, 4H), 2,05 (m, 1H), 2,1 (q, 2H), 2,75 (bq, 4H), 3,15 (m, 1H), 3,30 (m, 1H), 3,85 (q, 1H), 7,55 (m, 3H), 7,8 (d, 1H), 7,9 (t, 1H), 8,1 (t, 1H), 8,25 (d, 1H), 8,9 (bd, 2H), 9,10 (bd, 2H).

### EXEMPLE 80 :

### Dichlorhydrate de l'acide 2-[(2-naphtylsulfonyl)amino]-3-{[3-({4-(4-pipéridinyl)-2-[2-(4-pipéridinyl)éthyl]butanoyl}amino)butanoyl]amino} propanoïque (CRL43195)

Produit de départ : le composé 34
Rendement = 100%
MS (ES) : *m*/*z* 644 (M + H)⁺
¹H-RMN (400MHz, DMSO-d₆) : δ 0,9 (d, 3H), 1,05 (m, 4H), 1,25 (m, 6H), 1,40 (m, 4H), 1,70 (m, 4H), 1,95 (m, 1H), 2,00 (m, 1H), 2,15 (dd, 1H), 2,75 (bq, 4H), 3,05 (m, 1H), 3,20 (bd, 4H), 3,35 (m, 1H), 3,95 (m, 2H), 7,70 (m, 3H), 7,8 (d, 1H), 8,05 (m, 2H), 8,10 (dd, 2H), 8,30 (d, 1H), 8,35 (s, 1H), 8,75 (bt, 2H), 9,0 (bd, 2H).

### EXEMPLE 81:

### Dichlorhydrate de l'acide 2-[(2-naphtylsulfonyl)amino]-3-{[3-phényl-3-({4-(4-pipéridinyl)-2-[2-(4-pipéridinyl)éthyl]butanoyl}amino)butanoyl]amino} propanoïque (CRL43196)

Produit de départ : le composé 35
Rendement = 87%
MS (ES) : *m*/*z* 706 (M + H)⁺
¹H-RMN (400MHz, DMSO-d₆) δ 1,00 (m, 4H), 1,25 (m, 6H), 1,40 (m, 4H), 1,65 (dd, 4H), 2,10 (m, 1 H), 2,40 (m, 1H), 2,75 (bd, 4H), 3,00 (m, 1H), 3,10 (bs, 4H), 3,25 (m, 1H), 3,75 (m, 1H), 3,98 (q, 1H), 7,25 (m, 5H), 7,65 (m, 2H), 7,80 (m, 1H), 8,00 (t, 1H), 8,10 (m, 3H), 8,30 (t, 1H), 8,45 (d, 1H), 8,48 (m, 1H), 8,75 (bt, 2H), 9,05 (bt, 2H).

### EXEMPLE 82 :

### Dichlorhydrate de l'acide 2-[(2-naphtylsulfonyl)amino]-3-{[((3R)-1-{4-(4-pipéridinyl)-2-[2-(4-pipéridinyl)éthyl]butanoyl}hexahydro-3-pyridinyl)carbonyl]amino} propanoïque (CRL43197)

Produit de départ : le composé 36
Rendement = 93%
MS (ES): *m*/*z* 670 (M + H)⁺
¹H-RMN (400MHz, DMSO-d₆) δ 1,05 (m, 4H), 1,25∼1,60 (m, 14H), 1,73 (bt, 4H), 2,00 (m, 1H), 2,45 (m, 1H), 2,75 (m, 6H), 3,10 (m, 5H), 3,35 (m, 1H), 3,90 (m, 2H), 4,30 (dd, 1H), 7,70 (m, 2H), 7,80 (m, 1H), 8,00 (m, 1H), 8,10 (m, 3H), 8,40 (m, 2H), 8,85∼9,25 (m, 4H).

### EXEMPLE 83 :

### Dichlorhydrate de l'acide 2-[(2-naphtylsulfonyl)amino]-3-{[2-phényl-2-({4-(4-pipéridinyl)-2-[2-(4-pipéridinyl)éthyl]butanoyl}amino)éthanoyl]amino} propanoïque (CRL43210)

Produit de départ : le composé 91
Rendement = 94%
¹H-RMN (400MHz, DMSO-d₆) δ 0,95 (m, 1 H), 1,0∼1,5 (m, 13H), 1,60 (bd, 2H), 1,70 (bd, 2H), 2,35 (m, 1H), 2,70 (m, 4H), 3,15 (m, 5H), 3,25 (m, 1H), 3,95 (m, 1H), 5,50 (t, 1H), 7,25 (m, 5H), 7,65 (m, 2H), 7,80 (d, 1H), 8,01 (d, 1H), 8,10 (m, 1H), 8,15 (d, 1H), 8,30 (q, 1H), 8,40 (s, 1H), 8,50 (t, 2H), 8,75 (bs, 2H), 9,00 (bs, 2H).

### EXEMPLE 84 :

### Dichlorhydrate de l'acide 2-[(2-naphtylsulfonyl)amino]-3-{[2-({4-(4-pipéridinyl)-2-[2-(4-pipéridinyl)éthyl]butanoyl}amino)propanoyl]amino} propanoïque (CRL43214)

Produit de départ : le composé 92
Rendement = 89%
¹H-RMN (400MHz, DMSO-d₆) δ 1,00 (d, 3H), 1,08 (m, 4H), 1,27 (m, 6H), 1,42 (m, 4H), 1,70 (bd, 4H), 2,12 (m, 1H), 2,73 (bd, 4H), 3,00 (m, 1H), 3,20 (bs, 4H), 3,48 (m, 1H), 3,89 (q, 1H), 4,18 (t, 1H), 7,65 (m, 2H), 7,82 (d, 1H), 7,95 (d, 1H), 8,06 (d, 2H), 8,15 (q, 2H), 8,35 (d, 1H), 8,40 (s, 1H), 8,85 (m, 2H), 9,10 (bs, 2H).

### EXEMPLE 85 :

### Dichlorhydrate de l'acide 2-({[(7,7-diméthyl-2-oxobicyclo[2.2.1]hept-1-yl)méthyl]sulfonyl}amino)-3-{[2-({4-(4-pipéridinyl)-2-[2-(4-pipéridinyl)éthyl]butanoyl}amino)acétyl]amino}propanoïque CRL43215)

Produit de départ : le composé 89
Rendement = 90%
¹H-RMN (400MHz, DMSO-d₆) δ 0,80 (s, 3H), 1,05 (s, 3H), 1,15 (m, 4H), 1,30 (m, 6H), 1,45 (m, 6H), 1,75 (bd, 4H), 1,90 (m, 2H), 2,05 (t, 1H), 2,15 (m, 1H), 2,35 (m, 2H), 2,75 (bq, 4H), 3,05 (d, 1H), 3,20 (bd, 4H), 3,30 (m, 1H), 3,35 (d, 1H), 3,50 (m, 1H), 3,70 (dq, 2H), 4,00 (m, 1H), 7,65 (d, 1H), 8,10 (m, 2H), 8,85 (bs, 2H), 9,10 (bs, 2H).

### EXEMPLE 86 :

### Dichlorhydrate de l'acide (2R)-2-[(2-naphtylsulfonyl)amino]-3-{[2-({4-(4-pipéridinyl)-2-[2-(4-pipéridinyl)éthyl]butanoyl}amino)acétyl]amino} propanoïque (CRL42956)

L'acide (2R)-3-({2-[(4-[1-(*tert*-butoxycarbonyl)-4-pipéridinyl]-2-{2-[1-(tert-butoxycarbonyl)-4-pipéridinyl]éthyl}butanoyl)amino]acétyl}amino)-2-[(2-naphtylsulfonyl)amino]propanoïque (composé 90) (8 g, 9,8 mmol) est solubilisé dans 30 ml d'acétate d'éthyle ; puis 150 ml d'acétate d'éthyle-acide chlorhydrique 3 N sont ajoutés à température ambiante. L'agitation est maintenue pendant 40 minutes puis de l'acétate d'éthyle est ajouté. Une poudre blanche est obtenue par sèchage sous vide. Après addition de 500 ml d'eau, on obtient 5,92 g d'un solide blanc par lyophilisation.
Rendement = 98 %
[α]_{D} + 9,5 (C = 0,15, H₂O)
MS (ES) : *m*/*z* 616 (M + H)⁺
¹H-RMN (400MHz, DMSO-d₆) : δ 1,1 (m, 4H), 1,25 (m, 6H), 1,45 (m, 4H), 1,7 (m, 4H), 2,15 (m, 1H), 2,75 (bd, 4H), 3,2 (bd, 5H), 3,4 (m, 1H), 3,55 (dq, 2H), 3,95 (q, 1H), 7,7 (m, 2H), 7,8 (d, 1H), 8,0 (m, 3H), 8,15 (dd, 2H), 8,35 (d, 1H), 8,4 (s, 1H), 8,7 (bs, 2H), 8,95 (bd, 2H).

L'étude de l'activité inhibitrice de l'agrégation plaquettaire des composés de formule I a été réalisée *in vitro*, c'est-à-dire par contact direct de solution de concentrations variables des composés avec des plaquettes fraîchement séparées d'un échantillon de sang total, prélevé dans des conditions standardisées, chez des animaux de laboratoire (cobaye) et chez des sujets humains en bonne santé n'ayant pas reçu de substances ou de médicaments pouvant interférer avec la coagulation du sang. L'activité anti-agrégante plaquettaire a été également étudiée *ex vivo*/*vitro*, c'est-à-dire après administration des substances revendiquées au cobaye pour mesurer l'intensité et la durée de l'action anti-agrégante induite par la fraction absorbée et circulant dans le sang du produit étudié.

### 1. Etudes pharmacologiques in vitro

### 1.1. Etudes sur plaquettes de cobaye

Du sang est prélevé par ponction intracardiaque sur des cobayes Dunkin-Hartley de sexe mâle (poids 330 g environ), à raison de 4,5 ml pour 0,5 ml de citrate trisodique (concentration de la solution aqueuse : 1,55 %) afin de prévenir toute trace de coagulation. Le plasma riche en plaquettes (PRP) est obtenu par centrifugation des tubes de sang total 15 minutes à 150 g.

Les PRP sont rassemblés en "pools". Une numération des plaquettes contenues dans ces pools est pratiquée à l'aide d'un automate d'hématologie de type Coulter ZM : si nécessaire, une dilution est réalisée afin que la concentration du plasma en plaquettes soit comprise entre 200 000 et 400 000 plaquettes/mm³. Simultanément, d'autres échantillons de ces pools servent à préparer du plasma pauvre en plaquettes (PPP) par centrifugation à 1 500 g pendant 15 minutes.

L'étude cinétique de l'agrégation plaquettaire est réalisée par addition d'une solution de collagène ( 1 µg/ml) à un volume de PRP, à l'aide d'un agrégomètre de type Chrono-log Corporation (490- D₁ ou 560 VS) qui utilise une détection optique de l'apparition du thrombus.

La détermination de la concentration inhibitrice 50 % (CI₅₀) est réalisée en additionnant à des échantillons des pools de PRP, un volume déterminé de solvant (référence contrôle) et des concentrations croissantes des composés : 1,5 x10⁻⁸ M, 7 x10⁻⁸ M, 1,5 x10⁻⁷ M, 3 x10⁻⁷ M, 7 x 10⁻⁷ M, 1,5 x 10⁻⁶ M et 7 x 10⁻⁵ M. Les mesures de l'inhibition de l'agrégation sont faites après 3 minutes de contact à 37°C sous agitation.

### 1.2 Etude sur plaquettes humaines

Chez un groupe d'une dizaine de sujets humains, d'âge homogène et en bonne santé, du sang veineux est prélevé par ponction dans une veine du pli du coude pour être recueilli dans un tube de verre contenant une solution aqueuse de citrate de sodium 0,129 M (1 volume de solution citratée pour 9 volumes de sang). Chaque tube est ensuite centrifugé une première fois à 20°C et 100 g pendant 15 minutes pour obtenir le plasma riche en plaquettes (PRP) ; après prélèvement de ce PRP, le tube est à nouveau centrifugé à 2 000 g pendant 15 minutes pour prélever cette fois le plasma pauvre en plaquettes (PPP).

Pour chaque échantillon identifié de PRP, une numération plaquettaire est réalisée à l'aide du compteur Coulter ZM. Chaque échantillon est alors utilisé pour étudier la variation de l'inhibition de l'agrégation plaquettaire déclenchée par l'addition d'une solution glucose de collagène Chromo-par-Reagent de Coultronics (utilisé à la concentration de 5 µg/ml) en fonction de l'addition de concentrations croissantes de chaque composé dans une gamme couvrant l'intervalle 10⁻⁸ M→10⁻⁵ M (exemple de concentrations : 10⁻⁸ M, 5 x 10⁻⁷ M, 3 x10⁻⁷ M, 10⁻⁷M, 8 x10⁻⁶ M, 6 x10⁻⁶M, 4 x 10⁻⁶ M, 2 x10⁻⁶M, 10⁻⁶M, 5 x 10⁻⁵M, 10⁻⁵M). Au préalable, pour chaque composé, une solution aqueuse à 10⁻³M est préparée. Dans chaque série de mesure, est introduit un essai contrôle destiné à vérifier l'effet éventuel des solvants (valeur référence) sur l'agrégation plaquettaire qui est mesurée après 3 minutes de contact à 37°C sous agitation.

A partir des pourcentages d'inhibition de l'agrégation plaquettaire mesurés pour chaque concentration de chaque composé, est calculée la concentration inhibitrice 50 % (CI₅₀).

### 2. Etude pharmacologique ex vivo/vitro chez le cobaye

L'évaluation de l'activité anti-agrégante plaquettaire des composés est réalisée chez les mêmes cobayes que ceux évoqués ci-dessus (souche Dunkin-Hartley). L'administration de chaque produit dans une gamme de doses qui va de 150 à 10 mg/kg et de chaque véhicule (5 ml/kg) est faite par voie gastrique (v.g.) 1h ou 2h ou 4h ou 6h ou 8h ou 12h avant le prélèvement de sang chez les cobayes mis à jeun la veille. L'allocation des traitements aux animaux est aléatoire.

Le sang est prélevé puis traité dans les mêmes conditions que celles décrites ci-dessus pour les études *in vitro.*

Les résultats de l'inhibition de l'agrégation plaquettaire obtenus pour chaque concentration essayée, permettent de calculer la concentration CI₅₀ de chaque produit essayé et la cinétique de l'effet inhibiteur, leur durée d'action.

Les résultats sont rassemblés dans le tableau suivant :

| Exemples | Composé CRL | CI₅₀ (M) *in vitro* | | % d'inhibition v.g. cobaye *ex vivo* | | | |
|---|---|---|---|---|---|---|---|
| | | Cobaye | Homme | d = 150 mg/kg | | d = 10 mg/kg | |
| | | | | 1h | 2h | 1h | 2h |
| 6 | 42548 | 1,4 x 10⁻⁵ | 2,7 x 10⁻⁶ | -9 | -4 | - | - |
| 7 | 42549 | 10⁻⁵ | | -39 | 0 | - | - |
| 8 | 42590 | 3,5 x 10⁻⁵ | 1,6 x 10⁻⁶ | -10 | -31 | - | - |
| 29 | 42591 | 1,3 x 10⁻⁵ | 10⁻⁵ | -35 | -23 | | |
| 16 | 42592 | 3,5 x 10⁻⁶ | 8,5 x 10⁻⁷ | -54 | -47 | -5 | - |
| 5 | 42630 | 2,0 x 10⁻⁶ | 2,8 x 10⁻⁷ | -63 | -66 | -15 | - |
| 9 | 42639 | 5,0 x 10⁻⁶ | - | -53 | -5 | - | - |
| 10 | 42660 | 1,3 x 10⁻⁵ | 9,2 x 10⁻⁷ | -14 | -18 | - | - |
| 17 | 42678 | 7,4 x 10⁻⁶ | 6,0 x 10⁻⁶ | -19 | - | - | - |
| 12 | 42722 | 9,8 x 10⁻⁷ | 1,5 x 10⁻⁶ | -74 | -68 | - | - |
| 26 | 42724 | 4,4 x 10⁻⁷ | 5,3 x 10⁻⁷ | -77 | -68 | -41 | -20 |
| 25 | 42731 | 2,9 x 10⁻⁵ | - | -33 | -48 | - | - |
| 27 | 42796 | 1,9 x 10⁻⁸ | 4.4 x 10⁻⁸ | - | - | - | - |
| 28 | 42811 | 1,5 x 10⁻⁶ | 1,1 x 10⁻⁶ | -73 | -75 | -18 | - |
| 30 | 42913 | 1,2 x 10⁻⁷ | 1,4 x 10⁻⁷ | - | -72 | -67- | - |
| 31 | 42914 | 4,4 x 10⁻⁸ | 5,8 x 10⁻⁷ | - | - | -81 | -81 |
| 32 | 42969 | 1,2 x 10⁻⁵ | - | - | - | -8 | -8 |
| 33 | 42985 | 7,1 x 10⁻⁷ | 4,9 x 10⁻⁷ | - | - | -14 | -5 |
| 34 | 42986 | 5,0 x 10⁻⁷ | 8,9 x 10⁻⁷ | - | - | -19 | -8 |
| 35 | 42999 | 1,8 x 10⁻⁶ | 1,3 x 10⁻⁶ | - | - | -5 | 0 |
| 36 | 43000 | 4,1 x 10⁻⁸ | 7,2 x 10⁻⁸ | - | - | -63 | -69 |
| 78 | 43021 | 4,6 x 10⁻⁸ | 10⁻⁷ | - | - | -65 | -73 |
| 37 | 43031 | 1,4 x 10⁻⁷ | 1,3 x 10⁻⁷ | - | - | -9 | -16 |
| 39 | 43033 | 2,5 x 10⁻⁷ | - | - | - | -32 | -10 |
| 15 | 43042 | 4,3 x 10⁻⁶ | 1,5 x 10⁻⁷ | -84 | -26 | - | - |
| 40 | 43043 | 4,0 x 10⁻⁷ | - | - | - | -32 | -10 |
| 41 | 43055 | 8.6 x 10⁻⁶ | - | - | - | 13 | 1 |
| 46 | 43056 | 1,2 x 10⁻⁶ | - | - | - | -15 | -4 |
| 42 | 43057 | 4,2 x 10⁻⁷ | | - | - | -14 | -26 |
| 44 | 43058 | 5,1 x 10⁻⁷ | | - | - | -22 | -35 |
| 45 | 43059 | 5,0 x 10⁻⁷ | - | - | - | -36 | -51 |
| 46 | 43060 | 3,8 x 10⁻⁷ | - | - | - | -18 | -31 |
| 47 | 43061 | 1,9 x 10⁻⁶ | - | - | - | -4 | -17 |
| 48 | 43068 | 4,2 x 10⁻⁷ | - | - | - | -61 | -15 |
| 49 | 43069 | 4,9 x 10⁻⁸ | 5,6 x 10⁻⁸ | - | - | -69 | -68 |
| 50 | 43070 | 3,3 x 10⁻⁶ | - | - | - | -22 | -39 |
| 51 | 43078 | 1,1 x 10⁻⁵ | - | - | - | 4 | -21 |
| 52 | 43079 | 3,5 x 10⁻⁸ | 9,3 x 10⁻⁸ | | | -58 | -30 |
| 53 | 43080 | 3,7 x 10⁻⁸ | 7,3 x 10⁻⁸ | - | - | -73 | -9 |
| 54 | 43120 | 1,3 x 10⁻⁶ | - | - | - | -40 | -15 |
| 55 | 43121 | 3,5 x 10⁻⁶ | - | - | - | -21 | -17 |
| 56 | 43122 | 2,3 x 10⁻⁷ | - | - | - | -61 | -50 |
| 57 | 43123 | 8,3 x 10⁻⁷ | - | - | - | -38 | -24 |
| 58 | 43124 | 5,9 x 10⁻⁷ | - | - | - | -36 | -21 |
| 59 | 43125 | 6,6 x 10⁻⁸ | 1,5 x 10⁻⁷ | - | - | -66 | -34 |
| 60 | 43131 | 6,6 x 10⁻⁷ | - | - | - | -53 | -32 |
| 61 | 43132 | 1,4 x 10⁻⁶ | - | - | - | -21 | -9 |
| 62 | 43133 | 9,5 x 10⁻⁸ | - | - | - | -69 | -33 |
| 63 | 43134 | 5,3 x 10⁻⁸ | 10⁻⁶ | - | - | -69 | -50 |
| 64 | 43142 | 3,7 x 10⁻⁷ | - | - | - | -65 | -5 |
| 65 | 43143 | 2,0 x 10⁻⁷ | - | - | - | -65- | -12 |
| 66 | 43144 | 3,5 x 10⁻⁷ | - | - | - | -18 | 0 |
| 68 | 43147 | 1,8 x 10⁻⁷ | - | - | - | -72 | -71 |
| 69 | 43158 | 1,9 x 10⁻⁸ | - | - | - | -69 | -3 |
| 70 | 43159 | 4,0 x 10⁻⁷ | - | - | - | -32 | -34 |
| 71 | 43160 | 6,3 x 10⁻⁸ | 1,9 x 10⁻⁷ | - | - | -72 | -70 |
| 72 | 43161 | 3,4 x 10⁻⁷ | - | - | - | -60 | -67 |
| - : donnée non disponible. | | | | | | | |

La présente invention a donc également pour objet des compositions pharmaceutiques comprenant une quantité efficace d'un composé de formule (1) ou d'un de ses sels avec des acides pharmaceutiquement acceptables.

Elle a plus particulièrement pour objet des compositions pour inhiber l'agrégation des plaquettes sanguines comprenant une quantité efficace d'un de ces composés.

Elle a également pour objet
- l'utilisation de ces composés pour la fabrication d'un médicament antithrombotique, notamment pour traiter une thrombose survenue chez un patient ou prévenir un risque de thrombose, ainsi que pour la fabrication de médicaments pour inhiber la fixation du fibrinogène sur les plaquettes sanguines, et pour inhiber l'agrégation des plaquettes sanguines chez un patient.

Les composés de formule (I) peuvent être utilisés, notamment dans les domaines suivants :
i) Prévention aiguë du risque thrombotique artériel au cours de la chirurgie cardiaque (pontage coronarien) ou de la cardiologie interventionnelle (angioplastie percutanée transluminale, endartectomie, pose de "stent") :
   - dans ces situations, les composés sont ajoutés au traitement préventif reconnu du risque thrombotique artériel ; administration orale d'acide acétylsalicylique démarrant avant l'intervention (150 à 500 mg/j per os) puis se poursuivant ensuite ; perfusion intraveineuse d'héparine non fractionnée démarrée pendant l'intervention puis se poursuivant pendant 48 à 96 heures. L'administration du composé de formule I peut alors se faire soit par voie orale (0,5 à 1,5 mg/kg) en même temps que l'administration d'aspirine, soit par perfusion intraveineuse (0,25 à 1 mg/kg/24 h) associée ou non à un bolus. Après la 48^{ème} heure, si le traitement a été administré par voie intraveineuse, il sera relayé par l'administration orale (0,25 à 10 mg/kg en deux prises espacées de 12 heures) afin de faciliter les soins en hospitalisation puis en traitement ambulatoire.
ii) Prophylaxie secondaire du risque thrombotique artériel chez les patients pouvant présenter des épisodes d'angor instable ou un infarctus du myocarde : dans ces situations, la biodisponibilité importante des composés revendiqués, c'est-à-dire la possibilité d'obtenir rapidement des concentrations circulantes efficaces car capables d'inhiber la fixation du fibrinogène sur les plaquettes permet d'utiliser les médicaments revendiqués par la voie orale pendant la période où les patients présentent ce risque de thrombose artérielle. Dans ces situations, ces composés pourront être administrés avantageusement à raison de 1 à 3 prises orales par jour, grâce à leur biodisponibilité élevée et leur longue durée d'action, la dose étant choisie dans la gamme 0,5-10 mg/kg. Les compositions pharmaceutiques qui comprennent l'un des principes actifs décrits dans la présente demande incorporent la substance active soit sous forme de base, soit sous forme de sel pharmaceutiquement acceptable, soit encore sous la forme d'une pro-drogue comprenant une fonction ester, fonction qui est ensuite libérée *in vivo* après administration orale. Ces compositions pharmaceutiques incorporent les adjuvants de fabrication, les véhicules qui sont connus de l'homme de l'art. Ceux-ci sont choisis parmi la panoplie des outils galéniques reconnus par les Pharmacopées. Comme exemples, peuvent être mentionnés pour la préparation des formes galéniques destinées à la voie orale : amidon, stéarate de magnésium, talc, gélatine, agar, pectine, lactose, polyéthylène-glycols, etc ... Les formes galéniques utilisables seront choisies parmi les propositions suivantes : comprimés sécables ou non, gélules, lyocs, granulés, poudres. Selon les caractéristiques de la pathologie à traiter et la morphologie de chaque patient, la posologie orale quotidienne sera comprise entre 0,02 et 50 mg/kg/jour en 1 à 3 prises régulièrement espacées afin de maintenir un taux d'occupation efficace des récepteurs GpIIb/IIIa plaquettaires. Pour la voie intraveineuse, les formes pharmaceutiques destinées à la phase aiguë du traitement sont conçues de manière à permettre une adaptation posologique individuelle sur la base de l'inhibition de l'agrégation plaquettaire la plus efficace en fonction de l'évolution immédiate des suites opératoires. Dans ce cadre, le lyophilisat, le soluté pour perfusion prêt à l'emploi, permettent de moduler individuellement la posologie dans l'intervalle de dose 0,01 mg/kg/jour- 20 mg/kg/jour.

## Revendications

1. Composés de formule : 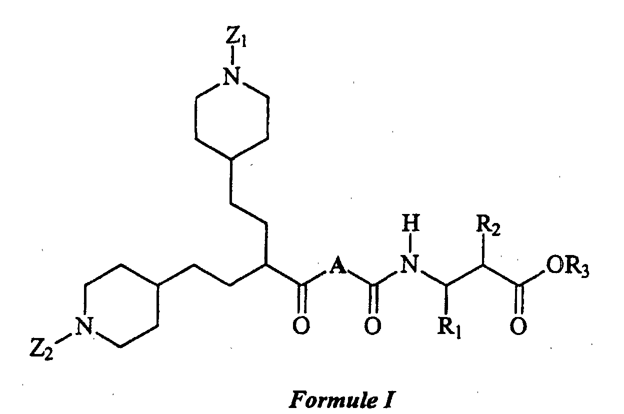 dans laquelle :
i) ou bien R₁ est choisi parmi :
- les groupes alkyle en C₁-C₄, cycloalkyle mono ou bicyclique en C₃-C₁₂, alkényle en C₂-C₄ ou alkynyle en C₂-C₄, ces groupes étant éventuellement substitués par des groupes choisis parmi les halogènes et le groupe hydroxy ;
- les groupes aryle mono, bi ou tricycliques en C₆-C₁₄,
- les groupes hétéroaryle choisis parmi les groupes pyridyle, thiényle, furannyle, quinolyle, benzodioxannyle, benzodioxolyle, benzothiényle, benzofurannyle et pyrazinyle ;
- les groupes phénylalkyle(C₁-C₄) et naphtylalkyle(C₁-C₄) éventuellement substitués sur le noyau aryle.
les groupes aryle et hétéroaryle pouvant être substitués par un ou plusieurs groupes choisis indépendamment parmi les halogènes, les groupes alkyle en C₁-C₄, trifluorométhyle, alkyl(C₁-C₄)thio, alkyl(C₁-C₄)sulfonyle, alkyl(C₁-C₄)oxy, nitro, les groupes -COOR, -CH₂COOR ou -O-CH₂-COOR, R étant un groupe alkyle en C₁-C₄,
- les groupes de formule : 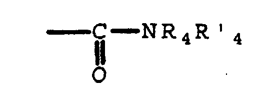
où R₄ et R'₄ sont choisis parmi les groupes alkyle en C₁-C₈, cycloalkyle mono ou polycyclique en C₃-C₁₂, ces groupes étant éventuellement substitués par des groupes choisis parmi les halogènes et la groupe hydroxy, R'₄ pouvant en outre être l'hydrogène ou bien R₄ et R'₄ forment ensemble un groupe tétraméthylène ou pentaméthylène, ces deux derniers groupes pouvant être eux-mêmes substitués par un reste aryle en C₆-C₁₄ ou aryle (C₆-C₁₄)alkyle(C₁-C₄);
- les groupes de formule : 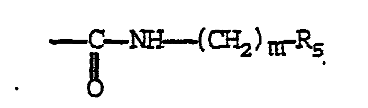
où m = 1 à 4 et R₅ est choisi parmi les groupes phényle, méthoxyphényle, indolyle, benzodioxolyle, benzodioxannyle, benzothiényle et benzofurannyle,
et R₂ est l'hydrogène,
ii) ou bien R₁ est l'hydrogène et R₂ est choisi parmi les groupes de formule :
-NH-CO-R₆,
R₆ étant choisi parmi les groupes alkoxy en C₁-C₄, cycloalkoxy en C₃-C₇, benzyloxy, méthoxyphényle, diméthoxyphényle, benzodioxolyle et benzodioxannyle,
et les groupes de formule :
-NH-SO₂-R₇,
R₇ étant choisi parmi :
- les groupes alkyle en C₁-C₅ éventuellement substitués par un ou plusieurs groupes choisis parmi les halogènes, les groupes hydroxy et le groupe trifluorométhyle ;
- les groupes alkényle en C₂-C₅;
- les groupes cycloalkyle mono ou bicycliques en C₃-C₁₂;
- les groupes aryle mono, bi ou tricycliques en C₆-C₁₄ ;
- les groupes hétéroaryle choisis parmi les groupes pyridyie, furyle, thiényle, quinolyle, benzodioxannyle, benzodioxolyle, isoxazolyle, benzodioxinnyle, benzothiényle, thiazolyle, pyrazolyle, benzofuryle et benzothiazolyle ;
- les groupes phénylalkyle (C₁-C₄) et naphtylalkyle (C₁-C₄) ;
- et les groupes de formule : 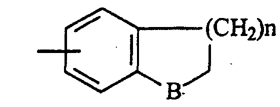
avec n = 1, 2 ou 3 et B étant choisi parmi -CH₂-, O ou S et -NH- les groupes aryle ou hétéroaryle étant éventuellement substitués par un ou plusieurs groupes choisis indépendamment parmi les halogènes, les groupes alkyle en C₁-C₄, cycloalkyle en C₃-C₇, trifluorométhyle, alkyl (C₁-C₄)thio, alkyl(C₁-C₄)oxy, alkyl(C₁-C₄)sulfonyle, nitro, di(alkylC₁-C₄)amino, les groupes -COOR, - CH₂-COOR, ou -O-CH₂COOR, R étant un groupe alkyle en C₁-C₄, les groupes phényle, naphtyle et les groupes hétéroaryle choisis parmi les groupes thiènyle, furyle et pyridyle,
iii) R₃ est choisi parmi un atome d'hydrogène, un groupe alkyle en C₁-C₄ et un groupe phényl(alkyle en C₁-C₄);
iv) A est choisi parmi les groupes -NH-CHR₁₀-, -NH-CHR₁₀-CH₂- et 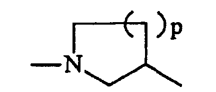 avec p = 1 ou 2,
- R₁₀ étant choisi parmi l'hydrogène, un groupe alkyle en C₁-C₄ et un groupe aryle en C₆-C₁₄,
v) et Z₁ et Z₂ représentent l'hydrogène ou un groupe protecteur d'amine, et leurs sels d'addition avec des acides pharmaceutiquement acceptables.

2. Composés selon la revendication 1 de formule 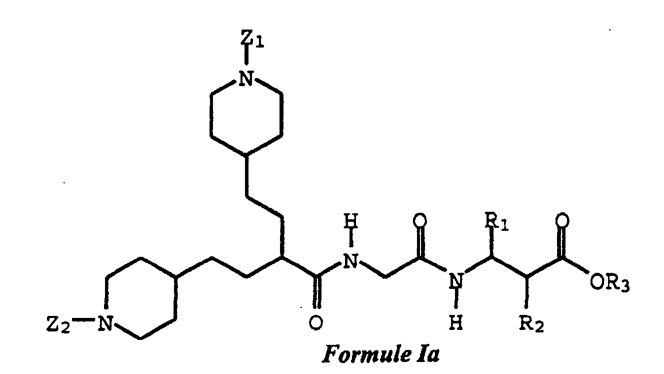 dans laquelle:
i) ou bien R₁ est choisi parmi :
- les groupes alkyle en C₁-C₄, cycloalkyle mono ou bicyclique en C₃-C₁₂, alkényle en C₂-C₄ ou alkynyle en C₂-C₄, ces groupes étant éventuellement substitués par des groupes choisis parmi les halogènes et le groupe hydroxy ;
- les groupes aryle mono, bi ou tricycliques en C₆-C₁₄,
- les groupes hétéroaryle choisis parmi les groupes pyridyle, thiényle, furannyle, quinolyle, benzodioxannyle, benzodioxolyle, benzothiényle, benzofurannyle et pyrazinyle ;
- les groupes phénylalkyle(C₁-C₄), naphtylalkyle(C₁-C₄) éventuellement substitués sur le noyau aryle.
les groupes aryle et hétéroaryle pouvant être substitués par un ou plusieurs groupes choisis indépendamment parmi les halogènes, les groupes alkyle en C₁-C₄, trifluorométhyle, alkyl(C₁-C₄)thio, alkyl(C₁-C₄)sulfonyle, alkyl(C₁-C₄)oxy, nitro, les groupes -COOR, -CH₂COOR ou -O-CH₂-COOR, R étant un groupe alkyle en C₁-C₄,
- les groupes de formule : 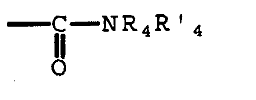
où R₄ et R'₄ sont choisis parmi les groupes alkyle en C₁-C₈, cycloalkyle mono ou polycyclique en C₃-C₁₂, ces groupes étant éventuellement substitués par des groupes choisis parmi les halogènes et le groupe hydroxy, R'₄ pouvant en outre être l'hydrogène ou bien R₄ et R'₄ forment ensemble un groupe tétraméthylène ou pentaméthylène, ces deux derniers groupes pouvant être eux-mêmes substitués notamment par un reste aryle en C₆-C₁₄ ou aryle (C₆-C₁₄)alkyle(C₁-C₄);
- les groupes de formule : 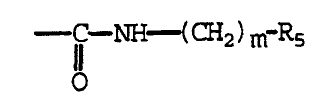
où m = 1 à 4 et R₅ est choisi parmi les groupes phényle, méthoxyphényle, indolyle, benzodioxolyle, benzodioxannyle, benzothiényle et benzofurannyle,
et R₂ est l'hydrogène,
ii) ou bien R₁ est l'hydrogène et R₂ est choisi parmi les groupes de formule :
-NH-CO-R₆,
R₆ étant choisi parmi les groupes alkoxy en C₁-C₄, cycloalkoxy en C₃-C₇, benzyloxy, méthoxyphényle, diméthoxyphényle, benzodioxolyle et benzodioxannyle,
et les groupes de formule :
-NH-SO₂-R₇,
R₇ étant choisi parmi :
- les groupes alkyle en C₁-C₅ éventuellement substitués par un ou plusieurs groupes choisis parmi les halogènes, les groupes hydroxy et le groupe trifluorométhyle ;
- les groupes cycloalkyle mono ou bicycliques en C₃-C₁₂;
- les groupes aryle mono, bi ou tricycliques en C₆-C₁₄ ;
- les groupes hétéroaryle choisis parmi les groupes pyridyle, thiényle, quinolyle, benzodioxannyle, benzodioxolyle, et isoxazolyle ;
- les groupes phénylalkyle (C₁-C₄) et naphtylalkyle (C₁-C₄) ;
- et les groupes de formule : 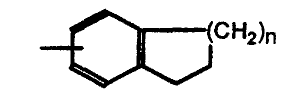
avec n = 1, 2 ou 3 ;
les groupes aryle ou hétéroaryle étant éventuellement substitués par ou plusieurs groupes choisis indépendamment parmi les halogènes, les group alkyle en C₁-C₄, trifluorométhyle, alkyl (C₁-C₄)thio, alkyl(C₁-C₄)oxy, alkyl(C₁-C₄)sulfonyle, nitro, di(alkylC₁-C₄)amino, les groupes -COOR, -CH₂-COOR, ou -O-CH₂COOR, R étant un groupe alkyle en C₁-C₄,
iii) R₃ est choisi parmi un atome d'hydrogène, un groupe alkyle en C₁-C₄ et un groupe phényl(alkyle en C₁-C₄),
iv) et Z₁ et Z₂ représentent l'hydrogène ou un groupe protecteur d'amine, et leurs sels d'addition avec des acides pharmaceutiquement acceptables.

3. Composés selon la revendication 1, dans lesquels R₁ = H et R₂ est un groupe de formule -NH-SO₂-R₇.

4. Composés selon la revendication 3, dans lesquels R₇ est choisi parmi les groupes naphtyle, naphtyle substitué, phénylthiényle et biphényle.

5. Composés selon l'une des revendications 1 à 4, dans lesquels les groupes Z1 et Z2 représentent l'hydrogène.

6. Composés selon la revendication 1 qui sont :
le (2S)-2-[(2-naphtylsulfonyl)amino]-3-{[2-({4-(4-pipéridinyl)-2-[2-(4-pipéridinyl)éthyl]butanoyl}amino) acétyl]amino}propanoate d'éthyle ; l'acide 3-(1,3-benzodioxol-5-yl)-3-{[2-({4-(4-pipéridinyl)-2-[2-(4-pipéridinyl)éthyl]butanoyl}amino)acétyl]amino}propanoïque ; l'acide 2-[(phénylsulfonyl)amino]-3-{[2-({4-(4-pipéridinyl)-2-[2-(4-pipéridinyl)éthyl]butanoyl}amino)acétyl]amino}propanoïque ; l'acide (2S)-2-[(2-naphtylsulfonyl)amino]-3-{[2-({4-(4-pipéridinyl)-2-[2-(4-pipéridinyl)éthyl]butanoyl}amino)acétyl]amino} propanoïque ; l'acide 2-[([1,1'biphényl]-4-ylsulfonyl)amino]-3-{[2-({4-(4-pipéridinyl)-2-[2-(4-pipéridinyl)éthyl]butanoyl}amino)acétyl]amino}propanoïque ; l'acide 2-[(1-naphtylsulfonyl)amino]-3-{[2-({4-(4-pipéridinyl)-2-[2-(4-pipéridinyl)éthyl]butanoyl}amino)acétyl]amino} propanoïque ; l'acide 2-[(2-thiénylsulfonyl)amino]-3-{[2-({4-(4-pipéridinyl)-2-[2-(4-pipéridinyl)éthyl]butanoyl}amino)acétyl]amino} propanoïque ; l'acide (2S)-2-{[(6-méthoxy-2-naphtyl)sulfonyl]amino}-3-{[2-({4-(4-pipéridinyl)-2-[2-(4-pipéridinyl)éthyl]butanoyl}amino)acétyl]amino}propanoïque ; l'acide 2-[(mésitylsulfonyl)amino]-3-{[2-({4-(4-pipéridinyl)-2-[2-(4-pipéridinyl)éthyl]butanoyl}amino)acétyl]amino} propanoïque ; l'acide 2-{[(4-méthylphényl)sulfonyl]amino}-3-{[2-({4-(4-pipéridinyl)-2-[2-(4-pipéridinyl)éthyl]butanoyl}amino)acétyl]amino} propanoïque ; l'acide 2-({[3-(trifluorométhyl)phényl]sulfonyl}amino) 3-{[2-({4-(4-pipéridinyl)-2-[2-(4-pipéridinyl)éthyl]butanoyl}amino)acétyl]amino} propanoïque ; l'acide 2-{[(3-nitrophényl)sulfonyl]amino}-3-{[2-({4-(4-pipéridinyl)-2-[2-(4-pipéridinyl)éthyl]butanoyl}amino)acétyl]amino} propanoïque ; l'acide 2-{[(3,5-diméthyl-4-isoxazolyl)sulfonyl]amino}-3-{[2-({4-(4-pipéridinyl)-2-[2-(4-pipéridinyl)éthyl]butanoyl}amino)acétyl]amino} propanoïque ; l'acide 2-({[5-(diméthylamino)-1-naphtyl)sulfonyl}amino)-3-{[2-({4-(4-pipéridinyl)-2-[2-(4-pipéridinyl)éthyl]butanoyl}amino)acétyl]amino} propanoïque ; l'acide 2-{[(6,7-diméthoxy-2-naphtyl)sulfonyl]amino}-3-{[2-({4-(4-pipéridinyl)-2-[2-(4-pipéridinyl)éthyl]butanoyl}amino)acétyl]amino}propanoïque ; l'acide 2-[(3-pyridinylsulfonyl)amino] 3-{[2-({4-(4-pipéridinyl)-2-[2-(4-pipéridinyl)éthyl]butanoyl}amino)acétyl]amino} propanoïque ; l'acide 2-[(1,3-benzodioxol-5-ylsulfonyl)amino]-3-{[2-({4-(4-pipéridinyl)-2-[2-(4-pipéridinyl)éthyl]butanoyl}amino)acétyl]amino} propanoïque ; l'acide 2-[(2,3-dihydro-1,4-benzodioxin-6-ylsulfonyl)amino]-3-{[2-((4-(4-pipéridinyl)-2-[2-(4-pipéridinyl)éthyl]butanoyl}amino)acétyl]amino} propanoïque ; l'acide 2-[(1-benzothiophèn-2-ylsulfonyl)amino]-3-{[2-({4-(4-pipéridinyl)-2-[2-(4-pipéridinyl)éthyl]butanoyl}amino)acétyl]amino}propanoïque ; l'acide 2-{[(2,5-diméthyl-3-furyl)sulfonyl]amino}-3-{[2-({4-(4-pipéridinyl)-2-[2-(4-pipéridinyl)éthyl]butanoyl}amino)acétyl]amino}propanoïque ; l'acide 2-{[(4-fluoro-1-naphtyl)sulfonyl]amino}-3-{[2-({4-(4-pipéridinyl)-2-[2-(4-pipéridinyl)éthyl]butanoyl}amino)acétyl]amino} propanoïque ; l'acide 2-{[(4-chloro-1-naphtyl)sulfonyl]amino}-3-{[2-({4-(4-pipéridinyl)-2-[2-(4-pipéridinyl)éthyl]butanoyl}amino)acétyl]amino} propanoïque ; l'acide 2-[(1-benzofuran-2-ylsulfonyl)amino]-3-{[2-({4-(4-pipéridinyl)-2-[2-(4-pipéridinyl)éthyl]butanoyl}amino)acétyl]amino}propanoïque ; l'acide 2-[(2,3-dihydro-1H-indèn-5-ylsulfonyl)amino]-3-{[2-({4-(4-pipéridinyl)-2-[2-(4-pipéridinyl)éthyl]butanoyl}amino)acétyl]amino}propanoïque ; l'acide 2-{[(5-phényl-2-thiényl))sulfonyl]amino}-3-{[2-({4-(4-pipéridinyl)-2-[2-(4-pipéridinyl)éthyl]butanoyl}amino)acétyl]amino}propanoïque ; l'acide 2-[(5,6,7,8-tétrahydro-2-naphthalenylsulfonyl)amino]3-{[2-({4-(4-pipéridinyl)-2-[2-(4-pipéridinyl)éthyl]butanoyl)amino)acétyl]amino} propanoïque ; l'acide 2-[(2-naphtylsulfonyl)amino]-3-{[3-({4-(4-pipéridinyl)-2-[2-(4-pipéridinyl)éthyl]butanoyl}amino)propanoyl]amino}propanoïque ; l'acide (2R)-2-[(2-naphtylsulfonyl)amino]-3-{[2-({4-(4-pipéridinyl)-2-[2-(4-pipéridinyl)éthyl]butanoyl}amino)acétyl]amino} propanoïque et leurs sels d'addition avec des acides pharmaceutiquement acceptables.

7. Procédé de préparation de composés de formule (I) par
a₁) réaction d'un acide de formule : 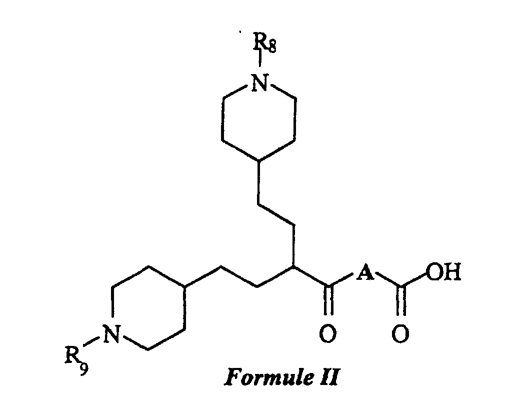 où R₈ et R₉ sont des groupes protecteurs, avec une amine de formule 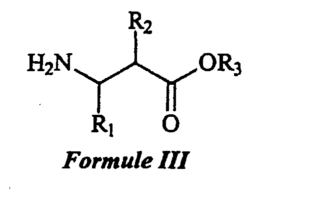 ou
a₂) réaction d'un acide de formule 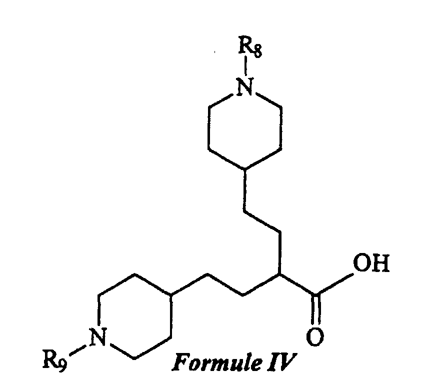 où R₈ et R₉ sont des groupes protecteurs, avec une amine de formùle 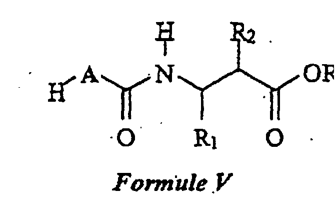 pour obtenir des composés de formule (I b) : 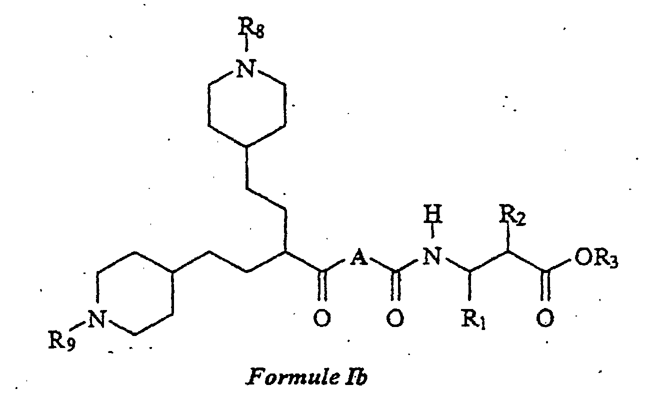
b) éventuellement conversion d'un groupe R₂ en un autre groupe R₂,
c) et éventuellement élimination des groupes protecteurs.

8. Composition thérapeutique qui comprend à titre de principe actif un composé tel que défini dans l'une des revendications 1 à 6.

9. Utilisation d'un composé tel que défini dans l'une des revendications 1 à 6 pour la fabrication d'un médicament antithrombotique.

10. Utilisation d'un composé tel que défini dans l'une des revendications 1 à 6 pour la fabrication d'un médicament pour inhiber la fixation du fibrinogène sur les plaquettes sanguines chez un mammifère.

11. Utilisation d'un composé tel que défini dans l'une des revendications 1 à 6 pour la fabrication d'un médicament pour inhiber l'agrégation des plaquettes sanguines chez un patient.

12. Utilisation selon la revendication 9 dans laquelle le médicament est destiné à traiter une thrombose chez un patient.

13. Utilisation selon la revendication 9 dans laquelle le médicament est destiné à la prévention de risque thrombotique chez un patient.

14. Composés de formule : 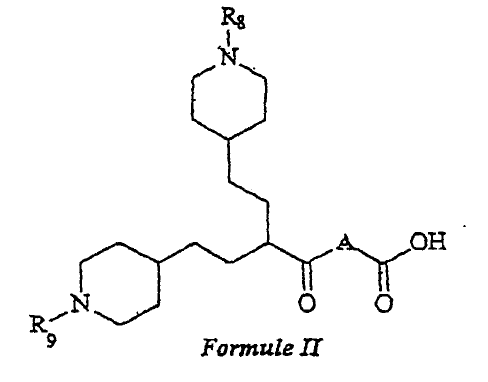 où R₈ et R₉ sont des groupes protecteurs et A a la signification donnée à la revendication 1.

15. Composés de formule : 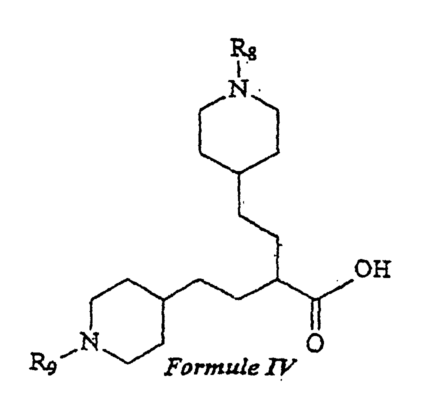 où R₈ et R₉ sont des groupes protecteurs.

## Claims

1. Compounds of formula: 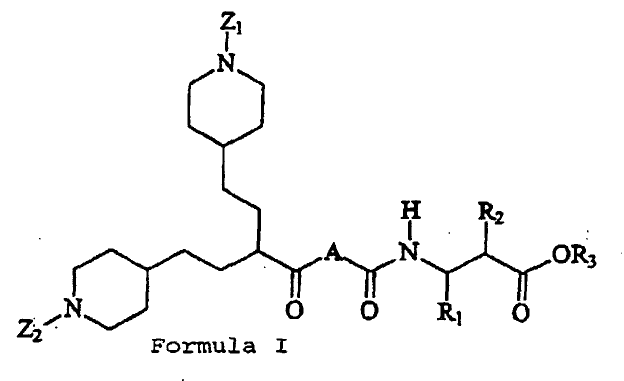 wherein:
i) either R₁ is selected from among:
- the C₁-C₄ alkyl groups, mono- or bicyclic C₃-C₁₂ cycloalkyl groups, C₂-C₄ alkenyl groups or C₂-C₄ alkynyl groups, these groups optionally being substituted by groups selected from among the halogens and hydroxy groups;
- the mono, bi or tricyclic C₆-C₁₄ aryl groups,
- the heteroaryl groups selected from among the pyridyl, thienyl, furanyl, quinolyl, benzodioxanyl, benzodioxolyl, benzothienyl, benzofuranyl and pyrazinyl groups
- the phenyl C₁-C₄ alkyl and naphthyl C₁-C₄ alkyl groups optionally substituted at the aryl nucleus,
the groups aryl and heteroaryl optionally being substituted by one or more groups selected independently from among the halogens, the C₁-C₄ alkyl, trifluoromethyl, C₁-C₄ alkyl thio, C₁-C₄ alkyl sulphonyl, C₁-C₄ alkyl oxy, nitro, the -COOR, -CH₂COOR or -O-CH₂-COOR groups, wherein R is a C₁-C₄ alkyl group,
- the groups of formula: 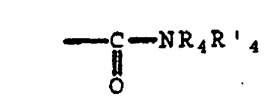
wherein R₄ and R'₄ are selected from among the C₁-C₈ alkyl, mono- or polycyclic C₃-C₁₂ cycloalkyl, these groups optionally being substituted by groups selected from among the halogen and hydroxy group, R'₄ also optionally being hydrogen or R₄ and R'₄ together form a tetramethylene or pentamethylene group, these two latter groups themselves optionally being substituted by a C₆-C₁₄ aryl or C₆-C₁₄ aryl C₁-C₄ alkyl group;
- the groups of formula: 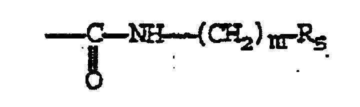
wherein m = 1 to 4 and R₅ is selected from among the phenyl, methoxyphenyl, indolyl, benzodioxolyl, benzodioxannyl, benzothienyl and benzofurannyl groups, and R₂ is hydrogen,
ii) or R₁ is hydrogen and R₂ is selected from among the groups of formula:
-NH-CO-R₆
R₆ being selected from among the C₁-C₄ alkoxy, C₃-C₇ cycloalkoxy, benzyloxy, methoxyphenyl, dimethoxyphenyl, benzodioxolyl and benzodioxannyl groups, and the groups of formula:
-NH-SO₂-R₇
R₇ being selected from among:
- the C₁-C₅ alkyl groups optionally substituted by one or more groups selected from among the halogens, the hydroxy groups and the trifluoromethyl group;
- the C₂-C₅ alkenyl groups;
- the mono- or bicyclic C₃-C₁₂ cycloalkyl groups;
- the mono-, bi, or tricyclic C₆-C₁₄ aryl groups;
- the hetero aryl groups selected from among the pyridyl, furyl, thienyl, quinolyl, benzodioxanyl, benzodioxolyl, isoxazolyl, benzodioxinyl, benzothienyl, thiazolyl, pyrazolyl, benzofuryl and benzothiazolyl;
- the C₁-C₄ phenylalkyl and C₁-C₄ naphthylalkyl groups;
- and the groups of formula: 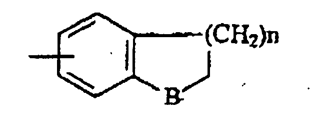
wherein n = 1, 2 or 3 and B is selected from among CH₂, O or S and NH,
the aryl or heteroaryl groups optionally being substituted by one or more groups selected independently from among the halogens, the C₁-C₄ alkyl, C₃-C₇ cycloalkyl, trifluoromethyl, C₁-C₄ alkylthio, C₁-C₄ alkyloxy, C₁-C₄ alkylsuphonyl, nitro, di (C₁-C₄ alkyi) amino groups, the groups -COOR, -CH₂-COOR or -O-CH₂COOR, R being a C₁-C₄ alkyl group, the phenyl and naphthyl groups and the heteroaryl groups selected from among the thienyl, furyl and pyridyl groups,
iii) R₃ is selected from among a hydrogen atom, a C₁-C₄ alkyl group and a phenyl (C₁-C₄ alkyl) group;
iv) A is selected from among the -NH-CHR₁₀-, -NH-CHR₁₀-CH₂- and 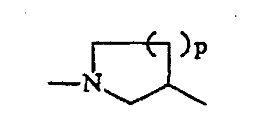 groups wherein p = 1 or 2,
- R₁₀ being selected from among hydrogen, a C₁-C₄ alkyl group and C₆-C₁₄ aryl group,
v) and Z₁ and Z₂ represent hydrogen or an amine protecting group,
and the addition salts thereof with pharmaceutically acceptable acids.

2. Compounds according to claim 1 of formula 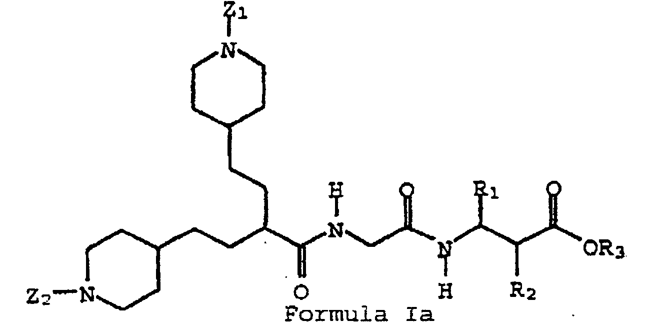 wherein:
i) either R₁ is selected from among:
- the C₁-C₄ alkyl groups, mono- or bicyclic C₃-C₁₂ cycloalkyl groups, C₂-C₄ alkenyl groups or C₂-C₄ alkynyl groups, these groups optionally being substituted by groups selected from among the halogens and hydroxy groups;
- the mono, bi or tricyclic C₆-C₁₄ aryl groups,
- the hetoroaryl groups selected from among the pyridyl, thienyl, furanyl, quinolyl, benzodioxanyl, benzodioxolyl, benzothienyl, benzofuranyl and pyrazinyl groups
- the phenyl C₁-C₄ alkyl and naphtyl C₁-C₄ alkyl groups optionally substituted at the aryl nucleus,
the groups aryl and heteroaryl optionally being substituted by one or more groups selected independently from among the halogens, the C₁-C₄ alkyl, trifluoromethyl, C₁-C₄ alkyl thio, C₁-C₄ alkyl sulfonyl, C₁-C₄ alkyl oxy, nitro, the -COOR, -CH₂COOR or -O-CH₂-COOR groups, wherein R is a C₁-C₄ alkyl group,
- the groups of formula: 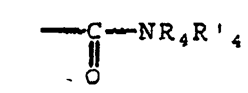
wherein R₄ and R'₄ are selected from among the C₁-C₈ alkyl, mono- or polycyclic C₃-C₁₂ cycloalkyl, these groups optionally being substituted by groups selected from among the halogen and hydroxy group, R'₄ also optionally being hydrogen or R₄ and R'₄ together form a tetramethylene or pentamethylene group, these two latt groups themselves optionally being substituted by a C₆- C₁₄ aryl or C₆-C₁₄ aryl C₁-C₄ alkyl group;
- the groups of formula 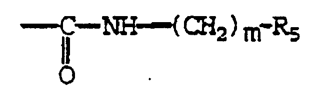
wherein m = 1 to 4 and R₅ is selected from among the phenyl, methoxyphenyl, indolyl, benzodioxolyl, benzodioxanyl, benzothienyl and benzofuranyl groups, and R₂ is hydrogen
ii) or R₁ is hydrogen and R₂ is selected from among the groups of formula:
-NH-CO-R₆
R₆ being selected from among the C₁-C₄ alkoxy, C₃-C₇ cycloalkoxy, benzyloxy, methoxyphenyl, dimethoxyphenyl, benzodioxolyl and benzodioxanyl groups, and the groups of formula:
- NH-SO₂-R₇
R₇ being selected from among:
- the C₁-C₅ alkyl groups optionally substituted by one or more groups selected from among the halogens, the hydroxy groups and the trifluoromethyl group;
- the mono or bicyclic C₃-C₁₂ cycloalkyl groups;
- the mono, bi, or tricyclic C₆-C₁₄ aryl groups;
- the hetero aryl groups selected from among the pyridyl, thienyl, quinolyl, benzodioxanyl, benzodioxolyl and isoxazolyl groups;
- the C₁-C₄ phenylalkyl and C₁-C₄ naphthylalkyl groups;
- and the groups of formula: 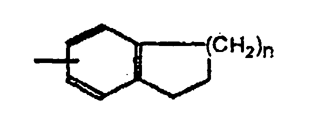
wherein n = 1, 2 or 3:
the aryl or heteroaryl groups optionally being substituted by one or more groups selected independently from among the halogens, the C₁-C₄ alkyl, trifluoromethyl, C₁-C₄ alkylthio, C₁-C₄ alkyloxy, C₁-C₄ alkylsuphonyl, nitro, di(C₁-C₄ alkyl) amino groups, the groups -COOR, -CH₂-COOR or -O-CH₂COOR, R being a C₁-C₄ alkyl group,
iii) R₃ is selected from among a hydrogen atom, a C₁-C₄ alkyl group and a phenyl C₁-C₄ alkyl group;
iv) and Z₁ and Z₂ represent hydrogen or an amine protecting group, and the addition salts thereof with pharmaceutically acceptable acids.

3. Compounds according to claim 1, wherein R₁ = H and R₂ is a group of formula -NH-SO₂-R₇.

4. Compounds according to claim 3, wherein R, is selected from among the naphthyl, substituted naphthyl, phenylthienyl and biphenyl groups.

5. Compounds according to one of claims 1 to 4 wherein the groups Z₁ and Z₂ represent hydrogen.

6. Compounds according to claim 1 which are:
ethyl (2S)-2-[(2-naphthylsulphonyl)amino]-3-{[2-({4-(4-piperidinyl)-2-[2-(4-piperidinyl)ethyl] butanoyl} amino)acetyl]amino}propanoate; 3-(1,3-benzodioxol-5-yl)-3-{[2-({4-(4-piperidinyl)-2-[2-(4-piperidinyl)ethyl] butanoyl}amino)acetyl]amino}propanoic acid; 2-[(phenylsulphonyl)amino]-3-{[2-({4-(4-piperidinyl)-2-[2-(4-piperidinyl)ethyl]butanoyl}amino)acetyl]amino} propanoic acid; (2S)-2-[(2-naphthylsulphonyl)amino]-3-{[2-({4-(4-piperidinyl)-2-[2-(4-piperidinyl)ethyl] butanoyl}amino)acetyl]amino}propanoic acid; 2[([1,1'-biphenyl]-4-ylsulphonyl)amino]-3-{[2-({4-(4-piperidinyl)-2-[2-(4-piperidinyl)ethyl] butanoyl} amino) acetyl]amino}propanoic acid; 2-[(1-naphthylsulphonyl) amino]-3-{[2-({4-(4-piperidinyl)-2-[2-(4-piperidinyl)ethyl]butanoyl}amino)acetyl]amino} propanoic acid; 2-[(2-thienylsulphonyl)amino]-3-{[2-({4-(4-piperidinyl)-2-[2-(4-piperidinyl)ethyl)butanoyl}amino) acetyl]amino} propanoic acid; (2S)-2-{[(6-methoxy-2-naphthyl)sulphonyl)amino]-3-{[2-({4-(4-piperidinyl)-2-[2-(4-piperidinyl)ethyl] butanoyl}amino)acetyl]amino} propanoic acid; 2- [(mesitylsulphonyl)amino] -3-{[2-({4-(4-piperidinyl)-2-[2-(4-piperidinyl)ethyl] butanoyl} amino)acetyl]amino}propanoic acid; 2-{[(4-methylphenyl) sulphonyl]amino)-3-{[2-({4-(4-piperidinyl)-2-[2-(4-piperidinyl)ethyl]butanoyl}amino)acetyl]amino} propanoic acid; 2-({[3-(trifluoromethyl)phenyl] sulphonyl}amino]-3-{[2-({4-(4-piperidinyl)-2-[2-(4-piperidinyl)ethyl]butanoyl}amino)acetyl]amino} propanoic acid; 2-{[(3-nitrophenyl)sulphonyl)amino]-3-{[2-({4-(4-piperidinyl)-2-[2-(4-piperidinyl)ethyl]butanoyl}amino} acetyl]amino} propanoic acid; 2-{[(3,5-dimethyl-4-isoxazolyl)sulphonyl)amino]-3-{[2-({4-(4-piperidinyl)-2-[2-(4-piperidinyl)ethyl]butanoyl}amino)acetyl]amino} propanoic acid; 2-([{5-(dimethylamino)-1-naphthyl) sulphonyl)amino]-3-{[2-({4-(4-piperidinyl)-2-[2-(4-piperidinyl)ethyl]butanoyl}amino)acetyl]amino} propanoic acid; 2-{[(6,7-(dimethoxy-2-naphthyl)sulphonyl]amino]-3-{[2-({4-(4-piperidinyl)-2-[2-(4-piperidinyl)ethyl] butanoyl}amino)acetyl]amino} propanoic acid; 2-[(3-pyridinylsulphonyl)amino]-3-{[2-({4-(4-piperidinyl)-2-[2-(4-piperidinyl)ethyl]butanoyl}amino)acetyl]amino} propanoic acid; 2-[(1,3-benzodioxol-5-ylsulphonyl) amino]-3-{[2-({4-(4-piperidinyl)-2-[2-(4-piperidinyl) ethyl]butanoyl}amino)acetyl]amino} propanoic acid; 2-[(2,3-dihydro-1,4-benzodioxin-6-ylsulphonyl)amino]-3-{[2-({4-(4-piperidinyl)-2-[2-(4-piperidinyl)ethyl] butanoyl}amino)acetyl]amino} propanoic acid; 2-[(1-benzothiophen-2-ylsulphonyl)amino]-3-{[2-({4-(4-piperidinyl)-2-[2-(4-piperidinyl)ethyl]butanoyl} amino)acetyl]amino} propanoic acid; 2-{[(2,5-(dimethyl-3-furyl)sulphonyl]amino]-3-{[2-({4-(4-piperidinyl)-2-[2-(4-piperidinyl) ethyl]butanoyl}amino)acetyl}amino} propanoic acid; 2-{[(4-fluoro-1-naphthyl)sulphonyl] amino]-3-{[2-({4-(4-piperidinyl)-2-[2-(4-piperidinyl) ethyl]butanoyl}amino)acetyl]amino} propanoic acid; 2-{[(4-chloro-1-naphthyl)sulphonyl]amino]-3-{[2-({4-(4-piperidinyl}-2-[2-(4-piperidinyl)ethyl)butanoyl}amino) acetyl]amino} propanoic acid; 2-[(1-benzofuran-2-ylsulphonyl) amino]-3-{[2-({4-(4-piperidinyl)-2-[2-(4-piperidinyl)ethyl]butanoyl}amino)acetyl]amino} propanoic acid; 2-[(2,3-dihydro-1H-inden-5-ylsulphonyl)amino]-3-{[2-({4-(4-piperidinyl)-2-[2-(4-piperidinyl)ethyl] butanoyl}amino)acetyl]amino} propanoic acid; 2-{[(5-phenyl-2-thienyl)sulphonyl]amino]-3-{[2-({4-(4-piperidinyl)-2-[2-(4-piperidinyl)ethyl]butanoyl} amino)acetyl]amino} propanoic acid; 2-[(5,6,7,8-tetrahydro-2-naphthalenylsulphonyl)amino]-3-{[2-({4-(4-piperidinyl)-2-[2-(4-piperidinyl)ethyl]butanoyl}amino) acetyl]amino} propanoic acid; 2-[(2-naphthylsulphonyl) amino]-3-{[3-({4-(4-piperidinyl)-2-[2-(4-piperidinyl) ethyl]butanoyl}amino)propanoyl]amino} propanoic acid; (2R)-2-[2-(naphthylsulphonyl)amino]-3-{[2-({4-(4-piperidinyl)-2-[2-(4-piperidinyl)ethyl]butanoyl}amino) acetyl]amino} propanoic acid and the addition salts thereof with pharmaceutically acceptable acids.

7. Process for preparing compounds of formula I by
a₁) reacting an acid of formula: 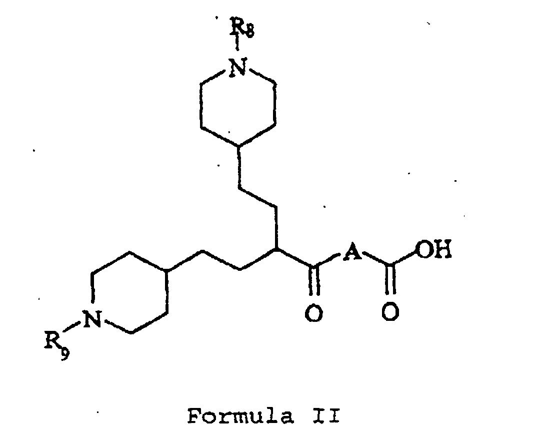 wherein R₈ and R₉ are protecting groups, with an amine of formula 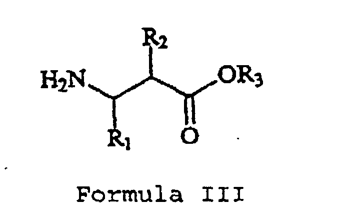 or
a₂) reacting an acid of formula 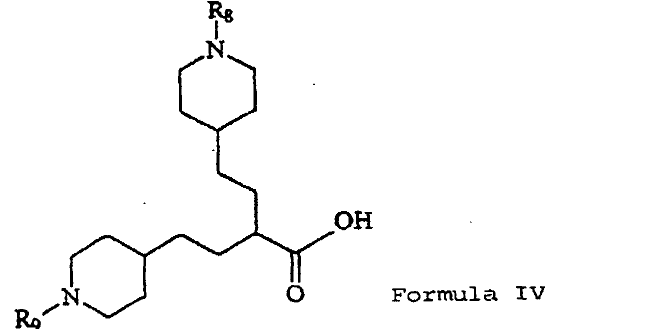 wherein R₈ and R₉ are protecting groups, with an amine of formula 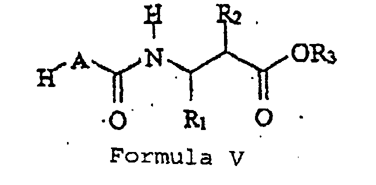 to obtain compounds of formula (1b): 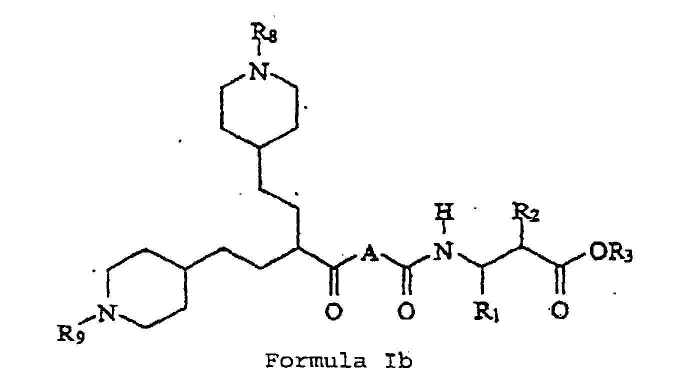
b) optionally converting a group R₂ into another group R₂,
c) and optionally eliminating the protecting groups.

8. Therapeutic composition which comprises as active ingredient a compound as defined in one of claims 1 to 6.

9. Use of a compound as defined in one of claims 1 to 6 for producing an antithrombotic medicament.

10. Use of a compound as defined in one of claims 1 to 6 for the production of a medicament for inhibiting the fixing of fibrinogen to blood platelets in a mammal.

11. Use of a compound as defined in one of claims 1 to 6 for the production of a medicament for inhibiting the aggregation of blood platelets in a patient.

12. Use according to claim 9 wherein the medicament is intended for treating a thrombosis in a patient.

13. Use according to claim 9 wherein the medicament is intended for preventing the risk of thrombosis in a patient.

14. Compounds of formula: 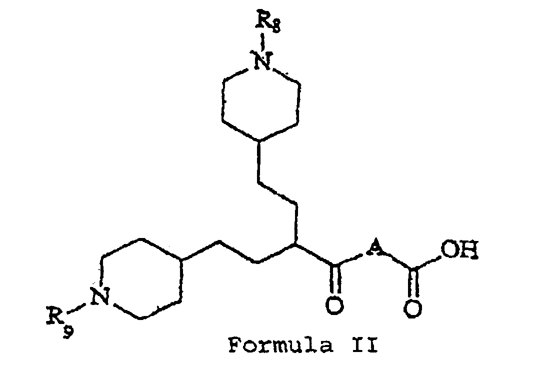 wherein R₈ and R₉ are protecting groups and A has the meaning given in claim 1.

15. Compounds of formula: 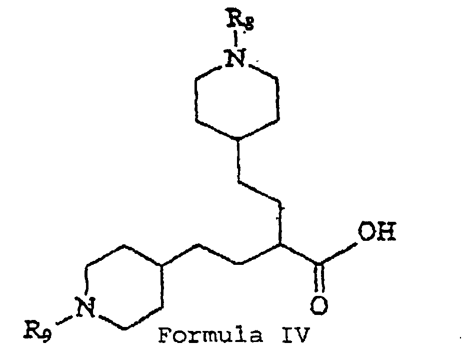 wherein R₈ and R₉ are protecting groups.

## Patentansprüche

1. Verbindungen der Formel 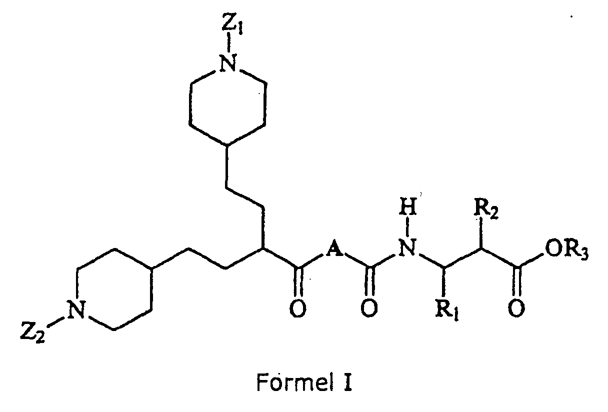 worin
i) R₁ ausgewählt ist aus:
- C₁-C₄-Alkyl-, mono- oder bicyclischen C₃-C₁₂-Cycloalkyl-, C₂-C₄-Alkenyloder C₂-C₄-Alkinylgruppen, wobei diese Gruppen gegebenenfalls durch aus Halogenen und einer Hydroxygruppe ausgewählte Gruppen substituiert sind;
- mono-, bi- oder tricyclischen C₆-C₁₄-Arylgruppen;
- Heteroarylgruppen, die aus Pyridyl-, Thienyl-, Furanyl-, Chinolyl-, Benzodioxanyl-, Benzodioxolyl-, Benzothienyl-, Benzofuranyl- und Pyrazinylgruppen ausgewählt sind;
- Phenyl-C₁-C₄-alkyl- und Naphthyl-C₁-C₄-alkylgruppen, die gegebenenfalls im Arylkern substituiert sind,
wobei die Aryl- und Heteroarylgruppen durch eine oder mehr Gruppen substituiert sein können, die unabhängig aus Halogenen, C₁-C₄-Alkyl-, Trifluormethyl-, C₁-C₄-Alkylthio-, C₁-C₄-Alkyfsulfonyl-, C₁-C₄-Alkyloxy-, Nitrogruppen, den Gruppen -COOR, -CH₂COOR oder -O-CH₂-COOR ausgewählt sind, wobei R eine C₁-C₄-Alkylgruppe ist;
- Gruppen der Formel 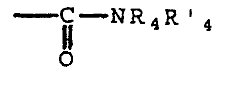
worin R₄ und R'₄ aus C₁-C₈-Alkyl-, mono- oder polycyclischen C₃-C₁₂-Cycloalkylgruppen ausgewählt sind und diese Gruppen gegebenenfalls durch aus Halogenen und einer Hydroxygruppe ausgewählte Gruppen substituiert sind, wobei R'₄ außerdem Wasserstoff sein kann, oder R₄ und R'₄ zusammen eine Tetramethylen- oder Pentamethylengruppe bilden und diese beiden letzten Gruppen selbst durch einen C₆-C₁₄-Aryl- oder C₆-C₁₄-Aryl-C₁-C₄-alkylrest substituiert sein können;
- Gruppen der Formel 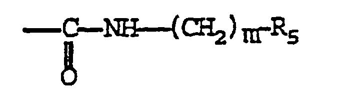
worin m = 1 bis 4 und R₅ aus Phenyl-, Methoxyphenyl-, Indolyl-, Benzodioxolyl-, Benzodioxanyl-, Benzothienyl- und Benzofuranylgruppen ausgewählt ist;
und R₂ Wasserstoff ist,
ii) oder R₁ Wasserstoff ist und R₂ ausgewählt ist aus Gruppen der Formel
-NH-CO-R₆,
wobei R₆ aus C₁-C₄-Alkoxy-, C₃-C₇-Cycloalkyloxy-, Benzyloxy-, Methoxyphenyl-, Dimethoxyphenyl-, Benzodioxolyl- und Benzodioxanylgruppen ausgewählt ist,
und Gruppen der Formel
-NH-SO₂-R₇,
wobei R₇ ausgewählt ist aus:
- C₁-C₅-Alkylgruppen, die gegebenenfalls durch eine oder mehr Gruppen substituiert sind, die aus Halogenen, einer Hydroxy- und Trifluormethylgruppe ausgewählt sind;
- C₂-C₅-Alkenylgruppen;
- mono- oder bicyclischen C₃-C₁₂-Cycloalkylgruppen;
- mono-, bi- oder tricyclischen C₆-C₁₄-Arylgruppen;
- Heteroarylgruppen, die aus Pyridyl-, Furyl-, Thienyl-, Chinolyl-, Benzodioxanyl-, Benzodioxolyl-, Isoxazolyl-, Benzodioxinyl-, Benzothienyl-, Thiazolyl-, Pyrazolyl-, Benzofuryl- und Benzothiazolylgruppen ausgewählt sind;
- Phenyl-C₁-C₄-alkyl- und Naphthyl-C₁-C₄-alkylgruppen;
- und Gruppen der Formel 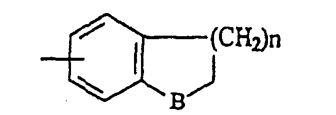
mit n = 1, 2 oder 3, wobei B aus -CH₂-, O oder S und -NH- ausgewählt ist; und die Aryl- oder Heteroarylgruppen gegebenenfalls durch eine oder mehr Gruppen substituiert sind, die unabhängig aus Halogenen, einer C₁-C₄-Alkyl-, C₃-C₇-Cycloalkyl-, Trifluormethyl-, C₁-C₄-Alkylthio-, C₁-C₄-Alkyloxy-, C₁-C₄-Alkylsulfonyl-, Nitro-, Di-C₁-C₄-alkylaminogruppe, den Gruppen -COOR, -CH₂-COOR oder -O-CH₂COOR ausgewählt sind, wobei R eine C₁-C₄-Alkylgruppe, Phenyl-, Naphthyl- und eine aus einer Thienyl-, Furyl- und Pyridylgruppe ausgewählte Heteroarylgruppe ist.
iii) R₃ aus einem Wasserstoffatom, einer C₁-C₄-Alkylgruppe und einer Phenyl-C₁-C₄-alkylgruppe ausgewählt ist;
iv) A aus den Gruppen -NH-CHR₁₀-, -NH-CHR₁₀-CH₂- und 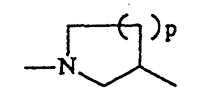 mit p = 1 oder 2 ausgewählt ist,
- wobei R¹⁰ aus Wasserstoff, einer C₁-C₄-Alkylgruppe und einer C₆-C₁₄-Arylgruppe ausgewählt ist,
v) und Z₁ und Z₂ Wasserstoff oder eine Aminschutzgruppe darstellen,
und ihre Additionssalze mit pharmazeutisch annehmbaren Säuren.

2. Verbindungen gemäß Anspruch 1 der Formel 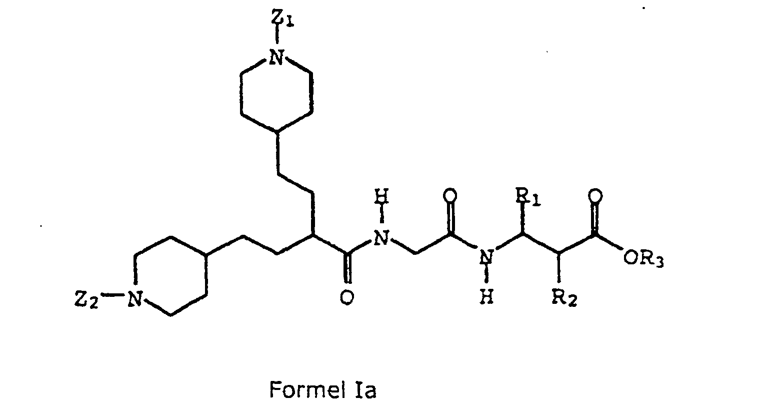 worin
i) R₁ ausgewählt ist aus
- C₁-C₄-Alkyl-, mono- oder bicyclischen C₃-C₁₂-Cycloalkyl-, C₂-C₄-Alkenyloder C₂-C₄-Alkinylgruppen, wobei diese Gruppen gegebenenfalls durch aus Halogenen und einer Hydroxygruppe ausgewählte Gruppen substituiert sind;
- mono-, bi- oder tricyclischen C₆-C₁₄-Arylgruppen;
- Heteroarylgruppen, die aus Pyridyl-, Thienyl-, Furanyl-, Chinolyl-, Benzodioxanyl-, Benzodioxolyl-, Benzothienyl-, Benzofuranyl- und Pyrazinylgruppen ausgewählt sind;
- Phenyl-C₁-C₄-alkyl- und Naphthyl-C₁-C₄-alkylgruppen, die gegebenenfalls im Arylkern substituiert sind,
wobei die Aryl- und Heteroarylgruppen durch eine oder mehr Gruppen substituiert sein können, die unabhängig aus Halogenen, C₁-C₄-Alkyl-, Trifluormethyl-, C₁-C₄-Alkylthio-, C₁-C₄-Alkylsulfonyl-, C₁-C₄-Alkyloxy-, Nitrogruppen, den Gruppen -COOR, -CH₂COOR oder -O-CH₂-COOR ausgewählt sind, wobei R eine C₁-C₄-Alkylgruppe ist;
- Gruppen der Formel 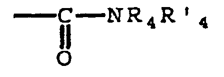
worin R₄ und R'₄ aus C₁-C₈-Alkyl-, mono- oder polycyclischen C₃-C₁₂-Cycloalkylgruppen ausgewählt sind und diese Gruppen gegebenenfalls durch aus Halogenen und einer Hydroxygruppe ausgewählte Gruppen substituiert sind, wobei R'₄ außerdem Wasserstoff sein kann oder R₄ und R'₄ zusammen eine Tetramethylen- oder Pentamethylengruppe bilden und diese beiden letzten Gruppen selbst durch insbesondere einen C₆-C₁₄-Aryl- oder C₆-C₁₄-Aryl-C₁-C₄-alkylrest substituiert sein können;
- Gruppen der Formel 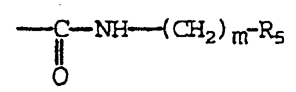
worin m = 1 bis 4 und R₅ aus Phenyl-, Methoxyphenyl-, Indolyl-, Benzodioxolyl-, Benzodioxanyl-, Benzothienyl- und Benzofuranylgruppen ausgewählt ist;
und R₂ Wasserstoff ist,
ii) oder R₁ Wasserstoff ist und R₂ ausgewählt ist aus Gruppen der Formel
-NH-CO-R₆,
wobei R₆ aus C₁-C₄-Alkoxy-, C₃-C₇-Cycloalkyloxy-, Benzyloxy-, Methoxyphenyl-, Dimethoxyphenyl-, Benzodioxolyl- und Benzodioxanyl ausgewählt ist,
und Gruppen der Formel
-NH-SO₂-R₇,
wobei R₇ ausgewählt ist aus
- C₁-C₅-Alkylgruppen, die gegebenenfalls durch eine oder mehr Gruppen substituiert sind, die aus Halogenen, einer Hydroxy- und Trifluormethylgruppe ausgewählt sind;
- mono- oder bicyclischen C₃-C₁₂-Cycloalkylgruppen;
- mono-, bi- oder tricyclischen C₆-C₁₄-Arylgruppen;
- Heteroarylgruppen, die aus Pyridyl-, Thienyl-, Chinolyl-, Benzodioxanyl-, Benzodioxolyl- und Isoxazolyl- ausgewählt sind;
- Phenyl-C₁-C₄-alkyl- und Naphthyl-C₁-C₄-alkylgruppen;
- und Gruppen der Formel 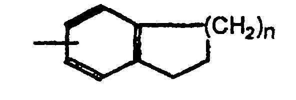
mit n = 1, 2 oder 3;
wobei die Aryl- oder Heteroarylgruppen gegebenenfalls durch eine oder mehr Gruppen substituiert sind, die unabhängig aus Halogenen, einer C₁-C₄-Alkyl-, Trifluormethyl-, C₁-C₄-Alkylthio-, C₁-C₄-Alkyloxy-, C₁-C₄-Alkylsulfonyl-, Nitro-, Di-C₁-C₄-alkylaminogruppe, den Gruppen -COOR, -CH₂-COOR oder -O-CH₂COOR ausgewählt sind, wobei R eine C₁-C₄-Alkylgruppe ist,
iii) R₃ aus einem Wasserstoffatom, einer C₁-C₄-Alkylgruppe und einer Phenyl-C₁-C₄-alkylgruppe ausgewählt ist,
iv) und Z₁ und Z₂ Wasserstoff oder eine Aminschutzgruppe darstellen,
und ihre Additionssalze mit pharmazeutisch annehmbaren Säuren.

3. Verbindungen gemäß Anspruch 1, bei denen R₁ = H und R₂ eine Gruppe der Formel -NH-SO₂-R₇ ist.

4. Verbindungen gemäß Anspruch 3, wobei R₇ aus Naphthyl-, substituierten Naphthyl-, Phenylthienyl- und Biphenylgruppen ausgewählt ist.

5. Verbindungen gemäß einem der Ansprüche 1 bis 4, bei denen die Gruppen Z₁ und Z₂ Wasserstoff darstellen.

6. Verbindungen gemäß Anspruch 1, die Ethyl-(2S)-2-[(2-Naphthylsulfonyl)amino]-3-{[2-({4-(4-piperidinyl)-2-[2-(4-piperidinyl)ethyl]butanoyl}amino)acetyl]amino}propanoat; 3-(1,3-Benzodioxol-5-yl)-3-{[2-({4-(4-piperidinyl)-2-[2-(4-piperidinyl)ethyl]butanoyl}amino)acetyl]-amino}propansäure; 2-[(Phenylsulfonyl)amino]-3-{[2-({4-(4-piperidinyl)-2-[2-(4-piperidinyl)ethyl]butanoyl}amino)acetyl]amino}propansäure; (2S)-2-[(2-Naphthylsulfonyl)amino]-3-{[2-({4-(4-piperidinyl)-2-[2-(4-piperidinyl)ethyl]-butanoyl}amino)acetyl]amino}propansäure; 2-[([1,1'-Biphenyl]-4-ylsulfonyl)-amino]-3-{[2-({4-(4-piperidinyl)-2-[2-(4-piperidinyl)ethyl]butanoyl}amino)-acetyl]amino}propansäure; 2-[(1-Naphthylsulfonyl)amino]-3-{[2-({4-(4-piperidinyl)-2-[2-(4-piperidinyl)ethyl]butanoyl}amino)acetyl]amino}propansäure; 2-[(2-Thienylsulfonyl)amino]-3-{[2-({4-(4-piperidinyl)-2-[2-(4-piperidinyl)ethyl]-butanoyl}amino)acetyl]amino}propansäure; (2S)-2-{[(6-Methoxy-2-naphthyl)-sulfonyl]amino}-3-{[2-({4-(4-piperidinyl)-2-[2-(4-piperidinyl)ethyl]butanoyl}amino)acetyl]amino}propansäure; 2-[(Mesitylsulfonyl)amino]-3-{[2-({4-(4-piperidinyl)-2-[2-(4-piperidinyl)ethyl]butanoyl}amino)acetyl]amino}propansäure; 2-{[(4-Methylphenyl)sulfonyl]amino}-3-{[2-({4-(4-piperidinyl)-2-[2-(4-piperidinyl)ethyl]butanoyl}amino)acetyl]amino}propansäure; 2-({[3-(Trifluormethyl)-phenyl]sulfonyl}amino)-3-{[2-({4-(4-piperidinyl)-2-[2-(4-piperidinyl)ethyl]butanoyl}amino)acetyl]amino}propansäure; 2-{[(3-Nitrophenyl)sulfonyl]amino}-3-{[2-({4-(4-piperidinyl)2-[2-(4-piperidinyl)ethyl]butanoyl}amino)acetyl]amino}propansäure; 2-{[(3,5-Dimethyl-4-isoxazolyl)sulfonyl]amino}-3-{[2-({4-(4-piperidinyl)-2-[2-(4-piperidinyl)ethyl]butanoyl}amino)acetyl]amino}propansäure; 2-({[5-(Dimethylamino)-1-naphthyl)sulfonyl}amino)-3-{[2-({4-(4-piperidinyl)-2-[2-(4-piperidinyl)ethyl]butanoyl}amino)acetyl]amino}propansäure; 2-{[(6,7-Dimethoxy-2-naphthyl)sulfonyl]amino)-3-{[2-({4-(4-piperidinyl)-2-[2-(4-piperidinyl)ethyl]butanoyl}amino)acetyl]amino}propansäure; 2-[(3-Pyridinylsulfonyl)-amino] 3-{[2-({4-(4-piperidinyl)-2-[2-(4-piperidinyl)ethyl]butanoyl}amino)-acetyl]emino}propansäure; 2-[(1,3-Benzodioxol-5-ylsulfonyl)amino]-3-{[2-({4-(4-piperidinyl)-2-[2-(4-piperidinyl)ethyl]butanoyl}amino)acetyl]amino}propansäure; 2-[(2,3-Dihydro-1,4-benzodioxin-5-ylsulfonyl)amino]-3-{[2-({4-(4-piperidinyl)-2-[2-(4-piperidinyl)ethyl]butanoyl}amino)acetyl]amino} propansäure; 2-[(1-Benzothiophen-2-ylsulfonyl)amino]-3-{[2-({4-(4-piperidinyl)-2-[2-(4-piperidinyl)ethyl]butanoyl}amino)acetyl]amino}propansäure; 2-{[(2,5-Dimethyl-3-furyl)sulfonyl]amino}-3-{[2-({4-(4-piperidinyl)-2-[2-(4-piperidinyl)ethyl]butanoyl}amino)acetyl]amino}propansäure; 2-([(4-Fluor-1-naphthyl)sulfonyl]-amino}-3-{[2-({4-(4-piperidinyl)-2-[2-(4-piperidinyl)ethyl]butanoyl}amino)-acetyl]amino}propansäure; 2-{[(4-Chlor-1-naphthyl)sulfonyl]amino}-3-{[2-({4-(4-piperidinyl)-2-[2-(4-piperidinyl)ethyl]butanoyl}amino)acetyl]amino}propansäure; 2-[(1-Benzofuran-2-ylsulfonyl)amino]-3-{[2-({4-(4-piperidinyl)-2-[2-(4-piperidinyl)ethyl]butanoyl}amino)acetyl]amino}propansäure; 2-[(2,3-Dihydro-1H-inden-5-ylsulfonyl)amino]-3-{[2-({4-(4-piperidinyl)-2-[2-{4-piperidinyl)-ethyl]butanoyl}amino)acetyl]amino}propansäure; 2-{[(5-Phenyl-2-thienyl)-sulfonyl]amino}-3-{[2-({4-(4-piperidinyl}-2-[2-(4-piperidinyl)ethyl]butanoyl}amino)acetyl]amino}propansäure; 2-[(5,6,7,8-Tetrahydro-2-naphthalinylsulfonyl)amino]-3-{[2-({4-(4-piperidinyl)-2-[2-(4-piperidinyl)ethyl]butanoyl}amino)-acetyl]amino}propansäure; 2-[(2-Naphthylsulfonyl)amino]-3-{[3-({4-(4-piperidinyl)-2-[2-(4-piperidinyl)ethyl]butanoyl}amino)propanoyl]amino}propansäure; (2R)-2-[(2-Naphthylsulfonyl)amino]-3-{[2-({4-(4-piperidinyl)-2-[2-(4-piperidinyl)ethyl]butanoyl}amino)acetyl]amino}propansäure und ihre Additionssalze mit pharmazeutisch annehmbaren Säuren sind.

7. Verfahren zur Herstellung von Verbindungen der Formel (I) durch
a₁) Reaktion einer Säure der Formel 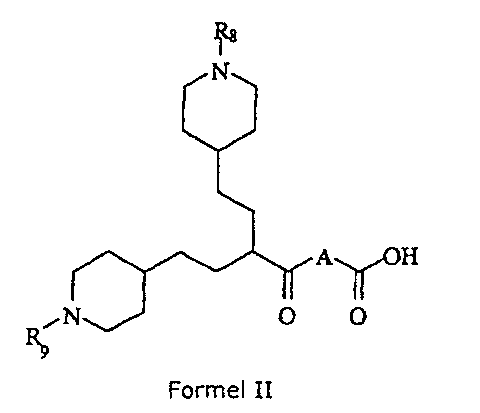 worin R₈ und R₉ Schutzgruppen sind, mit einem Amin der Formel 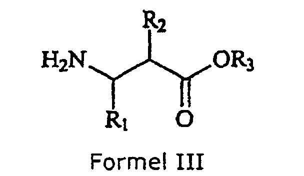 oder
a₂) Reaktion einer Säure der Formel 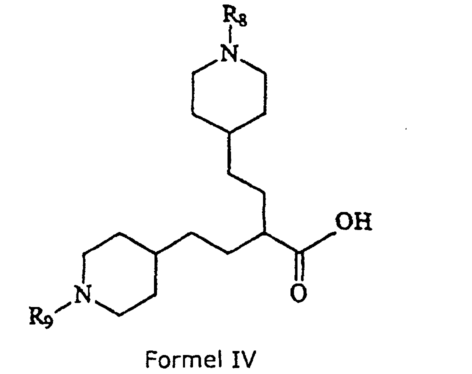 worin R₈ und R₉ Schutzgruppen sind, mit einem Amin der Formel 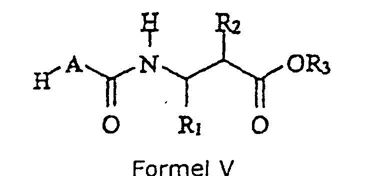 zum Erhalten von Verbindungen der Formel (Ib): 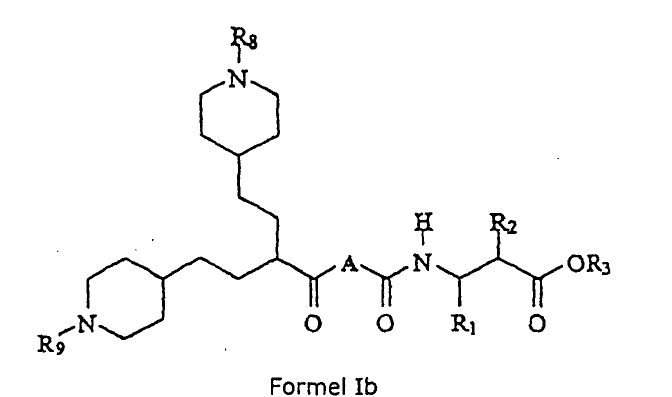
b) gegebenenfalls die Umwandlung einer Gruppe R₂ in eine andere Gruppe R₂,
c) und gegebenenfalls Entfernen der Schutzgruppen.

8. Therapeutische Zusammensetzung, die als aktiven Bestandteil eine in einem der Ansprüche 1 bis 6 definierte Verbindung umfaßt.

9. Verwendung einer in einem der Ansprüche 1 bis 6 definierten Verbindung zur Herstellung eines antithrombotischen Arzneimittels.

10. Verwendung einer in einem der Ansprüche 1 bis 6 definierten Verbindung zur Herstellung eines Arzneimittels zum Hemmen der Fixierung von Fibrinogen auf den Blutplättchen eines Säugers.

11. Verwendung einer in einem der Ansprüche 1 bis 6 definierten Verbindung zur Herstellung eines Arzneimittels zum Hemmen der Blutplättchenaggregation bei einem Patienten.

12. Verwendung gemäß Anspruch 9, bei der das Arzneimittel zum Behandeln einer Thrombose bei einem Patienten bestimmt ist.

13. Verwendung gemäß Anspruch 9, bei der das Arzneimittel zur Prophylaxe eines Thromboserisikos bei einem Patienten bestimmt ist.

14. Verbindungen der Formel 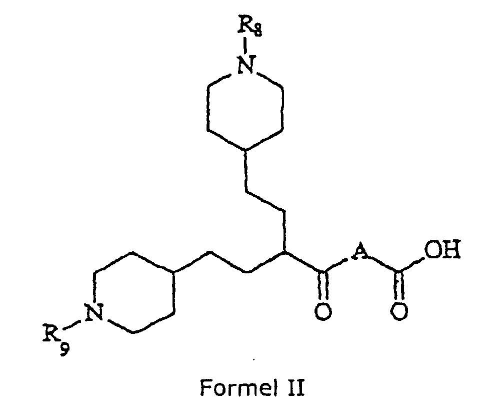 worin R₈ und R₉ Schutzgruppen sind und A die in Anspruch 1 bestimmte Bedeutung aufweist.

15. Verbindungen der Formel 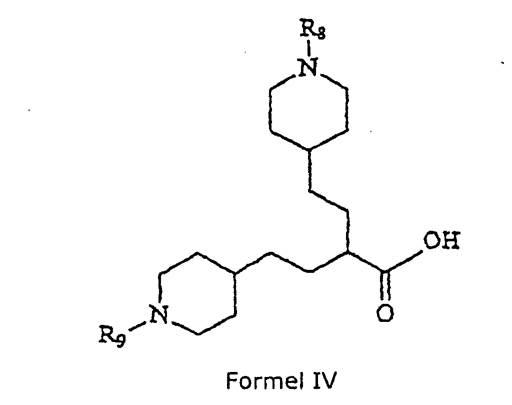 worin R₈ und R₉ Schutzgruppen sind.
